# EUROPEAN PATENT APPLICATION

(11) **EP 2 113 515 A2**
(43) Date of publication of application: **04.11.2009**
(21) Application number: 09157130.7
(22) Date of filing: 10.07.2007
(51) Int. Cl.: C07K 14/705, C07K 14/47

(54) **GalNAc specific binding molecules and uses thereof**

(30) Priority: 10.07.2006 WO PCT/NL2006/000348
(62) Divisional of application: 07768926.3
(71) Applicant: VERENIGING VOOR CHRISTELIJK WETENSCHAPPELIJK ONDERWIJS, NL-1081 HV Amsterdam (NL)
(72) Inventor: Van Vliet, Sandra Johanna, 1069 EG Amsterdam (NL); Van Kooyk, Yvette, 1081 HJ Amsterdam (NL)
(74) Representative: Habets, Winand

(57) **Abstract**

The present invention provides among others means and methods for detecting terminal GalNAc containing molecules. A preferred molecule for detecting said structures is a molecule comprising a carbohydrate binding part of MGL.

## Description

The present invention relates to GalNAc specific binding molecules, uses thereof and means and methods for manipulating macrophage galactose-type lectin (MGL) binding mediated signalling in cells that bind or have MGL. The invention further relates to uses of means of the invention as a diagnostic tool and/or as a medicament, in particular for the prophylaxis or treatment of chronic inflammatory conditions, autoimmune diseases, unwanted inflammatory responses in transplantation settings and/or tumours. It also relates to an in *vitro* method for diagnosis and/or prognosis of chronic inflammatory conditions, autoimmune diseases, and/or unwanted inflammatory responses in transplantation settings, a tumour and T cell leukaemia.

Dendritic cells (DC) and macrophages (MΦ) are professional antigen presenting cells (APC), which line all peripheral tissues where they screen their environment for incoming pathogens and for local changes in immune homeostasis. Both types of APC display a unique plasticity; thay are capable of adopting several functional phenotypes depending on environmental changes and external stimuli they receive. APC are instrumental in initiating adaptive immunity and pathogen clearance, however they also actively control several processes involved in tissue repair, clearance of apoptotic cells and maintenance of tolerance to non-harmful (self) antigens. Microbial products, like lipopolysaccharide (LPS), trigger DC maturation, thereby licensing DC for naïve T cell activation. MΦ exposure to LPS and IFNγ drives the differentiation of classically activated MΦ with an enhanced ability to produce nitric oxide and to kill intracellular pathogens. In contrast, glucocorticoids instruct the differentiation of tolerogenic DC or alternatively activated MΦ (aaMΦ). Both these glucocorticoid-elicited APC have a strongly reduced capacity to stimulate naïve T cell proliferation, implicating these APC in steady state control. Furthermore, aaMΦ can provide negative regulatory signals to protect the host form excessive inflammatory responses.

All APC subsets express exclusive sets of C-type lectin receptors (CLR), a protein family recognizing specific carbohydrate moieties on glycoproteins in a Ca²⁺-dependent fashion. CLR have been shown to recognize both exogenous (non-self) and endogenous (self) glycoproteins or -lipids.
These antigens can be internalized and presented on MHC class I or II.
However, in steady state, targeting antigen to CLR without any danger signals results in T cell unresponsiveness. Therefore, CLR are not only involved in pattern recognition of invading micro-organisms, but instead may play an important role in the control of immune homeostasis by clearing self-antigens, thereby actively inducing tolerance. In contrast, Dectin-1-mediated signaling synergizes with TLR2 induced pathways, suggesting that some CLR may also have immune-activating properties.

The CLR Macrophage Galactose-type lectin (MGL) is expressed by human immature DC and is considered to be a marker for aaMΦ. Recently the carbohydrate recognition profile of MGL was elucidated, having a unique specificity for terminal GalNAc-residues. So far no self-ligands have been identified that interact with human MGL.

To explore a role for human MGL in the immune system, the present inventors set out to identify counterreceptors for MGL and study the functional consequences of such interaction. By screening leukocytes for MGL recognition a strong interaction was observed between MGL and CD45 on effector-type T cells, making MGL the first cellular receptor identified on professional APC for CD45.

CD45 is the prototype tyrosine phosphatase expressed on the T cell surface. CD45 can dephosphorylate the negative regulatory tyrosine on the protein tyrosine kinase p56^{lck}. This results in an active pool of Lck that can phopshorylate TCR ITAMs leading to propagation of TCR signalling. CD45 is alternatively spliced into five different isoforms with differential glycosylation. Considering that glycosylation of CD45 changes during T cell differentiation and activation, cell-specific glycosylation could provide a means to influence various aspects of the immune system, including TCR and cytokine receptor signalling.

Autoimmune reactions often have fatal consequences. They can cause the destruction of vital organs, which manifests in the development of chronic autoimmune diseases such as multiple sclerosis, rheumatoid arthritis or diabetes. The driving forces in the progression of these diseases are autoreactive immune cells. In most cases these autoreactive immune cells are CD4+ T cells, which escaped the self-tolerance control mechanisms of the immune system. In non-pathogenic situations CD4+ T cells act as effector T cells. In pathogenic situations CD4+ T cells also act as effector cells, however, in situations some of the responses are undesired or out of control. They control or mediate the activation and differentiation of other immune cells such as cytotoxic CD8+ T cells (CTL), natural killer (NK) cells and B cells. In the situation of autoimmune diseases, however, they are not only mediators of the immune response, but also key players of the autoimmune response, and they are found in high numbers in chronic inflammatory diseases.
Directly or indirectly (via cytokines) they are, through action of CD8+ T cells, responsible for the characteristic tissue destruction, triggered by the recognition of autoantigens. Suppression of activated T cells is particularly important for the attenuation of overshooting immune reactions. In the present invention it was found that MGL on tolerogenic APC binds specifically to GalNAc structures present on CD45 on effector T cells.
MGL is involved in suppression of effector CD4⁺ T cell proliferation by tolerogenic DC or aaMΦ. Binding of MGL reduces CD45 phosphatase activity and inhibits calcium mobilization after CD3 triggering. The MGL-CD45 interaction inhibits TCR-mediated T cell activation characterized by reduced production of inflammatory cytokine and even the induction of T cell death.

Thus, activated T cells can be suppressed by the interaction between the carbohydrate recognition domain of MGL and the carbohydrate structures, in particular the terminal GalNAc residues, on CD45. Thus, MGL is potently involved in regulating the effector T cell function. In this respect it is interesting to note that in chronic inflammatory diseases such as Multiple Sclerosis, Rheumatoid arthritis and osteoarthritis, MGL expression is upregulated. The present inventors have found that the upregulation of MGL expression is a self-protecting mechanism initiated to prevent excessive inflammation and extensive tissue damage. Via the MGL-CD45 interaction tolerogenic APC are able to enhance the TCR threshold for activation, resulting in reduced cytokine production and proliferatio, providing another mechanism for APC to control unwanted effector responses.

As in the present invention it was found that terminal GalNAc residues are important in the recognition of CD45 positive cells by MGL the present invention in one embodiment provides a method for providing a CD45 positive cell with a binding molecule comprising providing said cell with a binding molecule specific for a terminal GalNAc residue and that comprises a carbohydrate binding part of a macrophage galactose-type lectin (MGL).

Several isoforms of CD45 are present on CD45 positive cells. It was found that MGL specific GalNAc residues are present on a subset of CD45 isoforms. As the different isoforms are expressed at different levels in CD45 expressing cells the invention further provides a method for differentially marking CD45 positive cells, comprising contacting a (sample containing a) subpopulation of CD45 positive cells with a binding molecule specific for a terminal GalNAc residue. In a preferred embodiment said CD45 isoform that binds said GalNAc specific binding molecule comprises at least the RA, RB or RC part of CD45 or a combination thereof. In a preferred embodiment said binding molecule comprises a carbohydrate binding part of an MGL. Said method for marking can be used, for example, for detecting chronic inflammation in an individual.

Using recombinant DNA technology it is possible to express CD45 in a variety of different cell types. Preferred target cells for binding of said GalNAc specific binding molecule are CD45 positive T-cells. Preferably said target cells are primate cells, preferably human cells.

In the present invention it was found that binding of a GalNAc specific binding molecule of the invention results in alteration of the response in a collection of T-cells. The present invention therefore further provides the use of a binding molecule specific for GalNAc for altering a T-cell response of a collection of T cells. One of the observed effects is that T-cell receptor mediated signalling is inhibited in the cell population. Further provided is thus the use of a method or a binding molecule of the invention for inhibiting a T-cell receptor (TCR)-mediated signalling pathway. In another embodiment the invention provides the use of a method or a binding molecule of the invention for inducing apoptosis in a T-cell wherein a TCR-mediated signalling pathway is activated. In another preferred embodiment the invention provides the use of a method or a binding molecule of the invention for raising the activation threshold of a CD45 positive effector type T-cell. In yet another preferred embodiment the invention provides the use of a method or a binding molecule of the invention for inhibiting proliferation of a CD45 positive effector type T-cell.

As it was found that MGL binds CD45 isoforms, GalNAc containing CD45 isoforms can be used to mark MGL positive cells. The invention thus further provides a method for providing an MGL positive cell with a binding molecule comprising providing said cell with a carbohydrate containing part of a CD45 molecule. Preferably said part comprises at least the RA, RB, RC part of CD45 or a combination thereof. It is furthermore preferred that said carbohydrate containing part of a CD45 molecule is soluble.

It was further found in the present invention that MUC-1 molecules containing terminal GalNAc residues bind MGL. Thus, the invention further provides a method for providing an MGL positive cell with a binding molecule comprising providing said cell with a terminal GalNAc containing part of a MUC-1 molecule. It was further found that a molecule that was artificially provided with one or more C-terminal GalNAc residues (a neo-glycoconjugated molecule) binds MGL. Means and methods for producing a neo-glycoconjugated molecule are known in the art. Another, novel method therefore is provided in example 5. Therefore, the invention provides a method for generating an activated carbohydrate with a free maleimide moiety, the method comprising providing a bi-functional crosslinker comprising a hydrazide moiety and a maleimide moiety, coupling a carbohydrate to the hydrazide moiety of said crosslinker, and stabilizing the resulting imine group with a mild reductant.
In this one pot reductive amination reaction, the hydrazide moeity of the crosslinker is covalently coupled to the reducing terminus of the carbohydrate since the carbohydrate has a free reducing terminus which is in equilibrium between the ring closed (cyclic) and ring open (acylic) forms. The hydrazide group of the crosslinker performs a nucleophilic attack on the carbonyl carbon of the acyclic reducing terminal residue to form a partially stable Schiff's base. The Schiff's base imine group is subsequently chemically reduced with a mild reductant to give a stable carbohydrate-derivate The resulting stabilized activated carbohydrate can be purified with any method known in the art, such as gel filtration. After said coupling and purifying, the activated carbohydrate structure can be stored indefinitely at minus -20. A preferred bi-functional crosslinker is (4-N-maleimidophenyl)butyric acid hydrazide.

The invention also provides an activated carbohydrate obtainable by the method of the invention. Said activated carbohydrate is ready-to-use and can be coupled to any protein of interest.

The invention further provides a method for modifying a cysteine residue or a lysine residue of a polypeptide with a carbohydrate, comprising providing the activated carbohydrate of the invention, and coupling said activated carbohydrate with a cystein-residue or lysine residue in said polypeptide. Modification of a cysteine residue can be performed by reaction of said activated carbohydrate at neutral pH with the side chain of cysteines (thiols). Modification of a lysine residue can be performed by changing the pH of the reaction.

Preferably, said molecule provided with said terminal GalNAc residues is a peptide. A preferred peptide is a polypeptide. A preferred polypeptide has a molecular weight of less than 100 kiloDalton (kD), more preferred less than 10 kD, more preferred less than 5 kD, more preferred less that 1 kD. It is furthermore preferred that said polypeptide is soluble in an aquous solution and has a pharmaceutically acceptable half life. Said polypeptide can be modified by for example PEGylation, the addition of polyethyleneglycol, to improve pharmaceutically relevant parameters such as shielding of antigenic and immunogenic epitopes, and preventing recognition and degradation by proteolytic enzymes, as is known to a skilled artisan.

Providing an MGL positive cell with said binding molecule comprising a carbohydrate containing part of a CD45 molecule, MUC-1 or a terminal GalNAc containing part of a MUC-1 molecule, or a terminal GalNAc containing molecule such as a neo-glycoconjugated molecule allows modulation of APC signalling and thereby modulation of various aspects of the immune system, including alteration of a T-cell response of a subset of T-cells, inhibition of T-cell receptor-mediated signalling, and induction of apoptosis in a T-cell wherein a TCR_mediated signalling pathway is activated.

Providing an MGL positive cell with said binding molecule comprising a carbohydrate containing part of a CD45 molecule, MUC-1 or a terminal GalNAc containing part of a MUC-1 molecule, or a terminal GalNAc-containing molecule such as a neo-glycoconjugated molecule, and/or an antibody specific for MGL or a functional part, derivative and/or analogue thereof allows modulation of APC signalling and thereby modulation of various aspects of the immune system, including but not limited to alteration of a T-cell response of a subset of T-cells, inhibition of T-cell DC maturation, antigen presentation and cytokine release -mediated, and/orinduction of apoptosis in a T-cell wherein a TCR_mediated signalling pathway is activated. In one embodiment, said modulation results in altered cytokine production, such as for example the production of interleukins, such as IL6, IL-10, IL-12p70, IL-27, IL-23p19 and signalling molecules such as interferon alpha, interferon gamma, TGFβ and TNFα. Said modulation also comprises enhanced antigen presentation of a MGL-positive cell to specific T cells, resulting in, for example, enhanced proliferation of said T cells and thus making MGL a suitable target in immunization and vaccination protocols.
Said modulation furthermore can lead to DC maturation, macrophage activation, and/or functional modification of the DC or macrophage phenotype resulting in an alteration of a Th2-inducing capacity. Said alteration of a Th2-inducing capacity provides an alternative strategy for treatment of autoimmune diseases, pro-inflammatory responses, anti-inflammatory responses, and allergic responses such as asthma.

In one aspect, said method for modulating an immune response in a subject preferably comprises initiating or enhancing an immune response.

As binding of a binding molecule to MGL on antigen presenting cells induces and/or stimulates presentation of antigen by said cells, said immune response is preferably induced and/or stimulated. In a preferred embodiment an antigen specific immune response is initiated or enhanced. This aspect of the invention can be directed toward specific antigens by providing said antigen presenting cells with the appropriate antigen. To this end it is preferred that said antigen is linked to said molecule or said complex thereby directing said antigen to MGL positive antigen presenting cells. In this aspect antigen is directed to the MGL positive antigen presenting cells, thereby enabling and/or enhancing presentation of said antigen by said antigen presenting cell.
It has been found that antigen presentation of antigens that are provided to the antigen presenting cell via MGL is enhanced in matured or upon maturation of the antigen presenting cell. Maturation can be stimulated in various ways. In a preferred embodiment of the invention maturation of antigen presenting cells is stimulated through administering to said subject a composition for stimulating DC-maturation and/or activation. Various agents and maturation stimulating compositions and compounds are known to the person skilled in the art. In a preferred embodiment said compound and/or composition for stimulating DC maturation comprises an anti CD40 molecule, a Toll-like receptor ligand, a pro-inflammatory cytokine such as Interleukin-1, IL-6, tumor necrosis factor alpha, and/or prostaglandin E-2, an general adjuvant such as Montanide adjuvant or Freund adjuvant, or in situ maturation through the injection of immature DCs into adjuvant-treated skin. These compounds/compositions are particularly effective in inducing and/or stimulating an immune response against an also provided antigen in said subject. The invention further provides a vaccine comprising a binding molecule comprising a terminal GalNAc residue and/or a binding molecule specific for MGL, and an antigen against which the immune response is to be directed. Said vaccine can be used to immunize of tolerize for said antigen, depending on the presence or absence of DC maturation or an agent that modulates the maturation of DC.

The invention provides a method for modulating an immune response in a subject, said method comprising administering to said subject said binding molecule. In one embodiment, said binding molecule comprises a terminal GalNAc residue, an anti-MGL antibody, or an MGL-binding part of a CD45 molecule, MUC-1 or a terminal GalNAc containing part of a MUC-1 molecule, or a terminal GalNAc-containing molecule such as a neo-glycoconjugate.

The invention further provides a method for modulating an immune response in a subject wherein said modulation comprises dampening said immune response. It has been found that immature DC efficiently take up antigen via MGL and that this presentation leads to a dampening of an immune response in said subject. This dampening effect can lead to the induction of tolerance in said subject. The dampening effect is more pronounced when no further composition for stimulating and/or inducing an immune response is provided to said subject. Thus preferably said method comprises administering to said subject said binding molecule in the absence of a composition for stimulating maturation of DC. The dampening effect of said binding molecule or complex comprising said binding molecule is enhanced when said subject is also administered afurther composition for dampening an immune response in said subject. Preferably said further composition comprises steroid, preferably a corticosteroid, preferably a glucocorticosteroid. In a preferred embodiment said steroid comprises cortisol, predisone, hydrocortisone and/or dexamethasone.

In a further embodiment the invention comprises a method for modulating an immune response comprising stimulating the Th2-inducing capacity in said subject. In another preferred embodiment modulation of said immune response comprises altering cytokine release by MGL positive antigen presenting cells. As mentioned herein above said modulation is preferably achieved with a binding molecule comprising a terminal GalNAc, an anti-MGL antibody, or an MGL-binding part of a CD45 molecule, MUC-1 or a terminal GalNAc containing part of a MUC-1 molecule, or a terminal GalNAc-containing molecule such as a neo-glycoconjugated molecule. In a preferred embodiment, said MGL specific binding molecule comprises an antibody or a fragment or analogue thereof.

In a further embodiment the invention comprises a method for modulating an immune response wherein said molecule and/or said complex further comprises a toxic moiety and/or immune suppresive agent. In this embodiment MGL positive cells are provided with said toxic moiety thereby counteracting the effect of said MGL positive cell in a subject.
In a further embodiment said method for modulation of said immune response comprises altering and preferably stimulating MHC-I and/or MHC-II presentation by antigen presenting cells. Preferably of an antigen also provided.
The methods for modulating an immune response as described herein above are all related to an immune response in a subject. It is also possible to obtain the mentioned effects in cultures of antigen presenting cells and T-cells in vitro using the mentioned binding molecules and/or additional measures.

In a preferred embodiment, said modulation results in altered cytokine release, altered antigen presentation, and/or altered DC maturation and macrophage activation. Altered cytokine release results, for example, in the production of interleukins, such as IL6, IL-10, IL-12p70, IL-27, IL-23p19 and signalling molecules such as interferon alpha, interferon gamma, TGFβ and TNFα. Altered antigen presentation, preferably enhanced antigen presentation, of a MGL-positive cell to specific T cells, results in, for example, enhanced proliferation of said T cells and thus making MGL a suitable target in immunization and vaccination protocols.

Therefore, the invention provides a method for providing an MGL positive cell with a binding molecule of the invention for enhancing antigen presentation in an immunization and/or vaccination protocol.

Said modulation furthermore can lead to DC maturation, macrophage activation, and/or functional modification of the DC or macrophage phenotype resulting in an alteration of the Th2-inducing capacity. Said alteration of a Th2-inducing capacity provides an alternative strategy for treatment of autoimmune diseases, pro-inflammatory responses, anti-inflammatory responses, and allergic responses such as asthma.

The invention further provides a method for preferentially marking immature and/or tolerogenic professional antigen presenting cells (APC) in a sample comprising marking said cells with a binding molecule specific for MGL. In one embodiment, said binding molecule comprises an anti-MGL antibody, or an MGL-binding part thereof. A preferred antibody is a Tn-specific antibody, that recognizes the blood cell epitope T-nouvelle (Tn).
Alternatively, said binding molecule comprises one or more terminal GalNAc residues. In one aspect this embodiment is used to purify said immature and/or tolerogenic professional antigen presenting cells (APC) from a collection of cells.

In another aspect, the invention provides a protein selected from a fusion protein comprising CD45, the RA, RB, RC part of CD45, MUC-1, or a terminal GalNAc containing part of a MUC-1 molecule. The invention furthermore provides the use of said fusion protein or of a neo-glycoconjugated protein for elucidating an immune response against said fusion protein or neo-glycoconjugated protein. Peptides derived from said fusion protein or neo-glycoconjugated protein will be presented by an MGL-positive APC upon contact of an APC with the fusion protein or neo-glycoconjugated protein. Said presentation can be more effective than presentation of peptides derived from a protein without GalNAc.
Furthermore, said presentation is specific for said protein. In a preferred embodiment, said fusion protein or neo-glycoconjugated protein comprises an antigen. An antigen is any substance that causes the production of antibodies or the induction of effector cells by the immune system. A preferred antigen is provided by a bacterial or viral protein or an antigenic part thereof. Another preferred antigen is provided by a tumor protein, or part of a tumor protein.
A tumor protein is a protein that is expressed in a tumor cell, such as, for example, MUC-1, EpCAM and ERBB2 (HER2/Neu). Another preferred antigen is a tumor-specific antigen, resulting from a tumor specific mutation, or a tumor-associated antigen. Yet a further preferred antigen is provided by a string-of-beads comprising multiple tumor-associated cytotoxic T lymphocyte epitopes (Toes et al., 1997 PNAS 94: 14660-65).

Stimulating an immune response is preferably performed on mature DC. If required, DC can be matured, for instance, by adding TLR ligand or anti-CD40 in combination with said fusion protein or neo-glycoconjugated protein.

In another embodiment, the invention provides a liposome comprising a terminal GalNAc sugar residue that is able to bind to a MGL-positive APC. Liposomes are often used for drug delivery. Targeting said liposomes to the MGL receptor can enhance the endocytosis of said liposomes and facilitate DC-specific targeting of liposomes.

As the present invention established the various effects of MGL-CD45 mediated signalling the invention further provides a method for altering cell-cell mediated signalling in a culture of T-cells and APC, comprising altering MGL-CD45 mediated signalling in said collection of cells.
Said MGL-CD45 mediated signalling is preferably altered by providing said culture with a molecule capable of blocking said interaction. A preferred molecule capable of blocking said interaction is GalNAc or a molecule comprising a terminal GalNAc residue. Another preferred example is a carbohydrate binding part of MGL, HPA, a terminal GalNAc specific antibody and/or EGTA. In a preferred embodiment said signalling is blocked for stimulating an immune response against an antigen. Blocking the interaction at least reduces the tolerogenic action of MGL positive cells. In this embodiment it is preferred that said molecule capable of blocking said interaction further comprises said antigen. In this way it is possible to stimulate the immune response against said antigen. The invention further provides a method for stimulating an immune response against an antigen of a pathogen or an auto-antigen in a subject comprising providing said subject with a molecule capable of blocking the interaction of MGL with CD45.

In the present invention it was further found that tumour cells of epithelial origin contain MUC-1 molecules that have terminal GalNAc residues that are recognized by MGL. The invention thus further provides a method for detecting tumour cells of epithelial origin comprising contacting a sample comprising said cells with a carbohydrate binding part of a macrophage galactose-type lectin (MGL) and detecting the presence of cells that have said carbohydrate binding part. Said part, when linked to a toxin or an otherwise cell death promoting compound said MGL can also be used to selective kill said tumour cells of epithelial origin. Thus the present invention further provides use of a carbohydrate binding part of MGL for the preparation of a composition for the detection and/or selective killing of tumour cells of epithelial origin. In a preferred embodiment said tumour cells comprises colon carcinoma cells. In a further preferred embodiment, said tumour cells comprise primary tumour cells.

The invention further provides the use of a binding molecule specific for a terminal GalNAc residue for detecting MUC-1 in sample containing body fluid. In a preferred embodiment said body fluid comprises blood, more preferably serum. Also provided is a method for diagnoses and prognosis of a individual suffering from a epithelial tumour, preferably a colon and/or breast tumour, comprising contacting a sample comprising body fluid, preferably serum, with a binding molecule specific for a terminal GalNAc residue and detecting binding thereof to MUC-1. In a preferred embodiment said binding molecule comprises a carbohydrate binding part of MGL.

The mentioned binding molecule specific for a terminal GalNAc residue is preferably used for the preparation of a composition for binding CD45 positive T-cells in an animal body. Such compositions are preferably used for at least inhibiting a T-cell mediated immune response in said animal body. Preferably said animal is a human.

In yet another embodiment, the invention provides a protein comprising a carbohydrate recognition domain of MGL. Preferably said MGL is human MGL or mouse MGL2. In a preferred embodiment said protein is not MGL-Fc. As MGL is normally associated with the cellular membrane, the extracellular part containing said carbohydrate recognition domain is preferably modified such that it rendered soluble. There are a number of ways in which membrane associated proteins can be rendered soluble. Often this is done by linking said extracellular part to the constant part of an immunoglobulin. In a preferred embodiment said carbohydrate recognition domain of MGL is connected to a multimerisation domain. Such a configuration closely mimics the cellular situation where MGL is at least in part present in multimers. This allows more efficient binding of the carbohydrate binding part of MGL to the GalNAc containing target molecule.
In a preferred embodiment said multimerisation domain is a dimerisation, trimerisation or tetramerisation domain. As a foreign protein is linked to said carbohydrate recognition domain of MGL, the resultant protein is a chimeric molecule. Preferred immunoglobulin constant regions or multimerization domains are mouse IgG2A Fc and human IgG1-Fc. In a preferred embodiment said protein is MGL-Fc.

The invention further provides protein comprising a carbohydrate recognition domain of MGL and multimerisation domain for use as a medicament.

The invention further provides use of a binding molecule specific for a terminal GalNAc residue in the manufacture of a medicament for the prophylaxis or treatment of chronic inflammatory conditions, autoimmune diseases, and/or unwanted inflammatory responses in transplantation settings. Further provided is the use of a binding molecule specific for a terminal GalNAc residue in the manufacture of a medicament for the prophylaxis or treatment of T cell leukaemia. Preferably said binding molecule comprises a carbohydrate binding part of MGL. Preferably said MGL is human MGL. In another preferred embodiment said binding molecule specific for a terminal GalNAc residue comprises an antibody specific a terminal GalNAc residue. Preferably an antibody specific for Tn-antigen.

In yet another embodiment, the invention provides an *in vitro* method for diagnosis and/or prognosis of chronic inflammatory conditions, autoimmune diseases, and/or unwanted inflammatory responses in transplantation settings, said method comprising the step of allowing a binding molecule specific for a terminal GalNAc residue, preferably comprising a carbohydrate recognition domain of MGL, to bind to effector T cells. Further provided is an *in vitro* method for diagnosis and/or prognosis of a tumour, said method comprising the step of allowing a binding molecule specific for a terminal GalNAc residue, preferably comprising a carbohydrate recognition domain of MGL, to bind to tumour cells. Also provided is an *in vitro* method for diagnosis and/or prognosis of T cell leukaemia, said method comprising the step of allowing a binding molecule specific for a terminal GalNAc residue, preferably comprising a carbohydrate recognition domain of MGL, to bind to T cell leukaemia cells. In another preferred embodiment said terminal GalNAc specific binding molecule comprises an antibody. Preferably said antibody is a Tn-specific antibody.

It has been found that MGL detects elevated levels of CD45 positive cells in blood and synovial fluid of patients suffering from chronic inflammation. Further provided is thus a method for the detecting CD45 positive cells in an individual suffering from chronic inflammation comprising contacting a carbohydrate binding part of MGL with a blood sample or a synovial fluid sample of said individual and detecting binding of said carbohydrate part to cells in said sample. In a preferred embodiment said chronic inflammation comprises multiple sclerosis, arthritis, preferably rheumatoid arthritis or osteoarthritis.

Since MGL is among others expressed as a trimeric protein, it is preferred that the carbohydrate recognition domain of MGL is coupled to a multimerisation domain as to create multimers. MGL is preferably a human MGL.

Therefore, the present invention further relates to a chimeric protein comprising a carbohydrate recognition domain of MGL and a multimerisation domain, preferably separated by means of a spacer. In an embodiment, MGL-Fc is excluded. The carbohydrate recognition domain of MGL is located in the 141 C-terminal amino acids, i.e. amino acids 152-292. Therefore, preferably the C-terminal 141 amino acids of MGL are present in the chimeric protein.

"Carbohydrate recognition domain" as used herein refers to the protein domain that is responsible for recognition of and binding to terminal GalNAc structures on a carbohydrate structure. A "carbohydrate" as used herein refers to a sequence of one or more sugars linked in any way possible. "Multimerisation domain" as used herein refers to an amino acid sequence that induces multimerisation. Such multimerisation domain may be a dimerisation domain, a trimerisation domain, a tetramerisation domain, and the like. Any multimerisation domain may be used. In an embodiment, the multimerisation domain is derivable from a soluble protein. Examples of a multimerisation domain that can be used in the present invention are the Fc chain, His -tag, or Avi-tag.

The carbohydrate recognition domain of MGL may be separated from the multimerisation by means of a spacer. A spacer may be any amino acid sequence allowing the carbohydrate recognition domain and the multimerisation domain to perform their function, i.e. recognition of and binding to a carbohydrate region comprising terminal GalNAc structures and multimerisation, respectively.

In an embodiment, the multimerisation domain is a dimerisation, trimerisation or tetramerisation domain. MGL is expressed as a trimer in its natural environment. The present inventors have found that a dimeric form of MGL (MGL-Fc) also recognizes and binds to carbohydrates comprising terminal GalNAc structures.

In another embodiment, the multimerisation domain is mouse IgG2A Fc. In a further embodiment, the multimerisation domain is IgG1-Fc, preferably human IgG1-Fc. IgG1-Fc is a dimerisation domain that when used in the present invention yields an active chimeric protein. In other preferred cases said Fc is a human Fc. In cases wherein no complement-mediated killing and/or cell death of the terminal GalNAc containing cell is desired it is preferred that said Fc is an Fc that is deficient in complement activation.
Preferably said deficient Fc is an IgG4 Fc or a mutant IgG1.

The present invention also relates to a DNA molecule encoding such chimeric protein, a gene construct comprising such DNA molecule, and a host cell comprising such gene construct.

In a further aspect the present invention relates to a chimeric protein comprising the carbohydrate recognition domain of MGL and a multimerisation domain for use as a medicament. Hitherto, no therapeutical implication for such chimeric protein, including MGL-Fc, has been envisaged and described.

In another aspect, the present invention relates to the use of a chimeric protein comprising the carbohydrate recognition domain of MGL and a multimerisation domain in the manufacture of a medicament for the prophylaxis or treatment of chronic inflammatory conditions, autoimmune diseases, and/or unwanted inflammatory responses in transplantation settings.

In yet another aspect, the present invention is directed to the use of a chimeric protein comprising the carbohydrate recognition domain of MGL and a multimerisation domain in the manufacture of a medicament for the prophylaxis or treatment of T cell leukaemia. The use of such chimeric protein such as MGL-Fc may induce apoptosis of the leukaemic T cells, thereby providing a system to manage the disease.

For use in a medicament, said chimeric protein can be combined with any suitable carrier, diluent, adjuvant, excipient, etc. in order to obtain the medicament in the desired administration form. Advantageously, said medicament is administered intravenously.

For the intended use, the chimeric protein according to the invention may be administered alone or in admixture with a pharmaceutically acceptable carrier, in suitable pharmaceutical formulations that are a further object of the invention.

Examples of said formulations, which may be prepared using well known methods and excipients, such as those described in "Remington's Pharmaceutical Sciences Handbook", Mack Pub. Co., N.Y. U.S.A., are tablets, capsules, syrups, and the like for oral administration, whereas for the parental administration suitable forms are sterile solutions or suspensions in acceptable liquids, implants, etc.

The posology will depend on several factors such as type and seriousness of the conditions to be treated, patient's weight and sex, etc. and will be easily determined by the skilled practitioner.

In yet a further embodiment, the chimeric protein is MGL-Fc, since it has been shown to have the activity desired for the present invention.

The immune system protects the body from potentially harmful substances (antigens). Harmful antigens are destroyed by the immune response. Immune system disorders occur when the immune response is inappropriate, excessive, or lacking.

Chronic inflammatory diseases are diseases that represent an inflammatory response of prolonged duration whose extended time course is provoked by persistence of the causative stimulus to inflammation in the tissue. Non-limiting examples of chronic inflammatory diseases are tuberculosis, chronic cholecystitis, bronchiectasis, rheumatoid arthritis, (Hashimoto's) thyroiditis, inflammatory bowel disease (ulcerative colitis and Chrohn's disease), silicosis and other pneumoconioses, and an implanted foreign body in a wound.

Autoimmmune diseases develop when the immune system destroys normal body tissues. Normally, the immune system is capable of differentiating "self" from "non-self" antigens. In autoimmune diseases the normal control process is disrupted and the immune system cannot differentiate anymore between "self" and "non-self" antigens. Non-limiting examples of autoimmune diseases are Graves' disease, Hashimoto's disease, multiple sclerosis, rheumatoid arthritis, systemic lupus erythematosus, pernicious anemia, Addison's disease, type I diabetes, dermatomyositis, Sjogren's syndrome, lupus erythematosus, myasthenia gravis, and Reiter's syndrome.

One of the most serious problems facing transplant patients has been the possibility that their own bodies will try to reject or destroy the transplant. Rejection is part of the body's natural reaction to foreign invaders, as also indicated above. To reduce the risk of rejection, physicians try to find donors whose MHC antigens are as genetically close to those of the recipient as possible. Nevertheless, most transplants, with the exception of those donated by identical twins, are recognized by the patient's immune system as foreign. Rejected transplants need to be surgically removed, and if the transplant is a life-sustaining organ such as a lung, liver, or heart, a patient may die before a replacement organ is found. Since a chimeric protein according to the present invention reduces the cytokine production and induced T cell death, unwanted inflammatory responses in transplantation settings may be prevented by therapeutical application of such chimeric protein.

In a further aspect, the present invention relates to an *in vitro* method for diagnosis and/or prognosis of chronic inflammatory conditions, autoimmune diseases, and/or unwanted inflammatory responses in transplantation settings, said method comprising the step of allowing a chimeric protein, said chimeric protein comprising the carbohydrate recognition domain of MGL and a multimerisation domain, to bind to effector T cells.

In a further aspect, the present invention relates to an *in vitro* method for diagnosis and/or prognosis of a tumour, said method comprising the step of allowing a chimeric protein, said chimeric protein comprising the carbohydrate recognition domain of MGL and a multimerisation domain, to bind to tumour cells. In a further aspect, the present invention relates to an *in vitro* method for diagnosis and/or prognosis of T cell leukaemia, said method comprising the step of allowing a chimeric protein, said chimeric protein comprising the carbohydrate recognition domain of MGL and a multimerisation domain, to bind to T cell leukaemia cells. To this end, cells obtained from a subject are investigated *in vitro* to provide a diagnosis and/or prognosis for the above-mentioned diseases.
Suitable diagnostic systems include ELISA-based tests
CD45 glycosylation and function

In the present invention we have identified the CLR MGL as the receptor expressed by professional APC for the tyrosine phosphatase CD45.
The mucin-type protein CD45 contains high amounts of O-linked carbohydrates, mainly localized in the differentially expressed A-, B-, and C-domains15. MGL clearly recognized all A-, B- and C-containing isoforms, whereas CD45RO was not bound. CD45RAneg memory and effector T cells retain expression of the CD45B and CD45RO isoforms. As CD45RO is not recognized, MGL-mediated T cell binding is most likely mediated via the B-isoform. Recently we have elucidated the carbohydrate recognition profile of MGL and showed that MGL has an exclusive specificity for terminal GalNAc structures¹³. CD45RO has only two O-linked glycan structures, which are likely not enough to sustain MGL recognition. Using the α-GalNAc-specific snail lectin HPA we detected terminal α-GalNAc structures on CD45 pulled down by MGL from human T cell lysates (data not shown). On Jurkat T cells both CD45 and CD43 were recognized by MGL. Jurkat cells contain a mutation in the glycosyltransferase chaperone Cosmc, resulting in increased exposure of terminal α-GalNAc structures on O-linked glycoproteins, explaining the high affinity binding of MGL to Jurkat²⁹. In contrast, on human T cells, CD43 is heavily sialylated, which abrogates MGL recognition²². This indicates that native human T cells do not display the appropriate glycan structures on CD43.

Glycosylation is a cell-specific process, depending not only on the cellular composition of glycosyltransferases and glycosidases, but also on the activation and differentiation status of the cell¹⁹. Glycosylation can thus influence diverse aspects of the immune system, although single glycans can have a highly specific functions. CD45 glycosylation changes during T cell development, pheripheral activation and aging^{18,30}. Furthermore, different CD45 isoforms were shown to be differentially glycosylated¹⁶. Therefore differential glycosylation of CD45 provides a scaffold for CLR-based modulation of TCR-mediated responses. Apparently CD45 on Effector T cells (T-eff) exhibits the necessary glycan structures, due to cell-specific glycosylation, to ensure MGL recognition.

Two putative counter-receptors, Galectin-1 and CD22, for human CD45 have been identified, however their exact role in regulating CD45 is still unclear^{31,32}. Although some controversy exists whether CD45 activity can be controlled via receptor-mediated mechanisms¹⁵; we clearly demonstrate here that MGL on APC is capable of modulating CD45 phosphatase activity and function.
C-type lectin mediated immunomodulation

Every professional APC expresses a wide variety but unique repertoire of CLR⁶. Here we show that the CLR MGL and DC-SIGN are localized on different APC in skin and LN, whereas in lamina propria of the jejunum they are co-expressed on the same APC. In addition, MGL is expressed on in vitro monocyte-derived DC and Macrophages (Mf). APC cultured with dexamethasone display an enhanced and prolonged MGL expression. Mature DC have lost all MGL surface expression.

Most CLR clear and internalize self-antigens for presentation under steady state conditions without causing overt DC maturation, thereby actively induce tolerance towards sequestered self-antigens⁷. In contrast, crosslinking of the CLR Dectin-1 induces intracellular signaling pathways leading to production of inflammatory mediators such as reactive oxygen species and TNFα¹⁰. Whereas CLR-mediated signaling modulates APC function, MGL is involved in regulation of the responder T cell. MGL preferentially recognized antigen-experienced CD27neg T-eff, which are characterized by high levels of pro-inflammatory cytokine production and tissue homing properties²⁷. T-eff no longer require extensive co-stimulation, antigen recognition is sufficient for these cells to be activated³³. They are found in high numbers in chronic inflammatory diseases and parasitic infections. Strikingly, during these conditions MGL expression is equally upregulated¹¹. Upregulation of MGL expression during RA disease course could therefore be a self-protecting mechanism initiated to prevent excessive inflammation and tissue damage. Via the MGL-CD45 interaction tolerogenic APC, expressing low levels of co-stimulatory molecules are able to suppress TCR-mediated T cell activation, resulting in reduced cytokine production and proliferation, providing another mechanism for APC to control unwanted effector responses. Likewise, LPS-matured DC that initiate adaptive T cell immunity, have no need for receptors with a downregulatory function, explaining why mature DC are essentially MGLneg. The overall negative regulatory effect of MGL on effector T cell activation, together with its expression profile, suggests that MGL controls unwanted effector T cell activation in the steady state or during chronic inflammation. Therefore MGL contributes to the maintenance of tolerance by controlling excessive immune responses.
CD45-mediated immunomodulation

All CD45 splice variants have similar phosphatase activities; still the different isoforms show very restricted expression patterns³⁴. How can changes in extracellular CD45 isoforms modulate CD45 function? One model proposes that homodimerization inhibits phosphatase activity through interactions between an inhibitory structural wedge and the catalytic site within the cytoplasmic CD45 phosphatase domains³⁵. However, the recently published crystal structure of CD45 seems incompatible with the wedge model³⁶. Another straightforward explanation is receptor-mediated regulation of CD45 phosphatase activity. MGL specifically recognizes CD45 depending on the isoform and the cellular context in which this isoform is expressed.
Thus, glycosylation patterns of single cells determine whether MGL is able to interact with and modulate CD45 function. Intriguingly, alternations in human CD45 splice variants are associated with autoimmune diseases, such as multiple sclerosis and systemic lupus erythromatosis^{37,34}. It would be interesting to investigate whether these mutations lead to altered glycosylation patterns, thus affecting MGL recognition and CD45 activity. Originally identified for its role in positively regulating antigen-receptor signaling via dephosphorylation of Src kinases, increasing evidence accumulates that CD45 can also negatively influence signaling pathways initiated by surface receptors³⁸. Several reports show that engagement of cell surface CD45 by monoclonal antibodies induced a programmed cell death pathway in lymphocytes³⁹⁻⁴¹. Moreover, CD45 regulates apoptosis in peripheral T cells of transgenic mice expressing one single CD45 isoform⁴².
Similar CD45-dependent apoptosis could be detected in Jurkat after MGL-Fc binding; however cell death was equally dependent on CD3-triggering, hinting to antigen-specific induction of cell death. Although we clearly demonstrate MGL to reduce T cell proliferation, so far we have not observed substantial apoptosis in human T cells after MGL-Fc/anti-CD3 triggering (data not shown), possibly due to required pre-activation status of the cells.
Some controversy exists regarding the requirement of CD45 phosphatase activity for eliciting cell death^{39,40} and a novel apoptotic pathway acting through the production of reactive oxygen species was proposed. Addition of reactive oxygen species scavengers could similarly block apoptosis induction in our system (data not shown). TCR-based signaling events can upregulate CD95 ligand expression, a process equally dependent on CD45⁴³. Accordingly, our combined TCR/MGL-Fc stimulation might result in enhanced CD95-mediated programmed cell death, as nuclear condensation is observed in both apoptotic pathways³⁹.

The unique activating and suppressive features of CD45 make it an attractive therapeutical target molecule. Especially anti-CD45RB antibodies potently inhibit T cell responses. In mice the CD45RBbrightCD4+ T cell population harbors T-eff, some of which are capable of inducing autoimmunity or inflammatory bowel disease^{44,45}. Treatment with anti-CD45RB monoclonal antibodies prolongs long-term allograft survival and donor-specific tolerance in several murine renal and islet transplantation models⁴⁶. An anti-human CD45RO/RB specific antibody effectively inhibited antigen-specific and polyclonal T cell responses, mediated apoptosis of CD4+CD45RO/RBbright T cells and induced type 1 T-regs in vitro⁴⁷. These IL-10 producing T-regs can likewise be generated by differentiating naive T cells in the presence of dexamethasone treated APC⁴⁸.

The observed effects with anti-CD45RB antibody therapy show remarkable similarities with the functional consequences of the MGL-CD45 interaction for T-eff. As CD45RB expression is retained on effector CD4+ T cells¹⁷ and MGL selectively recognizes all CD45RB containing isoforms, it is tempting to speculate that MGL is the natural receptor on APC capable of mediating such processes. To further investigate MGL-mediated immune suppression, MGL knockout mice could provide useful model systems.
However, in mice two functional copies of the MGL gene exist with carbohydrate recognition profiles distinct from human MGL¹⁴, suggesting that murine and human MGL possess different ligands and possibly immunological functions⁴⁹.

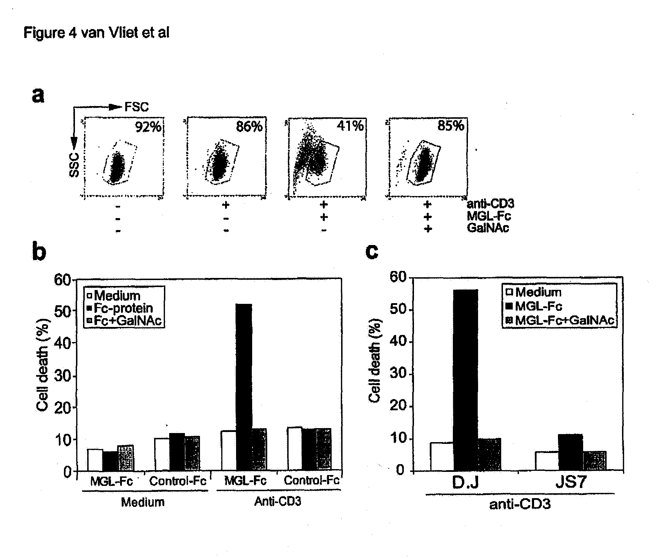

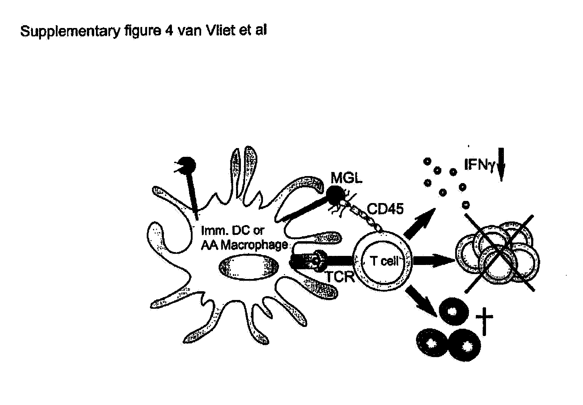

Taken together our results imply a model of MGL-mediated control of Teff function and demonstrate a unique and novel role for CLR on APC in the regulation of T cell homeostasis (Supplementary fig. 4). Compared to the anti-CD45 antibodies, MGL-Fc exhibits an enhanced specificity, targeting only those T-eff cells that have properly glycosylated CD45. In contrast anti-CD45RB antibodies bind and potentially affect all leukocytes that express the isoform, independent of cellular glycosylation patterns.

The invention further provides a method for providing cells with a specific binding molecule comprising providing said cells with a binding molecule specific for a terminal GalNAc residue. In a preferred embodiment said cells are human lymphatic endothelial cells. In antoher prefered embodiment said cells are human mammary epithelial cells. The invention further provides a method for providing human CD34 positive cells with a specific binding molecule said method comprising providing said cells with a binding molecule specific for MGL. Preferably said human CD34 positive cells are AML cells. Preferably said MGL binding molecule is an MGL specific antibody. In another preferred embodiment said binding molecule comprises a carbohydrate containing part of a CD45 or a MUC1 molecule as described herein above. In another preferred embodiment said MGL binding molecule is a binding molecule or complex comprising a terminal GalNAc residue as described herein above. Preferably said cells are provided with said binding molecule for marking said cells.

Breast milk is a rich source of glycosylated MUC1. In the present invention it was found that the carbohydrate content of MUC1 from healthy or allergic mothers exhibited differences in glycosylation that correlated with the disease status. The total MUC1 content did not vary significantly.
However, MUC1 from allergic mothers displays a higher MGL binding, which can be explained by the higher exposure of α-GalNAc epitopes as measured by the α-GalNAc-specific lectin HPA. MUC1 protein is also present in various infant formulas, particularly in those recommended for infants that are suffering from or at risk of suffering from an allergy. The invention now provides a method for preparing an infant food, preferably an infant formula or a protein containing component thereof comprising testing said food and/or said component for the presence of MUC1 comprising a terminal GalNAc residue (preferably an α-GalNAc epitope). The invention thus further provides an infant formula that is tested with a method of the invention. The number of α-GalNAc epitopes in an infant formula, particularly for the mentioned infants is preferably reduced. MUC1 is particularly prevalent in milk derived protein sources. The current processes for preparing milk derived protein source for infant formula favor the generation of MUC1 exhibiting α-GalNAc epitopes. These can be adapted to reduce the amount of α-GalNAc epitope on MUC1. Thus the invention further provides an infant formula comprising a protein source containing MUC1, preferably from milk, wherein the amount of α-GalNAc epitopes is reduced. Preferably, said infant formula is essentially free of α-GalNAc epitopes on MUC1. The invention further provides the use of such infant formula for feeding infants suffering from or at risk of suffering from an allergy. Preferably said α-GalNAc epitope comprises a terminal GalNAc residue.

In the present invention it is preferred that said MGL, CD45 and/or MUC1 protein and/or cells comprising said protein are human proteins and/or human cells.

The invention further provides the use of a terminal GalNAc specific antibody to bind to CD45 positive cells or MUC1 positive cells, preferably MUC1 positive tumor cells, preferably colon carcinoma cells. Preferably said antibody is used in a method for modulating an immune response in a subject. Preferably said antibody is a Tn-specific antibody.

An antibody is preferably a human, humanized or deimmunised antibody. An antibody may be produced by a cell or generated synthetically. In the present invention an antibody is said to be human when it is derived from a human cell, or the equivalent thereof. Suitable equivalents are for instance, transgenic mice comprising the human antibody locus or in vitro antibody (fragment) selection systems expressing (parts of) human antibodies. Non-limiting examples are phage libraries expression human single chain Fv fragments. A human antibody thus preferably comprises a sequence that has an equivalent sequence in a human. As CDR regions are typically generated in response to an antigen the equivalence typically does not extend to the CDR regions. A humanized antibody typically comprises the CDR regions of a non-human antibody grafted on a human constant region. The framework region is typically also equivalent to a human antibody. A deimmunized antibody comprises a variable region derived from an animal other than a human from which human T-cell epitopes in at least the framework, have essentially been removed through mutation of the original sequence.

The invention further provides a mouse MGL1 for use in detecting a lewis X antigen.

The invention further provides a method for providing a (poly)peptide with a carbohydrate residue, said method comprising producing a stable reactive carbohydrate intermediate.

The invention further provides a method for providing a CD45 positive cell with a binding molecule comprising providing said cell with a binding molecule specific for a terminal GalNAc residue. Preferably, said binding molecule comprises a carbohydrate binding part of macrophage galactose-type lectin (MGL).

The invention further provides a method for differentially marking CD45 positive cells, comprising contacting a collection of CD45 positive cells with a binding molecule specific for a terminal GalNAc residue, preferably a carbohydrate binding part of an MGL. In a preferred embodiment, said method is for the detection of chronic inflammation in an individual.

In a preferred embodiment, said cell is a CD45 positive T-cell. Preferably, said cell expresses a CD45 isoform comprising at least the RA, RB or RC part of CD45 or a combination thereof. Preferably, said method is used for altering a T-cell response of a subset of T cells. Preferably, said method is for inhibiting T-cell receptor (TCR)-mediated signalling. Preferably, said method is for inducing apoptosis in a T-cell wherein a TCR-mediated signalling pathway is activated. In a preferred embodiment, said method is for raising the activation threshold of a CD45 positive effector type T-cell. In a preferred embodiment, said method is for inhibiting proliferation of a CD45 positive effector type T-cell. In a preferred embodiment of the invention, said binding molecule comprises an antibody. Preferably, said antibody is a Tn specific antibody.

The invention further provides a method for providing an MGL positive cell with a binding molecule comprising providing said cell with a carbohydrate containing part, of a CD45 molecule or MUC-1 molecule.

Further provided is a method for modulating an immune response in a subject comprising providing said subject with a binding molecule comprising a terminal GalNAc residue or a complex comprising said binding molecule.

Preferably, said molecule or said complex comprises a (poly)peptide or lipid comprising said terminal GalNAc residue.

Further provided is a method for modulating an immune response in a subject, said method comprising administering to said subject an MGL specific binding molecule. Preferably, said MGL specific binding molecule comprises an antibody or a fragment or analogue thereof.

Preferred is a method for modulating an immune response in a subject, wherein modulating said immune response comprises initiating or enhancing an immune response. Preferred is a method, wherein an antigen specific immune response is initiated or enhanced. In a preferred embodiment, said antigen is linked to said molecule or said complex thereby directing said antigen to MGL positive antigen presenting cells.

In a preferred embodiment of a method for modulating an immune response in a subject, wherein modulating said immune response comprises initiating or enhancing an immune response, said method further comprises administering to said subject a composition for stimulating DC-maturation and/or activation. In a preferred embodiment, said composition for stimulating DC maturation comprises an anti-CD40 molecule, a Toll-like receptor ligand, a pro-inflammatory cytokine such as Interleukin-1, IL-6, tumor necrosis factor alpha, and/or prostaglandin E-2, a general adjuvant such as Montanide adjuvant or Freund adjuvant, or in situ maturation through the injection of immature DCs into adjuvant-treated skin.

In another preferred embodiment of a method for modulating an immune response in a subject, said modulation comprises dampening said immune response. Preferably, said damping comprises inducing tolerance. Preferably, said method comprises administering to said subject said binding molecule in the absence of a composition for stimulating maturation of DC.

In a preferred embodiment of a method for modulating an immune response in a subject, said method further comprises administering to said subject a further composition for dampening an immune response in said subject.
Preferably, said further composition comprises a steroid, preferably a corticosteroid.

In a preferred embodiment of a method for modulating an immune response in a subject, said modulation comprises stimulating the Th2-inducing capacity in said subject,

In a preferred method of modulating an immune response in a subject, modulation of said immune response comprises altering cytokine release by MGL positive antigen presenting cells.

In a preferred embodiment of a method for modulating an immune response in a subject, said molecule and/or said complex further comprises a toxic moiety and/or immune suppressive agent.

In a preferred embodiment of a method for modulating an immune response in a subject, said complex comprises a liposome.

In a preferred embodiment of a method for providing an MGL positive cell with a binding molecule comprising providing said cell with a carbohydrate containing part, of a CD45 molecule or MUC-1 molecule or a of method for modulating an immune response in a subject, said binding molecule comprises at least the RA, RB, RC part of CD45 or a combination thereof.
Preferably, said binding molecule is MUC-1 or a terminal GalNAc containing part of a MUC-1 molecule. In a preferred embodiment, said binding molecule is a glycoconjugate provided with said terminal GalNAc residue in vitro (a neo-glycoconjugate). In a preferred embodiment, said neo-glycoconiugate has been provided with said GalNAc residue in a cell free environment. More preferably, said neo-glyconjugate comprises at least 2 terminal GalNAc residues. Preferably, said terminal GalNAc residue is part of a LacdiNAc structure. Preferably, said terminal GalNAc residue is part of a Tn-structure.
Preferred is a method according to the invention for modulating functionality of said MGL-positive cell.

Further provided is a method for preferentially marking immature and/or tolerogenic professional antigen presenting cells (APC) in a subset of cells comprising marking said cells with a binding molecule specific for MGL.
Preferably, said binding molecule is an antibody or a MGL-binding part thereof. More preferably, said binding molecule comprises one or more terminal GalNAc residues.

Further provided is a protein selected from a fusion protein comprising CD45, the RA, RB, RC part of CD45, MUC-1 or a terminal GalNAc containing part of a MUC-1 molecule, and a neo-glycoconjugated protein. Preferably, said protein further comprises an antigen. Preferably, whereby said antigen is a tumor protein, a tumor-specific antigen, a tumor-associated antigen, or a string of beads comprising multiple tumor-associated epitopes.

Further provided is a liposome comprising a terminal GalNAc sugar residue.

Further provided is a method for altering cell-cell mediated signalling in a culture of T-cells and APC, comprising altering MGL-CD45 mediated signalling in said collection of cells. Preferably, said MGL-CD45 mediated signalling is altered by providing said culture with a molecule capable of blocking said interaction. Preferably said molecule is GalNAc or comprises a terminal GalNAc residue. In a preferred embodiment, said molecule comprises a carbohydrate binding part of MGL, HPA, a terminal GalNAc specific antibody and/or EGTA.

Further provided is a method for detecting tumour cells of epithelial origin comprising contacting a sample comprising said cells with a carbohydrate binding part of a macrophage galactose-tvpe lectin fMGL) and detecting the presence of cells that have bound said carbohydrate binding part.

Further provided is a use of a carbohydrate binding part of MGL for the preparation, of a composition for the detection of primary tumour cells of epithelial origin.

Further provided is a use of a binding molecule specific for a terminal GalNAc residue for the preparation of a composition for binding CD45 positive T-cells in an animal body.

Further provided is a protein comprising a carbohydrate recognition domain of MGL, said protein not being MGL-Fc. Preferably, said protein is a fusion protein comprising a carbohydrate recognition domain of MGL. Preferred is a protein according to the invention, further comprising a multimerisation domain. Preferably, the multimerisation domain is a dimerisation, trimerisation or tetramerisation domain. Preferably, the multimerisation domain comprises mouse IgG2A Fc.

Further provided is a protein comprising a carbohydrate recognition domain of MGL and preferably a multimerisation domain for use as a medicament.
Preferably said multimerisation domain is a dimerisation, trimerisation or tetramerisation domain. Preferably, said multimerisation domain is mouse IgG2A Fc. In a preferred embodiment, said protein comprises an extracellular domain of MGL fused at its C-terminus to a human Ig constant region, preferably a human IgGl-Fc. Preferably, said protein is MGL-Fc.

Further provided is a use of a binding molecule specific for a terminal GalNAc residue for detecting MUC-1 in sample containing a body fluid. In a preferred embodiment, said body fluid comprises blood.

Further provided is a use of a binding molecule specific for a terminal GalNAc residue in the manufacture of a medicament for the treatment of chronic inflammatory conditions, autoimmune diseases, and/or unwanted inflammatory responses in transplantation settings.

Further provided is a use of a binding molecule specific for a terminal GalNAc residue in the manufacture of a medicament for the prophylaxis or treatment of T cell leukaemia. In a preferred embodiment, said binding molecule comprises a carbohydrate binding part of MGL. Preferably, said binding molecule further comprises a multimerisation domain. Preferably, said binding molecule comprises an extracellular domain of MGL fused at its C-terminus to human IgG-Fc, preferably human IgGl-Fc. Preferably, said binding molecule comprises MGL-Fc.

Further provided is an *in vitro* method for diagnosis and/or prognosis of chronic inflammatory conditions, autoimmune diseases, and/or unwanted inflammatory responses in transplantation settings, said method comprising the step of allowing a binding molecule specific for a terminal GalNAc residue, preferably comprising a carbohydrate recognition domain of MGL, to bind to effector T cells, said method further comprising detecting said binding.

Further provided is an *in vitro* method for diagnosis and/or prognosis of a tumour, said method comprising the step of allowing a binding molecule specific for a terminal GalNAc residue, preferably comprising a carbohydrate recognition domain of MGL, to bind to tumour cells, said method further comprising detecting said binding.

Further provided is an *in vitro* method for diagnosis and/or prognosis of T cell leukaemia, said method comprising the step of allowing a binding molecule specific for a terminal GalNAc residue, preferably comprising a carbohydrate recognition domain of MGL, to bind to T cell leukaemia cells, said method further comprising detecting said binding.
Further provided is a use of a carbohydrate recognition domain of MGL for detecting terminal GaLNac containing CD45 positive cells in a sample, preferably T-cells.

Further provided is a use of a carbohydrate recognition domain of MGL for detecting terminal GaLNac containing MUC-1 in a sample of body fluid, preferably blood or a fraction thereof.

Further provided is a vaccine comprising a binding molecule comprising a terminal GaLNAc residue and/or a binding molecule specific for MGL, and an antigen against which the immune response is to be directed.

Further provided is a use of a binding molecule comprising a terminal GalNac residue and/or an antibody specific for MGL for the preparation of a composition for mobilizing antigen presenting cells, preferably dendritic cells.

Further provided is a method for mobilizing antigen presenting cells, preferably dendritic cells said method comprising providing a subject with a binding molecule comprising a terminal GalNac residue and/or an antibody specific for MGL, Preferably, said method further comprises harvesting said mobilized dendritic cells.

The invention will hereinafter be explained in more detail with reference to examples and drawings.

### Figure legends to the figures of example 1

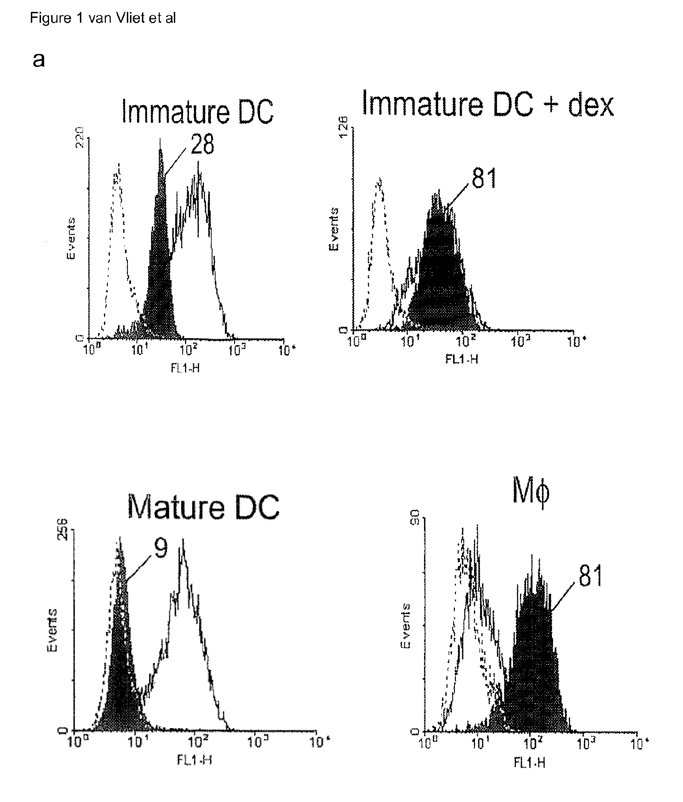

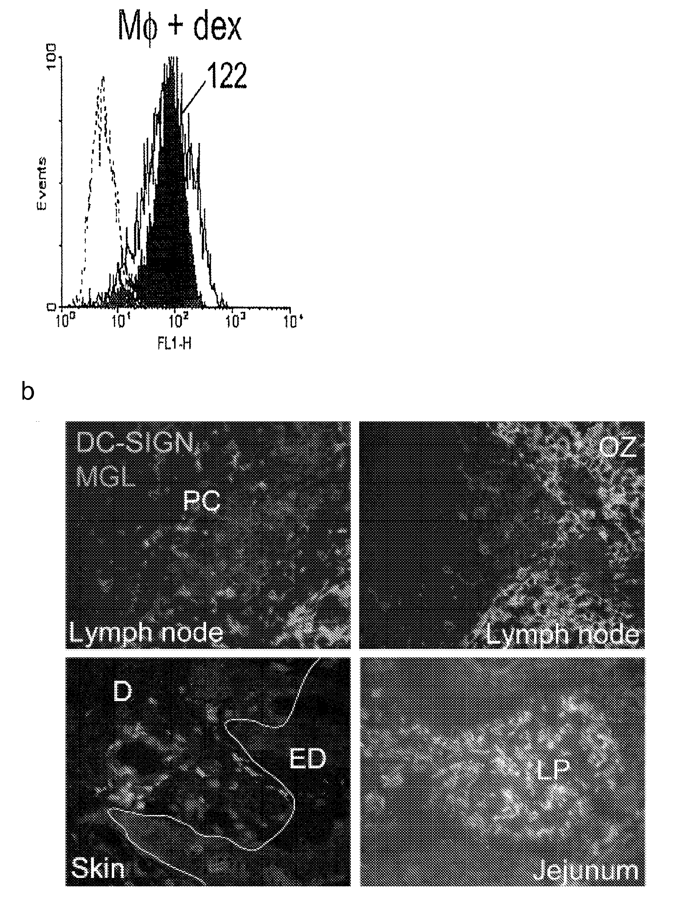

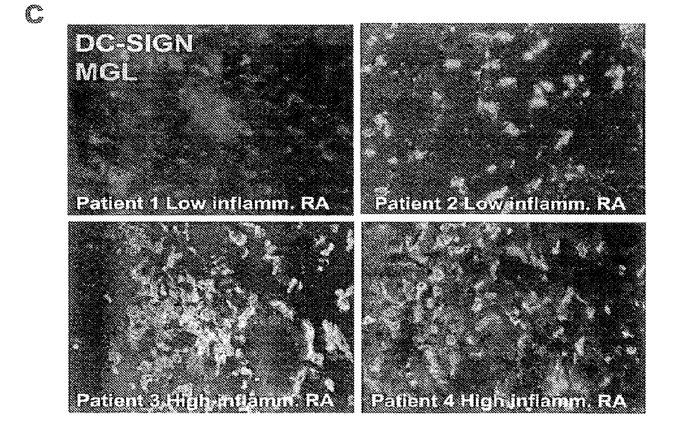

Figure 1 MGL expression in tissues and on in vitro generated APC. (a) MGL and DC-SIGN expression on monocyte-derived APC was determined by flowcytometry. The dotted line denotes the isotype control staining, the dark line shows DC-SIGN expression and the filled histogram represents MGL expression. Mean fluorescent intensities for MGL are indicated in the figure.
Results are representative for three independent experiments. (b) Human tissue sections of jejunum, lymph node (lung draining) and skin were stained with anti-MGL (red) and anti-DC-SIGN (green) antibodies. The red staining surrounding the jejunum LP is non-specific, due to background staining of the mucus layer. OZ, outer zone paracortex in proximity of the medullary and paracortical sinuses, PC, paracortex, ED, epidermis, D, dermis, LP, lamina propria. Original magnification 400x. (c) Human tissue biopsies of four RA patients were stained with anti-MGL (red) and anti-DC-SIGN (green) antibodies. Original magnification 400x.

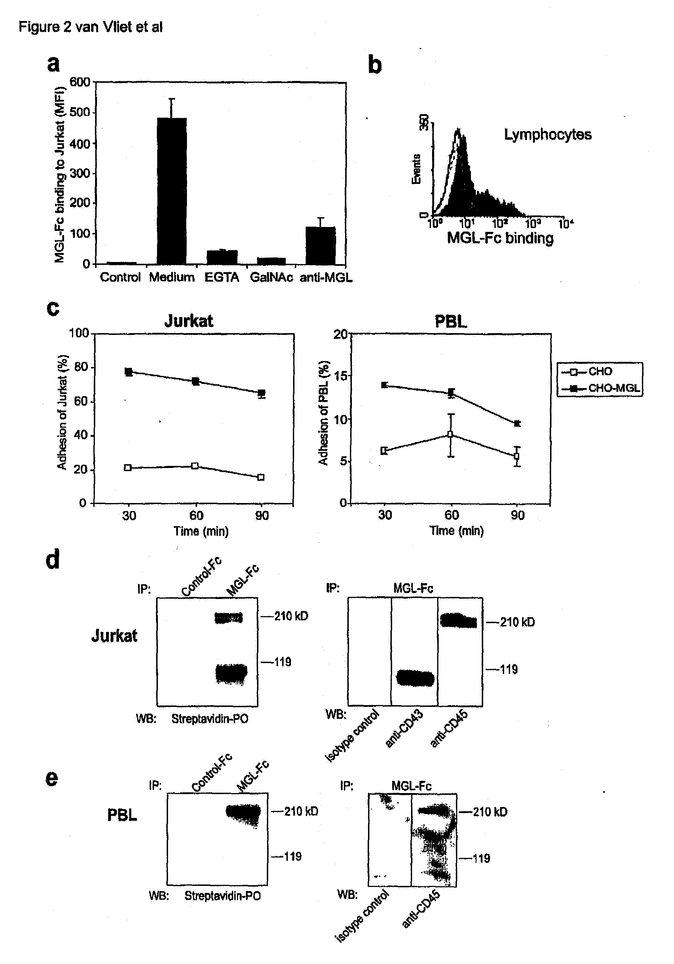

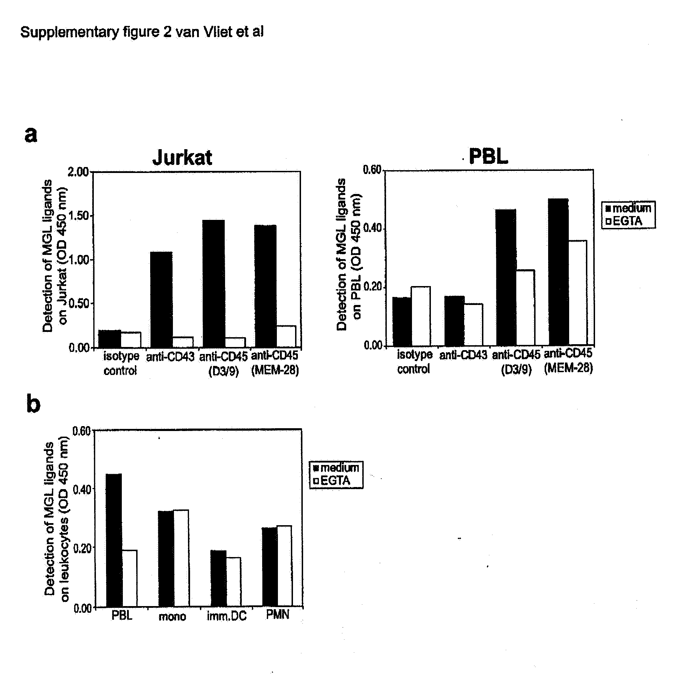

Figure 2 MGL recognizes CD45 on human lymphocytes. (a) MGL-Fc binds with high affinity to the T cell line Jurkat. MGL-Fc binding could be inhibited by the addition of EGTA (10 mM), free GalNAc (100 mM) or anti-MGL antibodies (20 µg/ml). (b) MGL-Fc recognizes a counterstructure on human PBL. Overlay represents control-Fc (dashed line), MGL-Fc (filled histogram) and MGL-Fc binding in the presence of 10 mM EGTA (solid line). One representative donor out of 20 is shown. (c) Jurkat and PBL adhere at higher levels to CHO-MGL compared to parental CHO, as measured in cellular adhesion experiments. (d) CD43 and CD45 are the counterreceptors for MGL on Jurkat. Surface-biotinylated Jurkat cells were lysed and immunoprecipitated with MGL-Fc or control-Fc. Immunoprecipitates were analyzed on immunoblot with streptavidin or with monoclonal antibodies to CD43, CD45 or LFA-1 (isotype control). IP, immunoprecipitation, WB, western blot detection (e) CD45 is the main counterreceptor for MGL on PBL.
Surface-biotinylated PBL were lysed and immunoprecipitated with MGL-Fc or control-Fc. Immunoprecipitates were analyzed on immunoblot with streptavidin or with monoclonal antibodies to CD45 or LFA-1 (isotype control). IP, immunoprecipitation, WB, western blot detection All results are representative for at least three independent experiments.

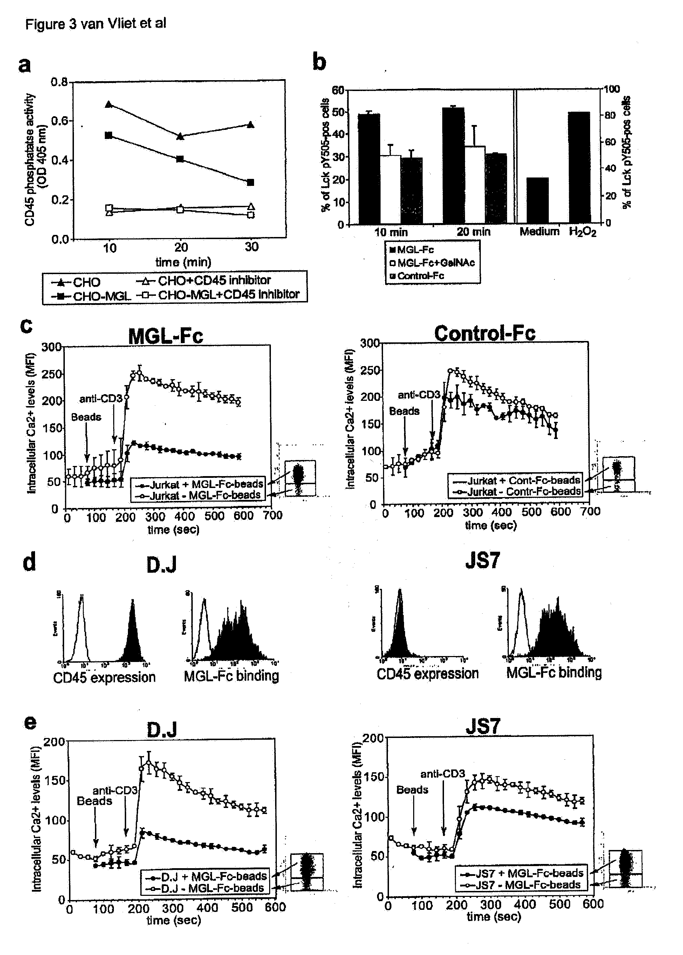

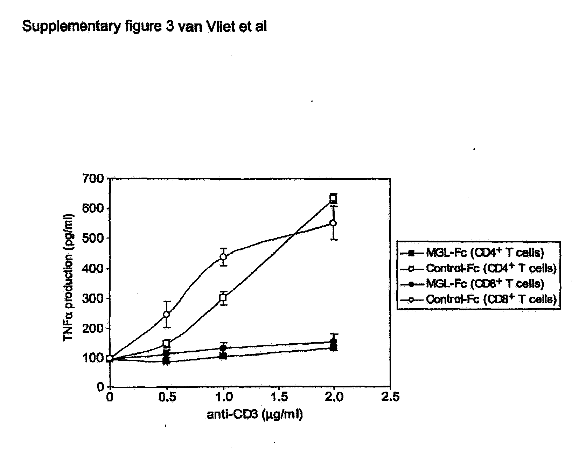

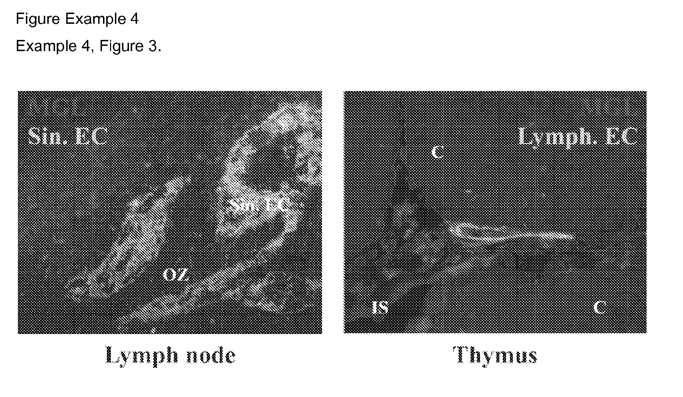

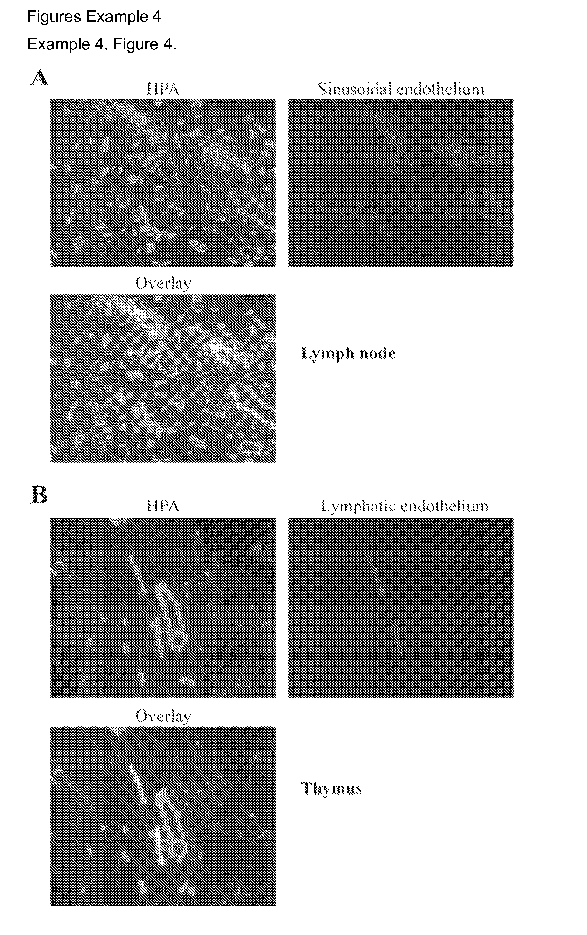

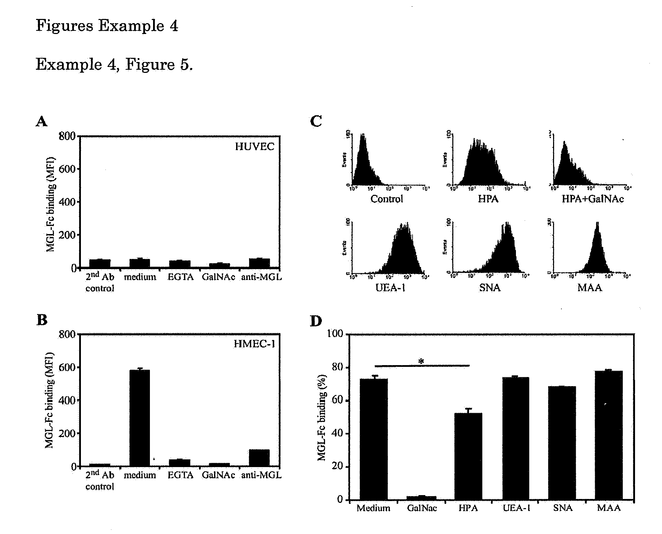

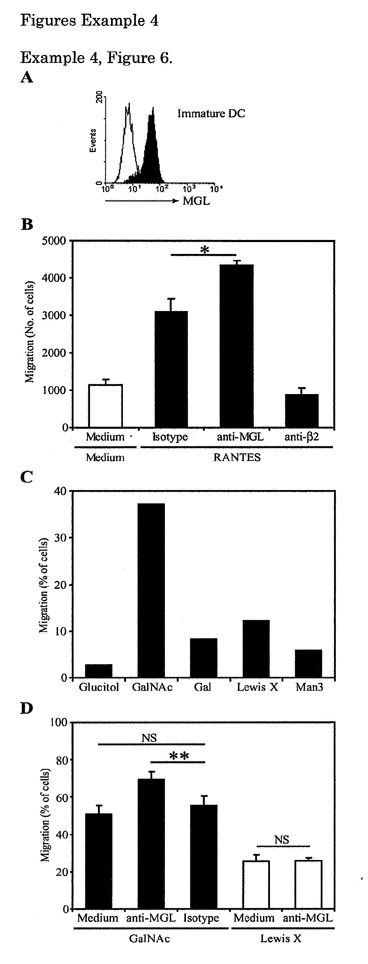

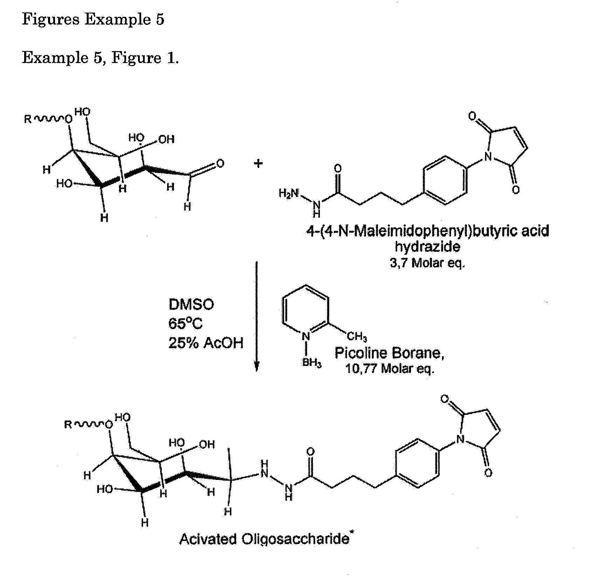

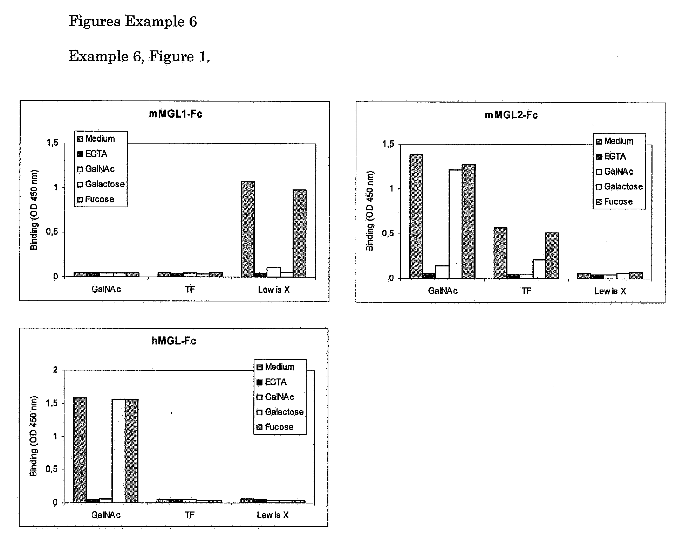

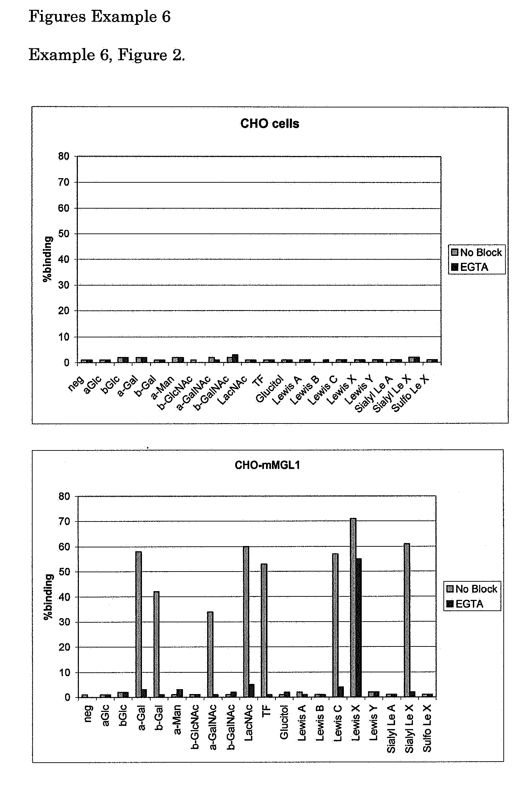

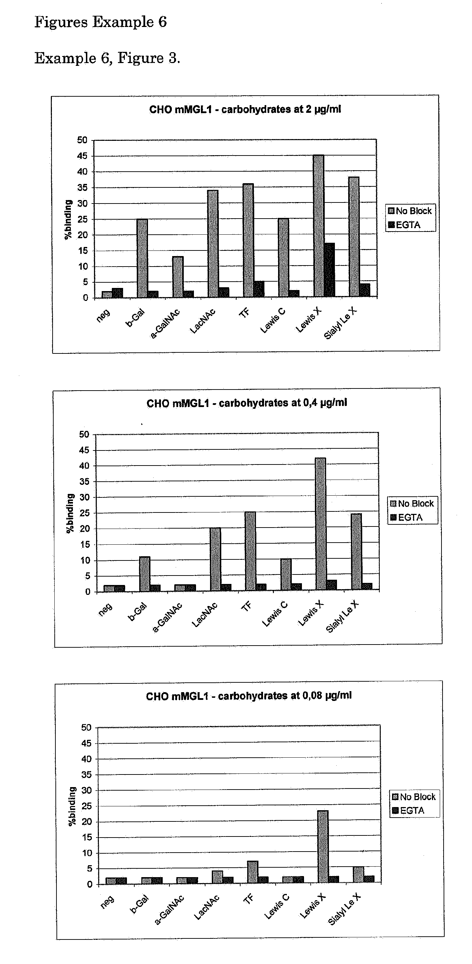

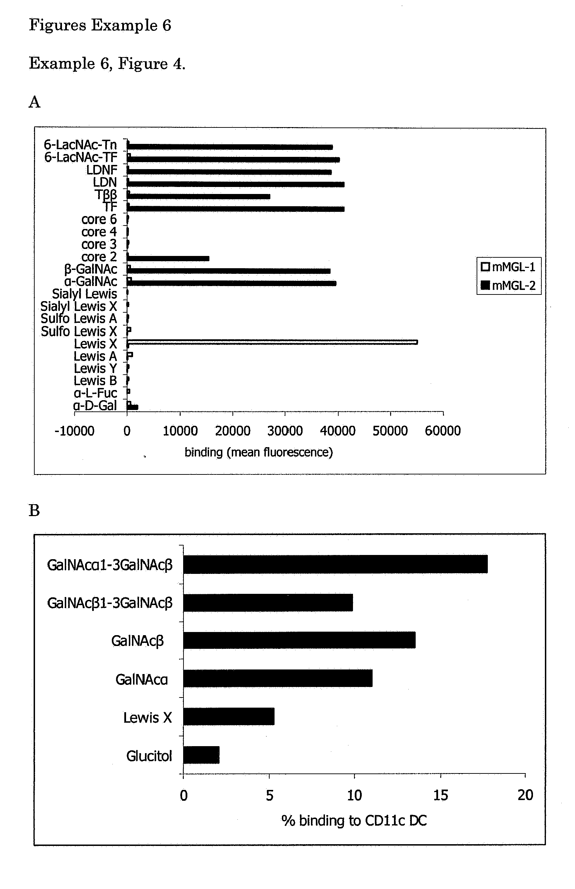

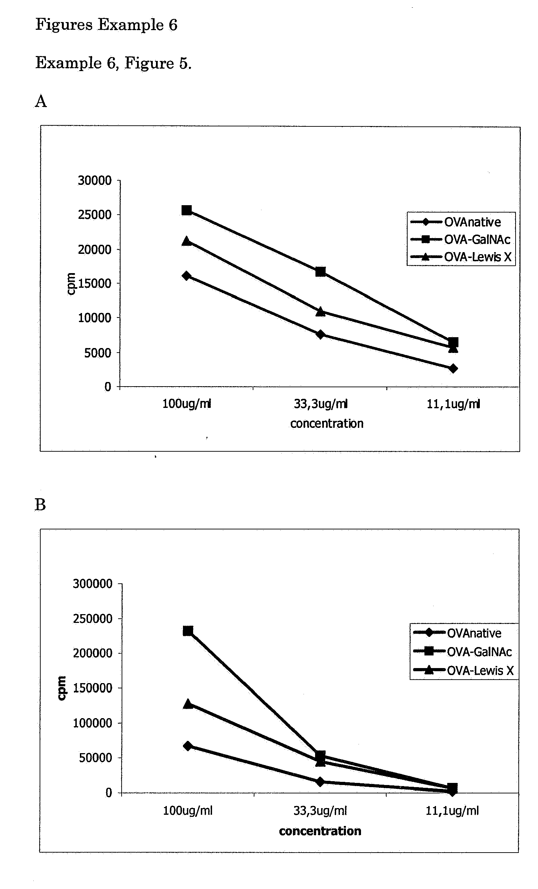

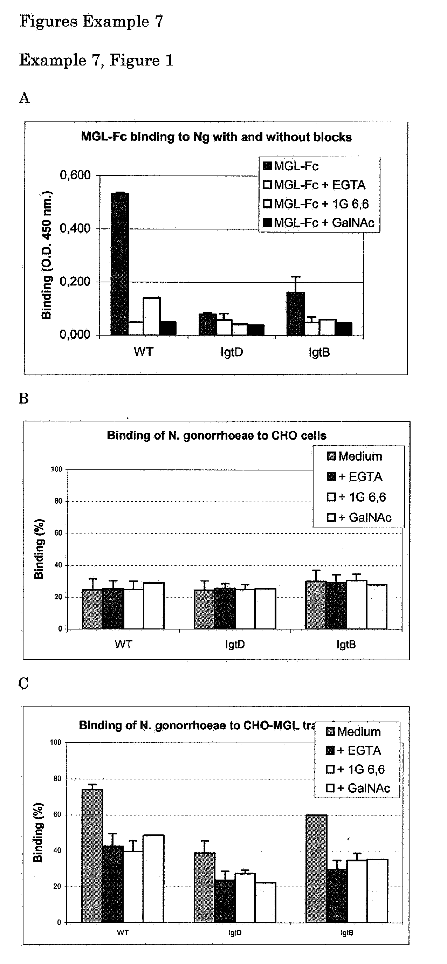

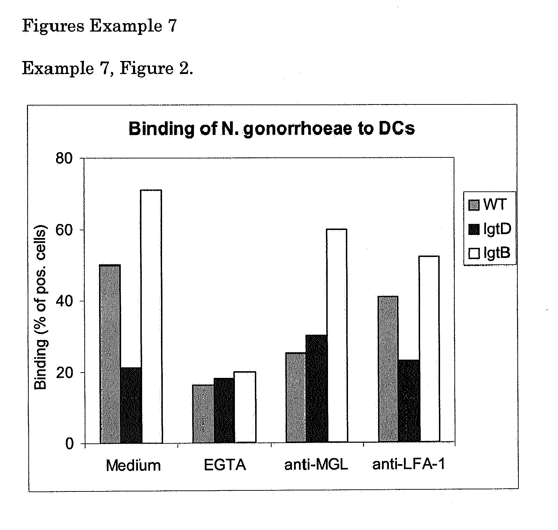

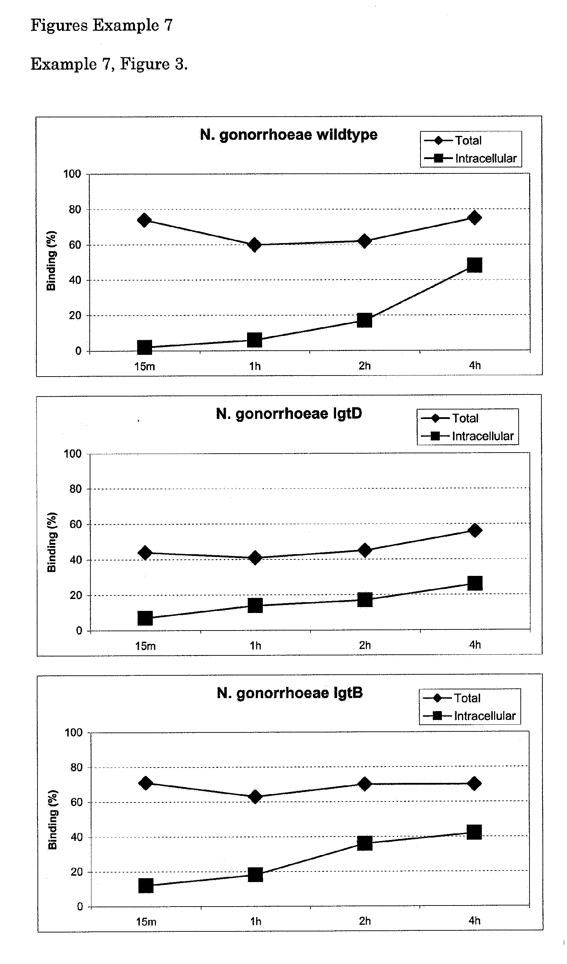

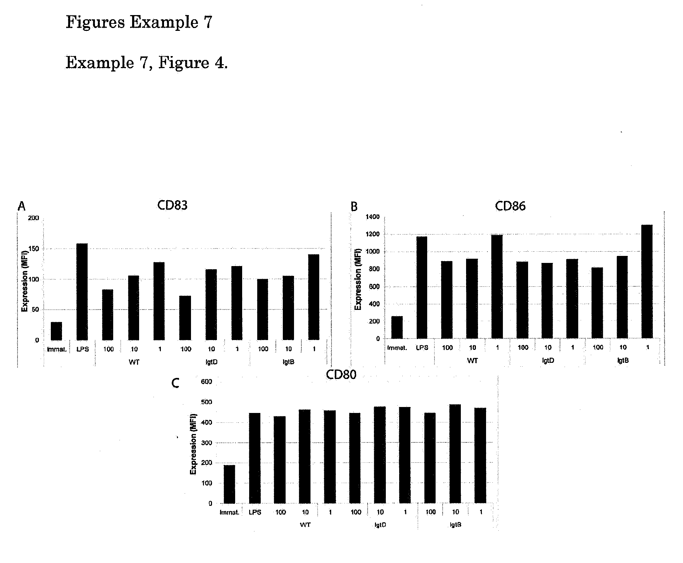

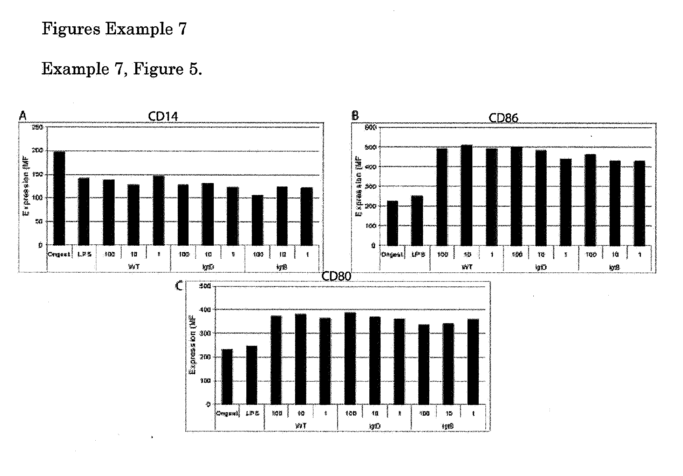

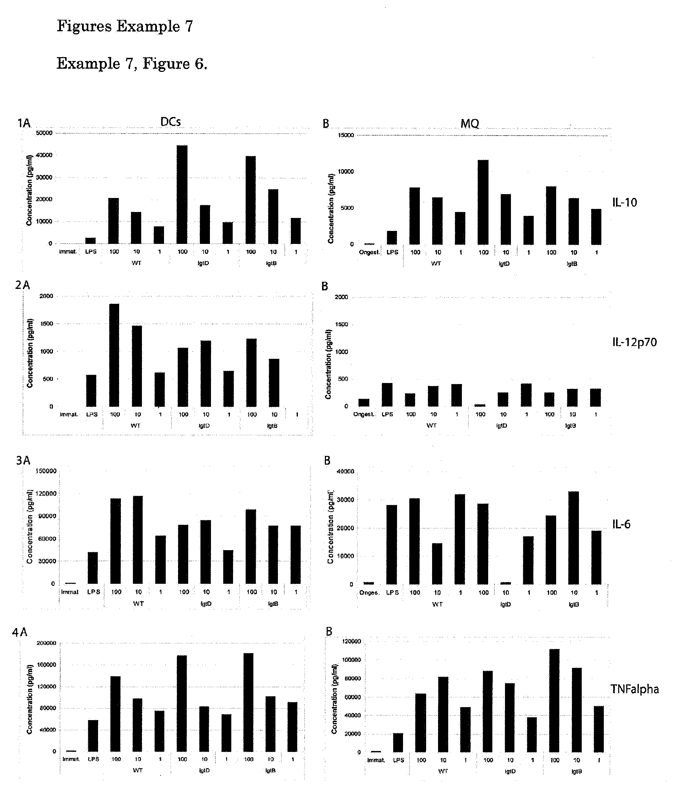

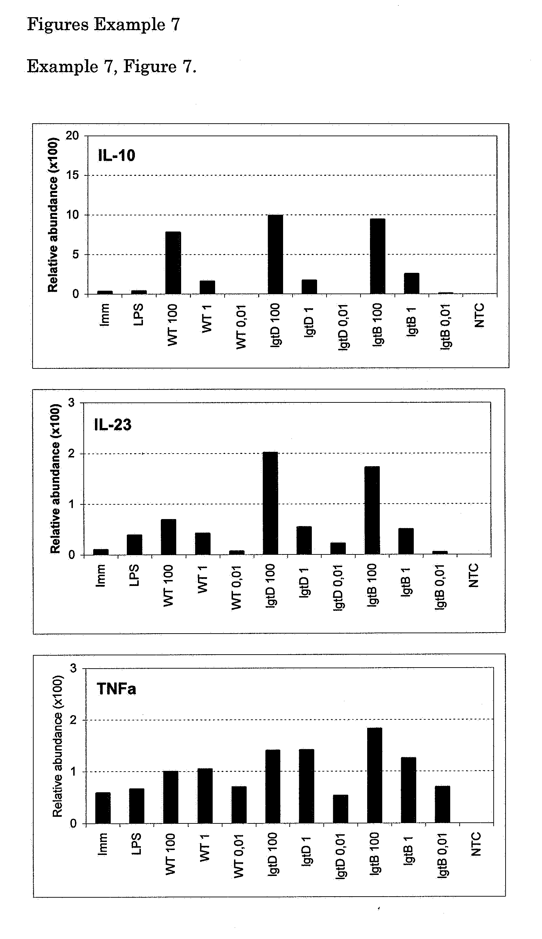

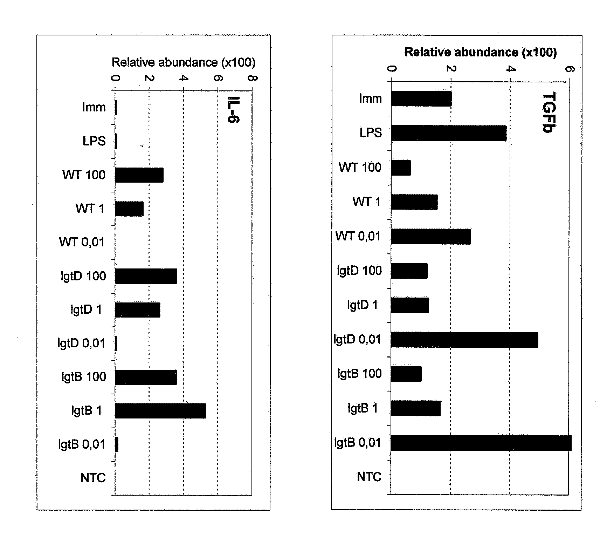

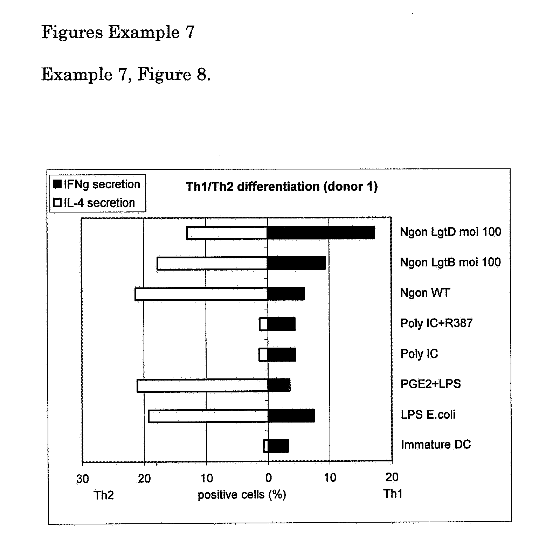

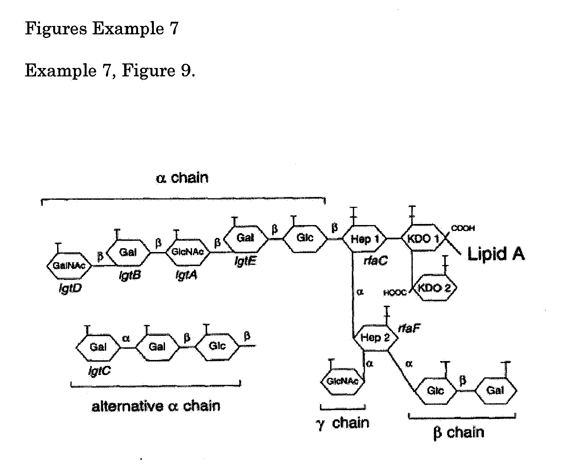

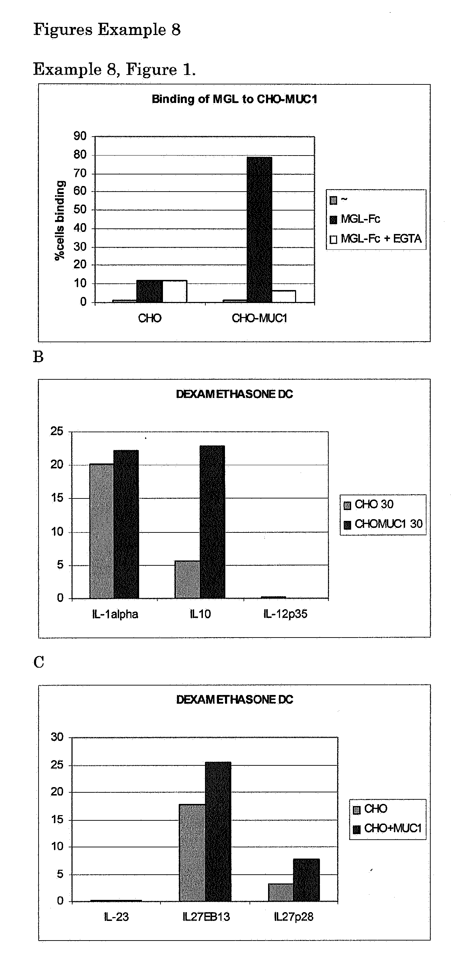

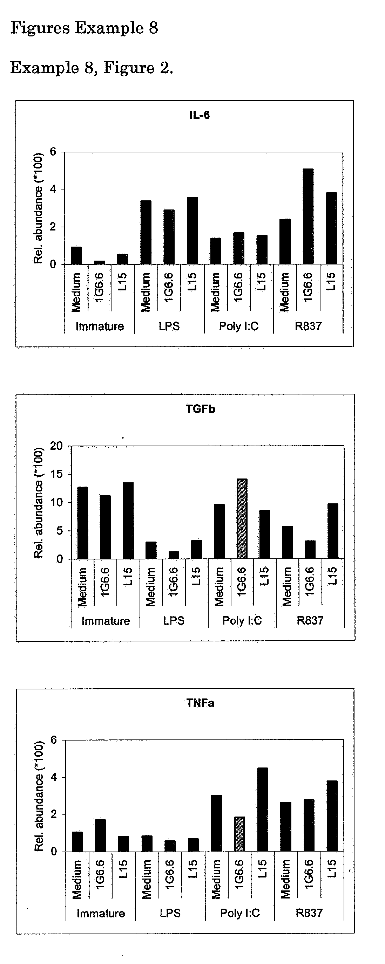

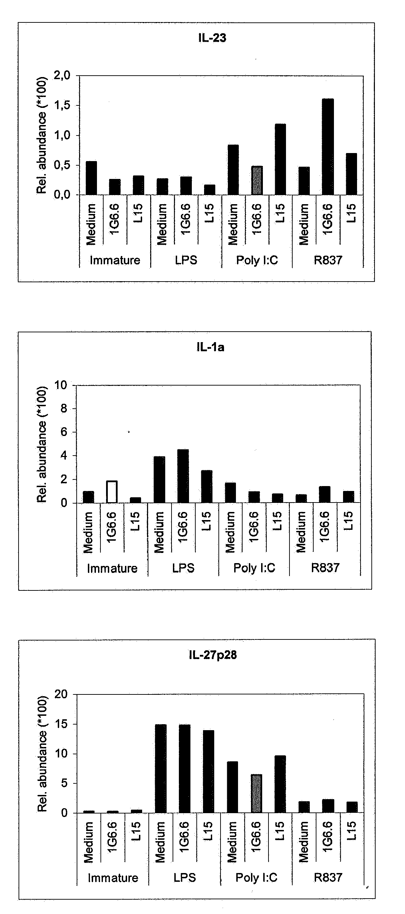

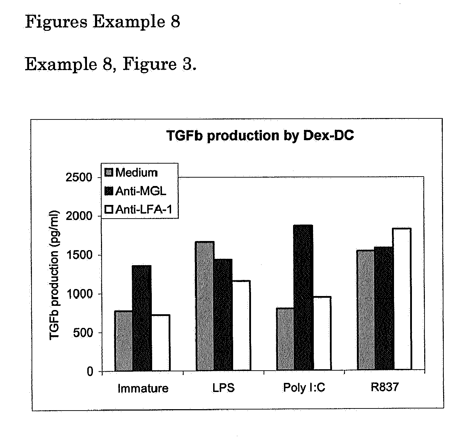

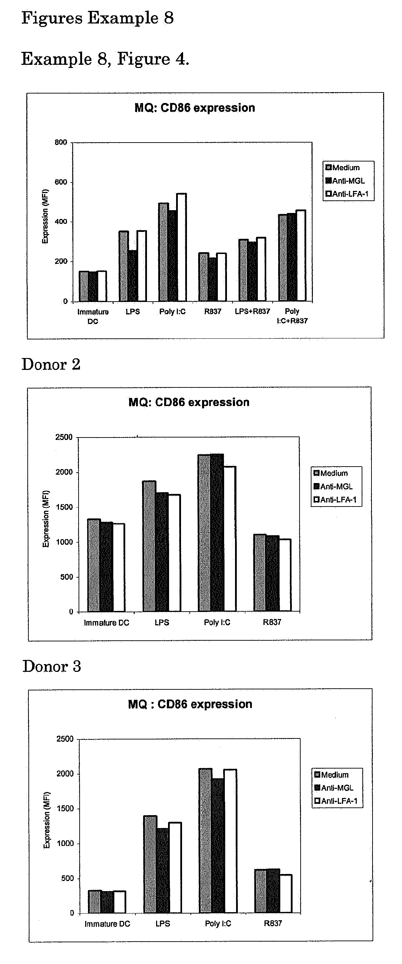

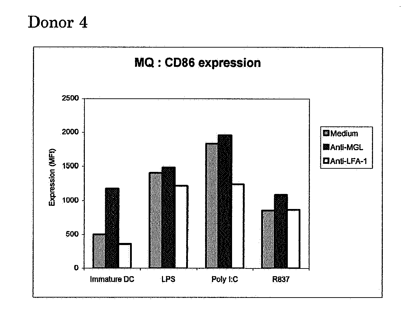

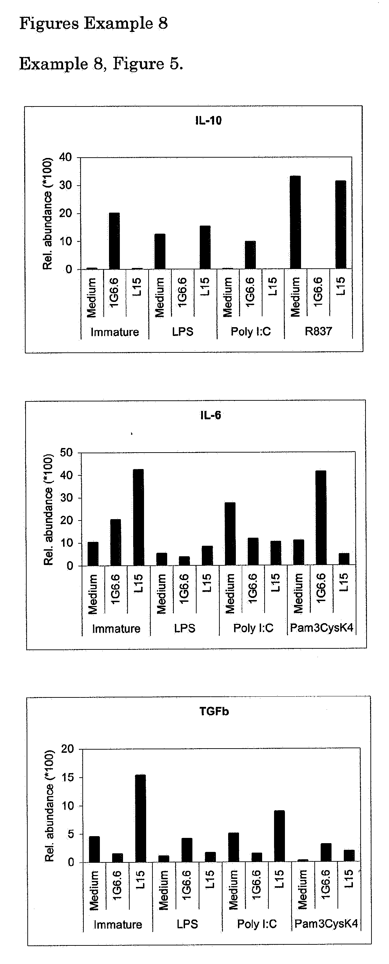

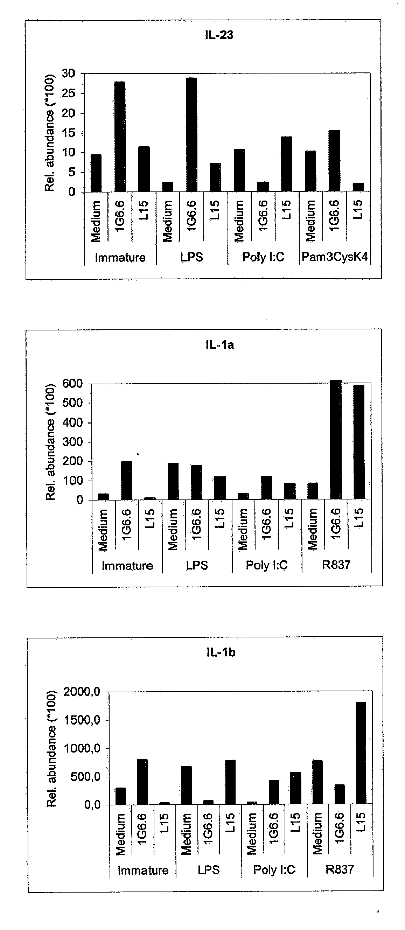

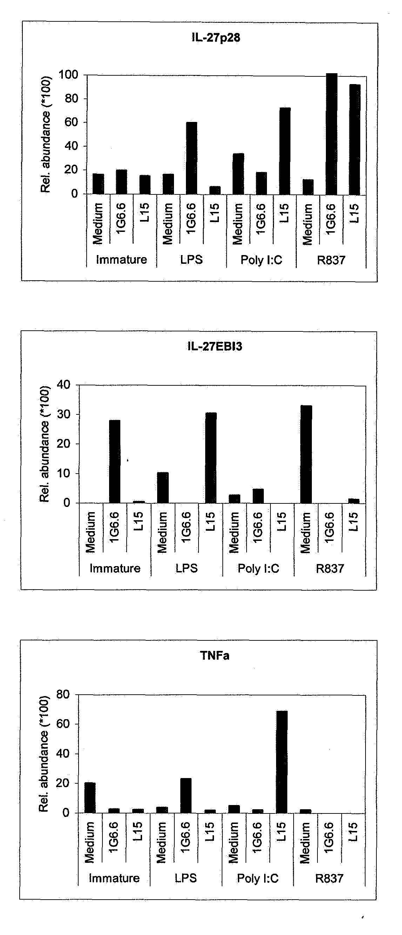

Figure 3 MGL binding downregulates CD45 phosphatase activity and TCR mediated calcium mobilization. (a) MGL binding significantly reduced CD45 phosphatase activity in Jurkat E6.1. The CD45-specific phosphatase activity, as determined by performing the phosphatase assay in the presence or absence of a specific CD45 phosphatase inhibitor, is shown. Standard deviation < 0.05 OD 405 nm. (b) MGL-Fc binding to CD45 on Jurkat specifically reduced the active pool of Lck as shown by the increased phosphorylation of the regulatory tyrosine Lck Y505 (left panel).
Phosphorylation was measured by flowcytometry using an antibody specific for phosphorylated form of Lck Y505. Right panel shows unstimulated and as a positive control, cells incubated with 5 mM H2O2. (c) Calcium mobilization was measured by flowcytometry on Fluo-3/AM loaded Jurkat E6.1 cells.
Arrows indicate the time points of MGL-Fc- or Control-Fc-beads and anti-CD3 addition. Binding of MGL-Fc coated beads and not of the Control-Fc-beads, greatly diminished the Ca2+-flux triggered by anti-CD3 antibodies. (d) Expression of CD45 and MGL-Fc binding was measured by flowcytometry on the parental D.J and its CD45neg clone JS7. (e) Calcium mobilization was measured by flowcytometry on Fluo-3/AM loaded D.J and JS7. Arrows indicate the time points of MGL-Fc-beads and anti-CD3 addition. Binding of MGL-Fc coated beads greatly diminished the Ca2+-flux triggered by anti-CD3 antibodies in D.J, in contrast to JS7. All results are representative for at least three independent experiments.

Figure 4 MGL induces cell death in a CD45-dependent manner. (a) Incubation of Jurkat cells in the presence of both MGL-Fc and stimulating anti-CD3 antibodies resulted in a marked proportion of cellular death as seen by FSC/SSC analysis. Indicated in the figure is the percentage of cells in the live-gate. (b) Cell death was quantified by flowcytometry using Annexin V-FITC and/or 7-AAD staining. Free GalNAc (50 mM) was added to block MGL-Fc function. Standard deviation <5%. (c) Incubation of both MGL-Fc and stimulating anti-CD3 antibodies could only induce cell death in the CD45pos D.J and not in the CD45neg JS7. Standard deviation <5%. All results are representative for at least three independent experiments.

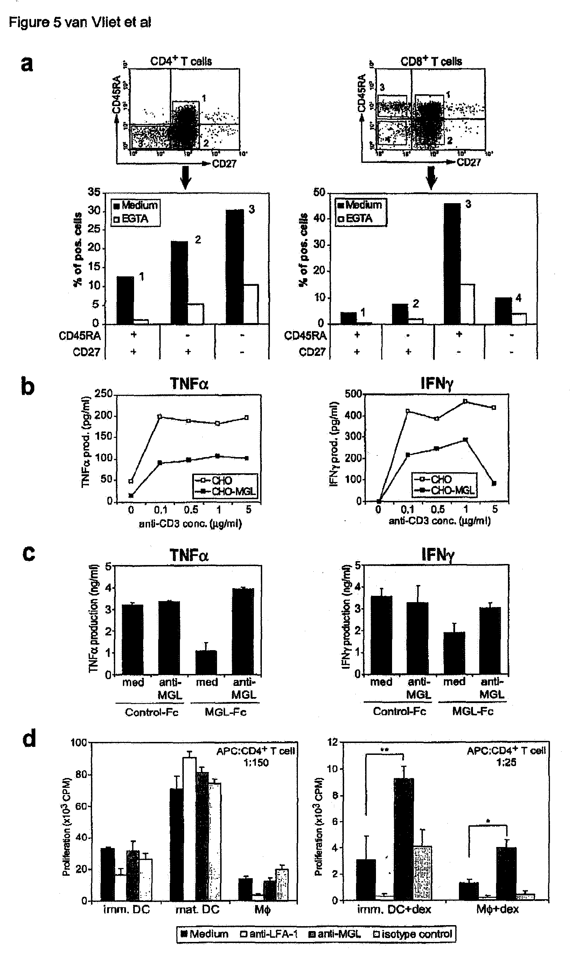

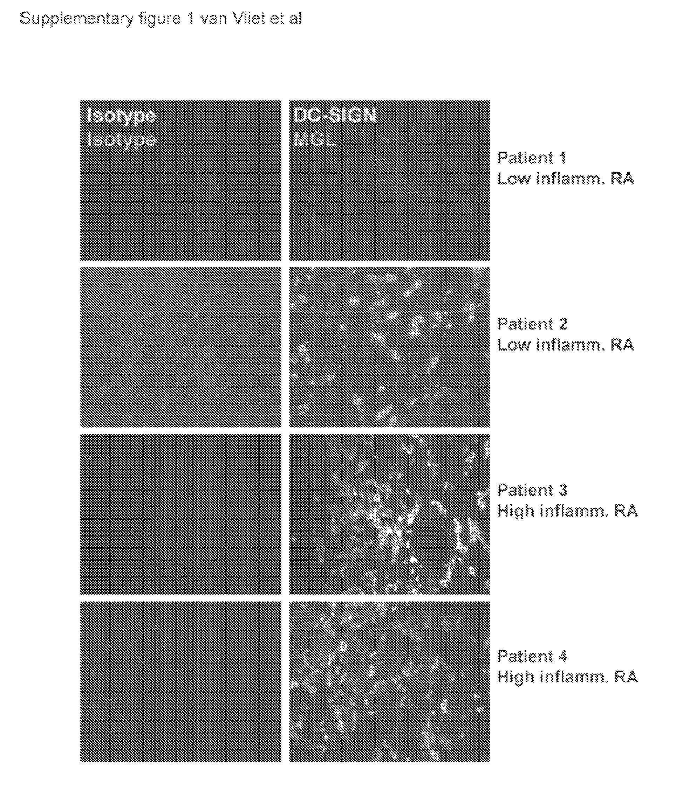

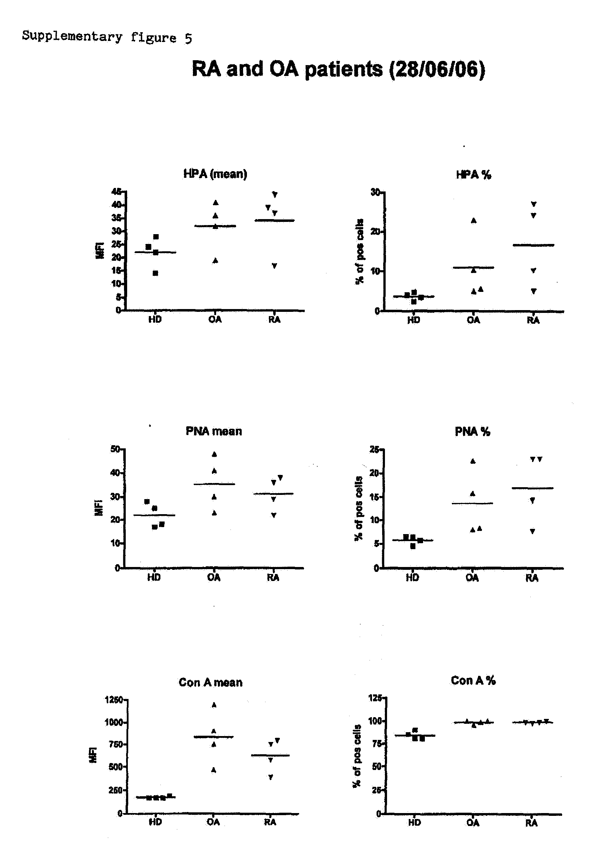

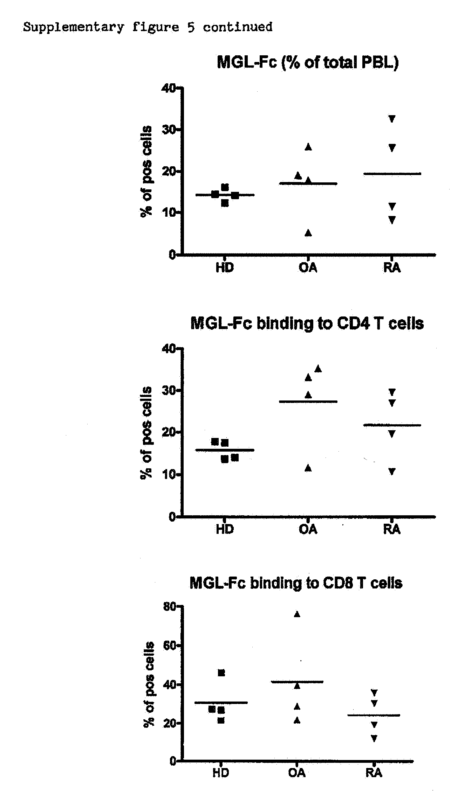

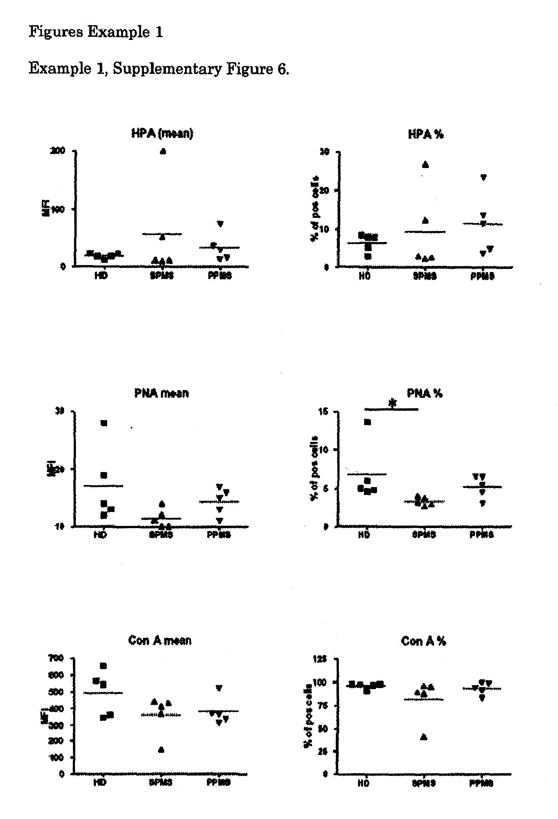

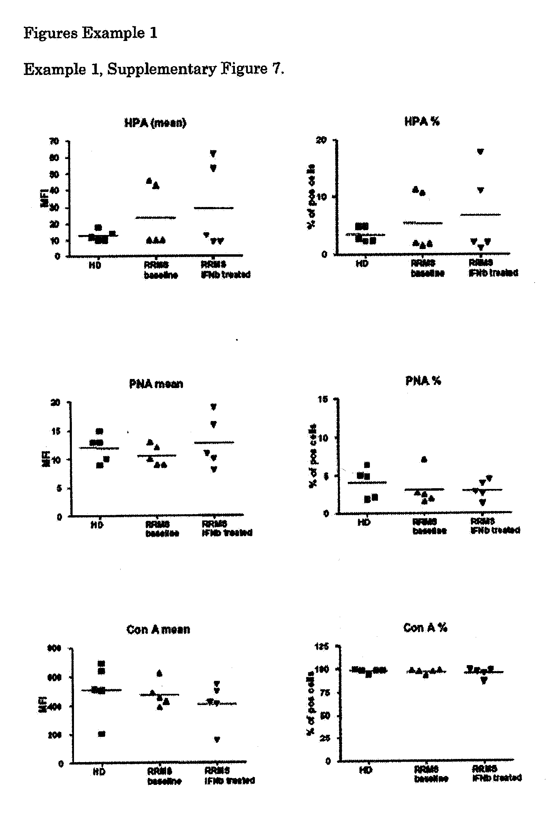

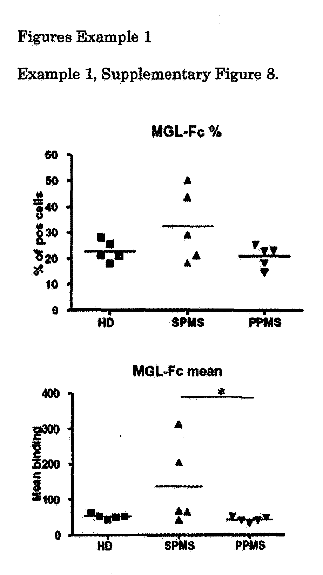

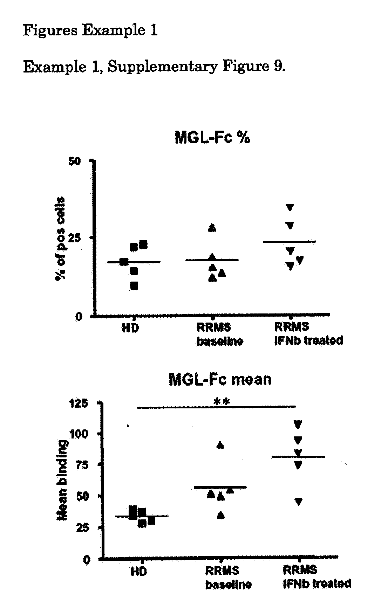

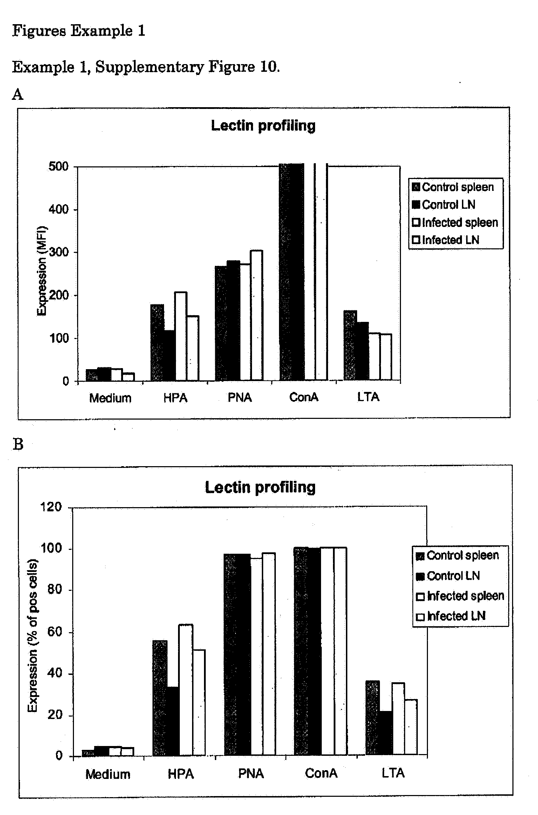

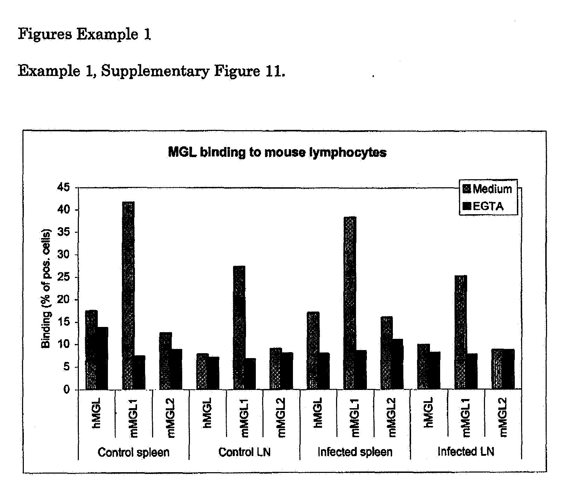

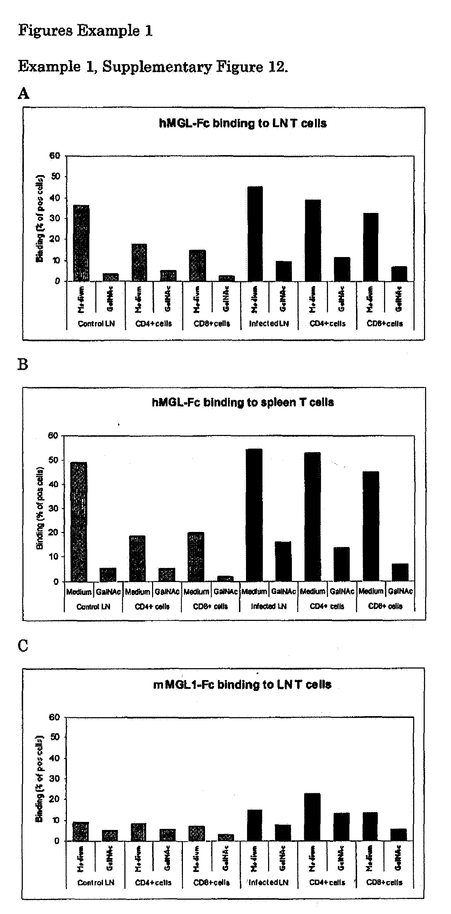

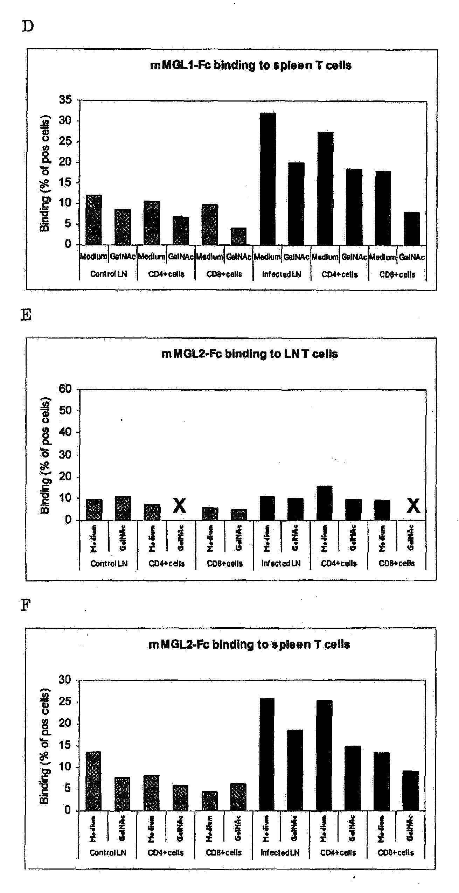

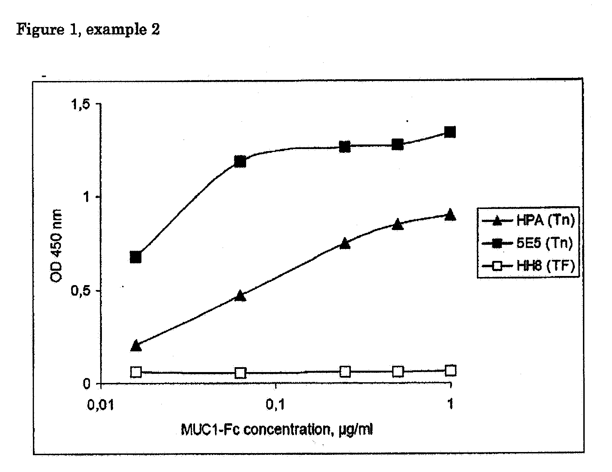

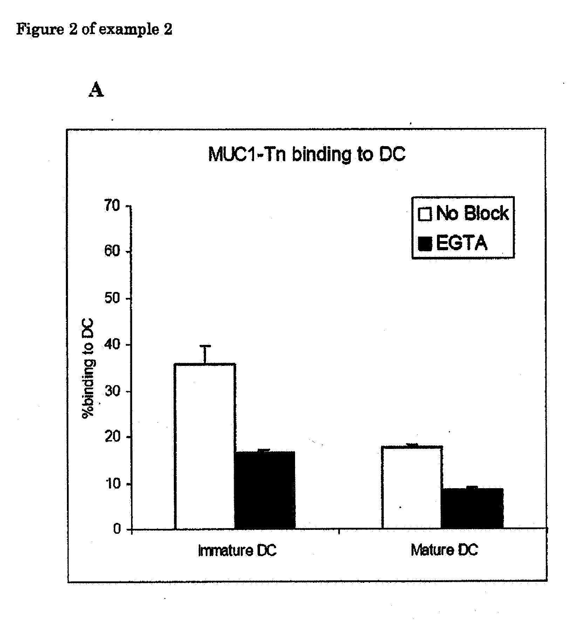

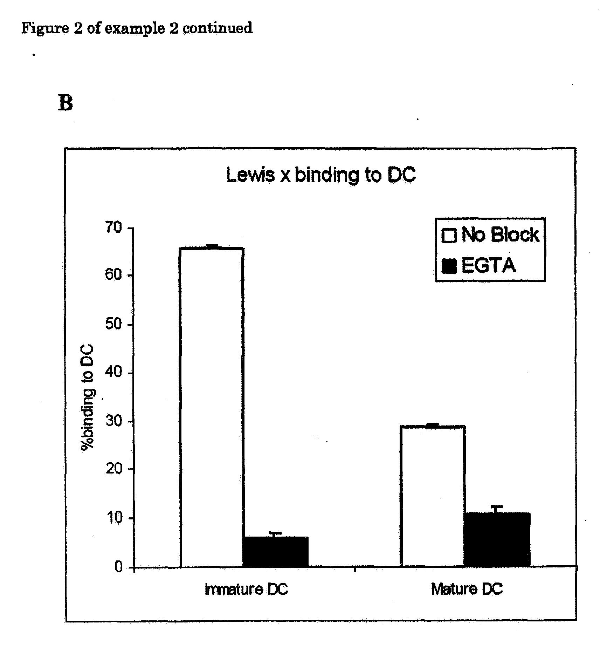

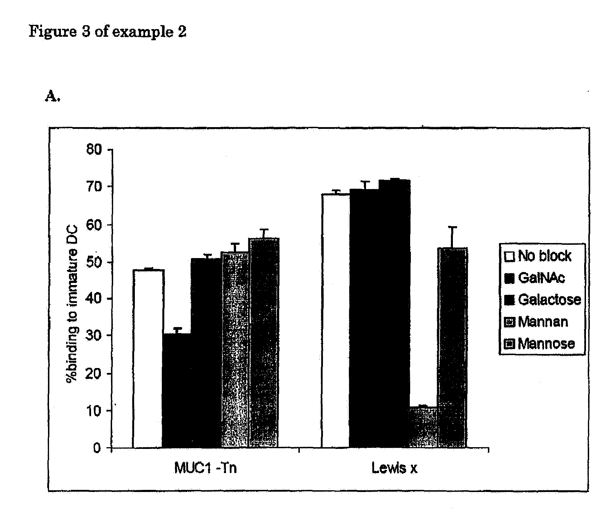

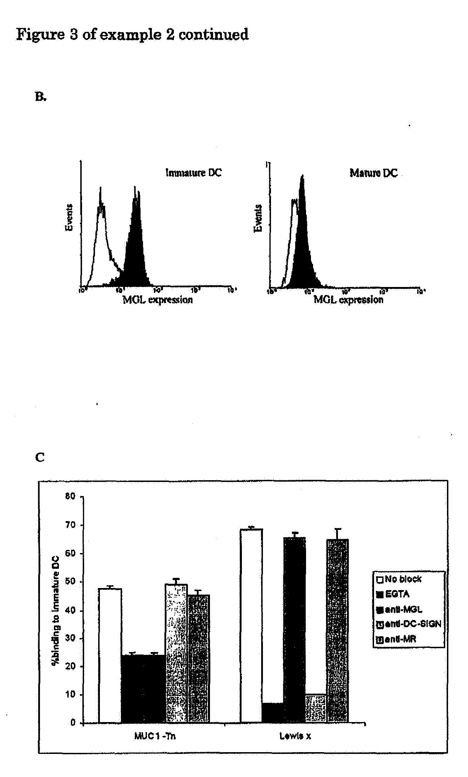

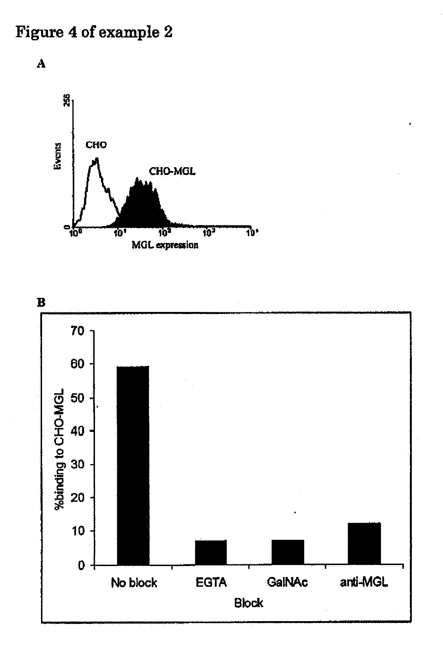

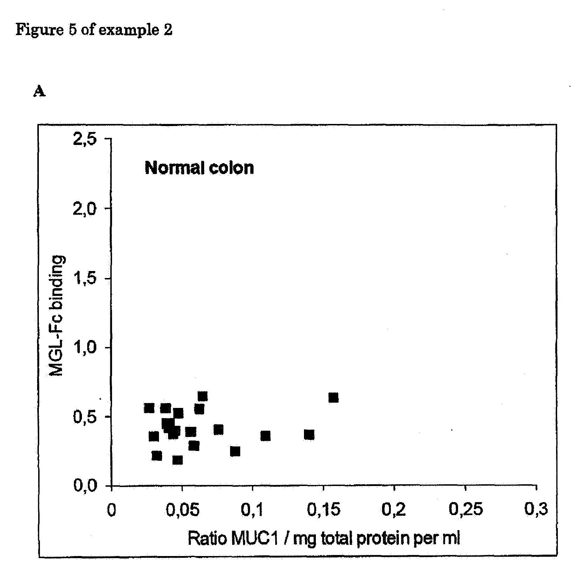

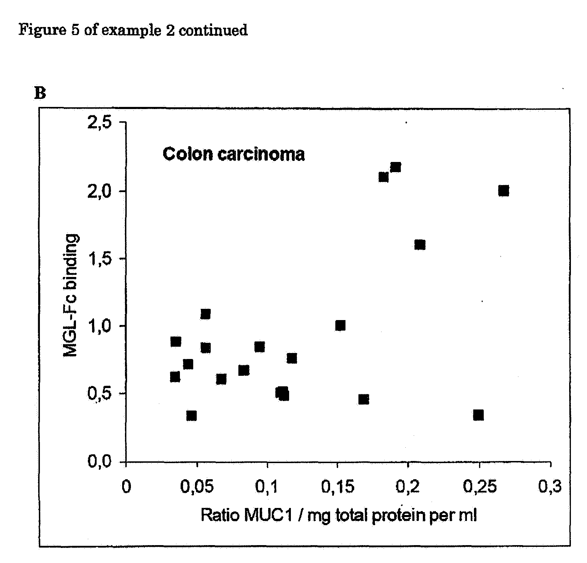

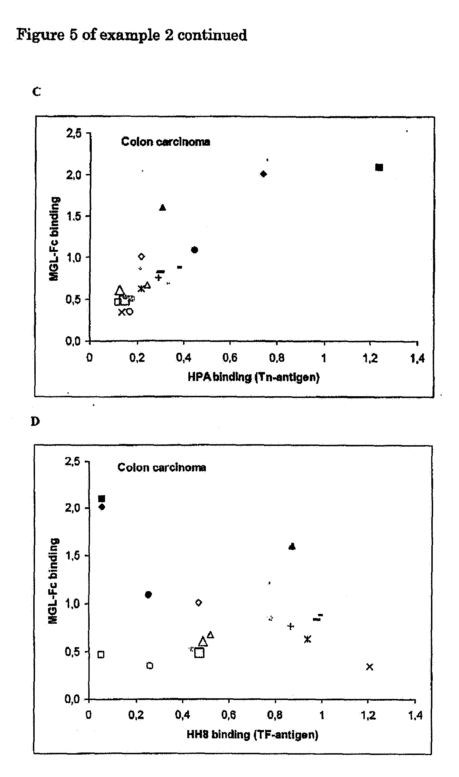

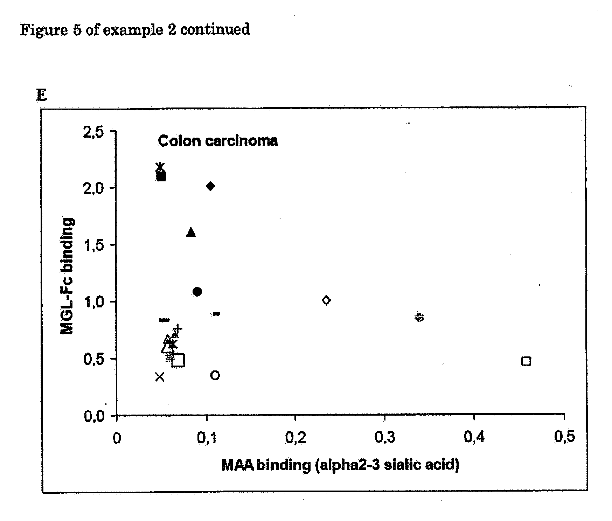

Figure 5 MGL on dexamethasone-treated APC provides negative regulatory signals to human effector T cells via CD45. (a) MGL preferentially recognizes effector-type T cells as measured by 4-color analysis of MGL-Fc binding and co-staining with anti-CD4 or CD8, CD27 and CD45RA-specific antibodies.
Standard deviation <5%. (b) MGL binding reduced pro-inflammatory cytokine production by PBL. PBL were seeded onto CHO or CHO-MGL and stimulated by addition of soluble anti-CD3 antibodies at concentrations indicated in the figure. After 24 hours supernatants were harvested and cytokines were measured by ELISA. (c) MGL binding reduced pro-inflammatory cytokine production by PBL. PBL were seeded onto MGL-Fc/anti-CD3- or control-Fc/anti-CD3-coated plates After 24 hours supernatants were harvested and cytokines were measured by ELISA. (d) Anti-MGL antibodies can partially revert suppressive activity of dexamethasone-cultured APC. APC and allogeneic purified CD4+ T cells (>97%) were co-cultured for 4 days at indicated ratios in the presence or absence of blocking LFA-1 or MGL-antibodies. 28-14-8 antibody was used as an isotype control. Proliferation was measured by [methyl-3H]Thymidine incorporation. No proliferation could be measured if CD4+ T cells were cultured without APC. (**) and (*) indicate a significant difference in proliferation (p<0.01 and p<0.05 respectively). All results are representative for three independent experiments.

Supplementary figure 1 Abundant MGL positive cells are present in high inflammatory RA tissues. Left panel: Isotype control stainings on tissue biopsies of four RA patients. Right panel: MGL- (red) and DC-SIGN-staining (green) on biopsies of four RA patients. Original magnification 400x.

Supplementary figure 2 CD45 is the main counterreceptor for MGL on lymphocytes. (a) Capture of ligands from PBL or Jurkat lysates on MGL-Fc coated plates was detected by specific antibodies to CD43, CD45 or LFA-1 (isotype control) in an ELISA-binding assay. MGL-Fc binding was blocked by the addition of 10 mM EGTA. One representative experiment out of three is shown. Standard deviation <0.05 OD 450 nm. (b) MGL only recognizes CD45 isolated from PBL. MGL binding proteins were captured from lysates of PBL, monocytes, immature DC or PMN by MGL-Fc coated plates was detected by specific antibodies to CD45 in an ELISA-binding assay.MGL-Fc binding was blocked by the addition of 10 mM EGTA. One representative experiment out is shown. Standard deviation <0.05 OD 450 nm.

Supplementary figure 3 MGL binding reduces TNFα production by CD4+ and CD8+ T cells. Purified CD4+ and CD8+ T cells (>95 pure) were seeded onto MGL-Fc/anti-CD3- or control-Fc/anti-CD3-coated plates After 24 hours supernatants were harvested and TNFα production was measured by ELISA.

Supplementary figure 4 Model of MGL mediated control of effector T cell homeostasis. MGL binding to CD45 on effector T cells negatively influences TCR-mediated signaling, leading to lower pro-inflammatory cytokine production, reduced proliferative responses and even cell death.

### Supplementary figure 5

MGL binds to PBLs from chronic infected patients with RA and OA.
Frozen total PBLs were thawed and stained with MGL-Fc as earlier mentioned. Also plant lectins such as HPA, with specificity for GalNAc, Con A, high mannose and diantennary, and PNA, TF-antigen Galβ1-3GalNAc, where used to determine glycan epitopes on subsets of cells.
Also HPA which has a similar glycan specificity as MGL showed enhanced staining of PBL from RA and OA patients. These results show that MGL-Fc can be used as a probe to detect chronic inflammatory status of patients that have RA and may be used for early detection of chronic inflammatory diseases.

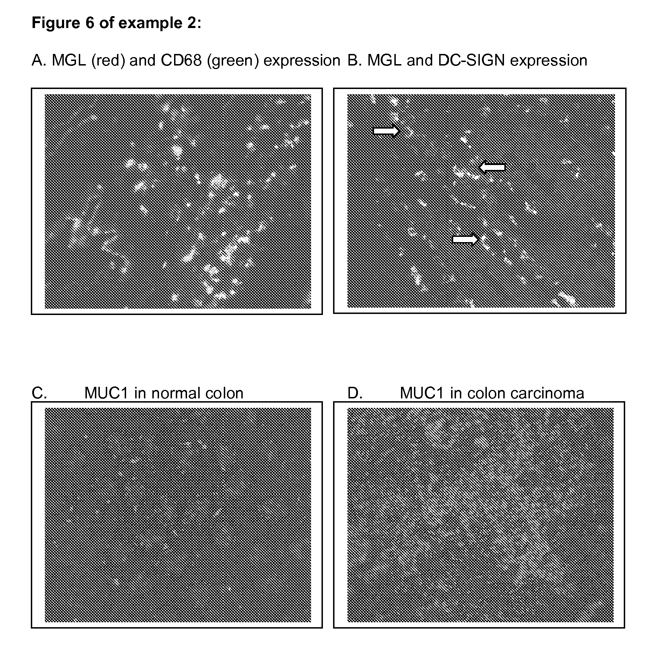

Example 1, Supplementary figure 6. Plant lectin staining on PBLs from healthy donors and MS patients (I).

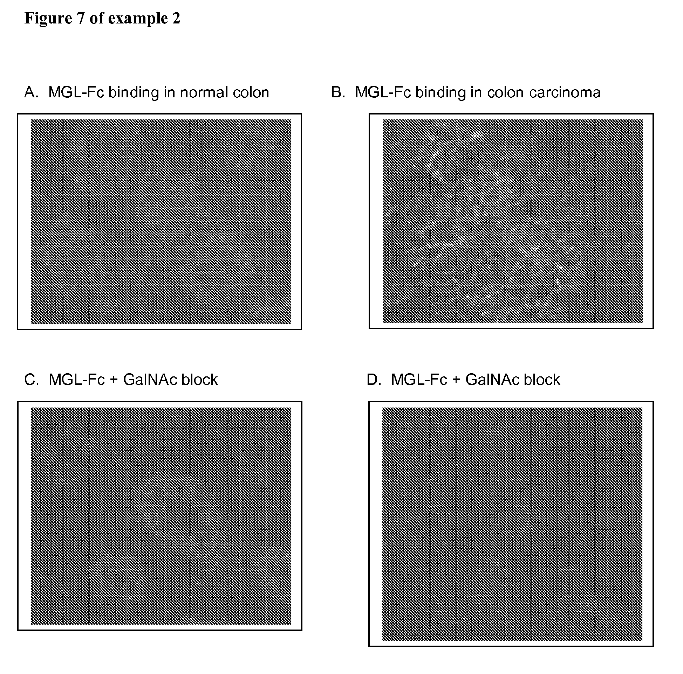

Example 1, Supplementary figure 7. Plant lectin staining on PBLs from healthy donors and MS patients (II).

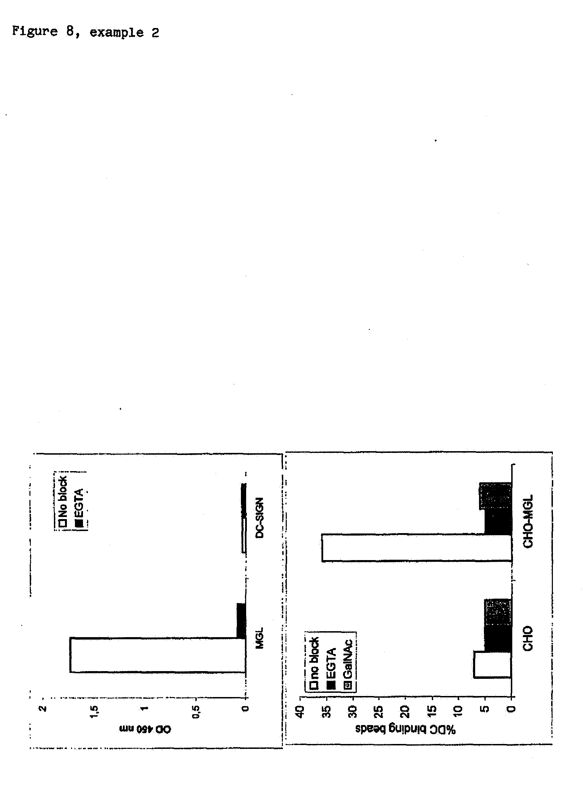

Example 1, Supplementary figure 8. MGL-Fc binding to PBLs from healthy donors and MS patients (I).

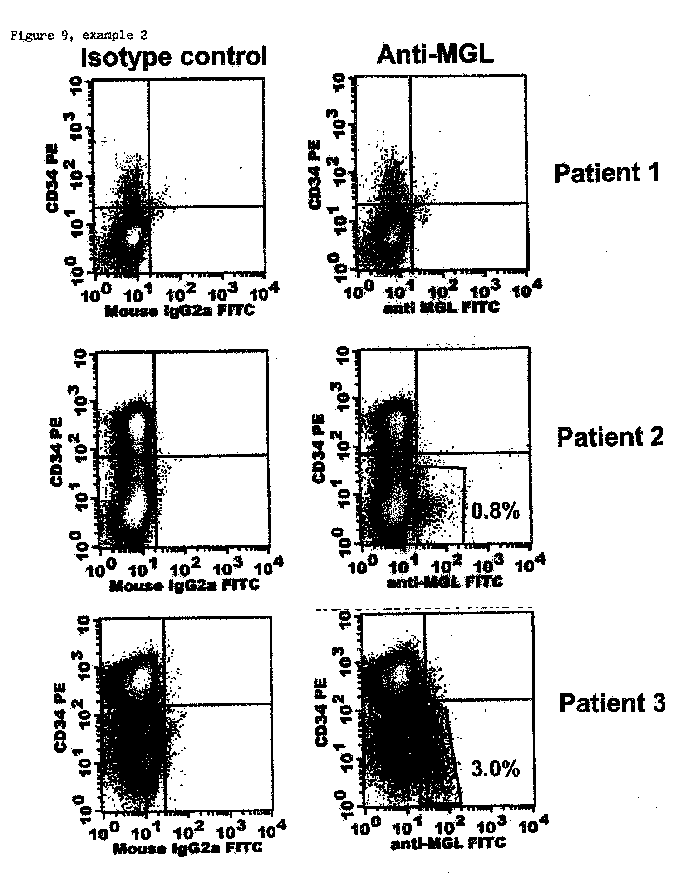

Example 1, Supplementary figure 9. MGL-Fc binding to PBLs from healthy donors and MS patients (II).

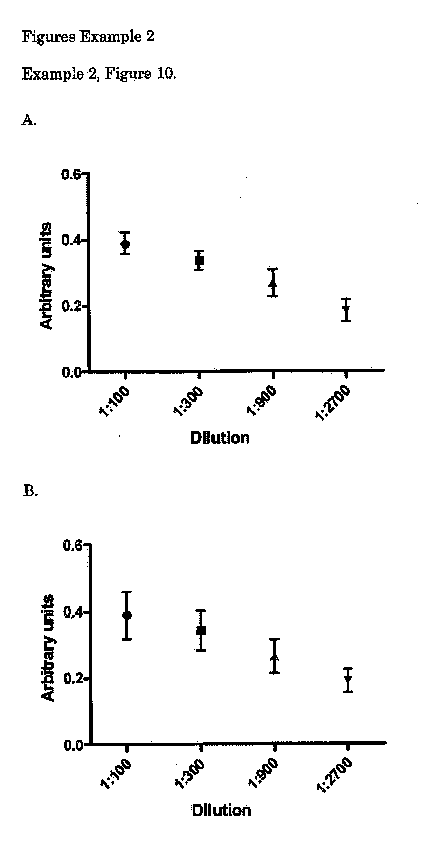

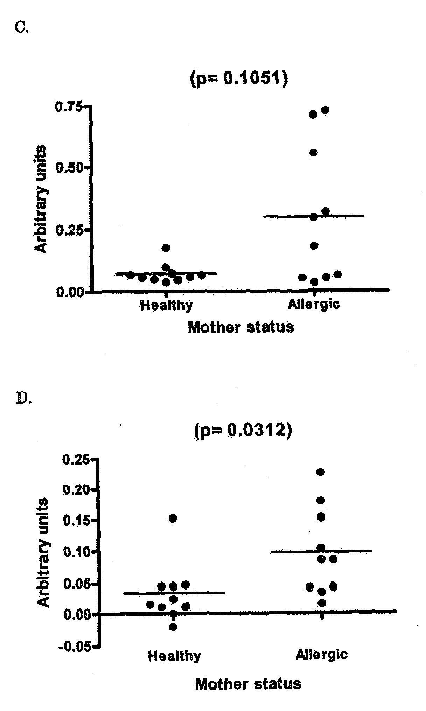

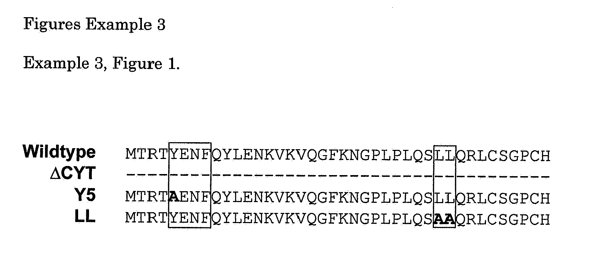

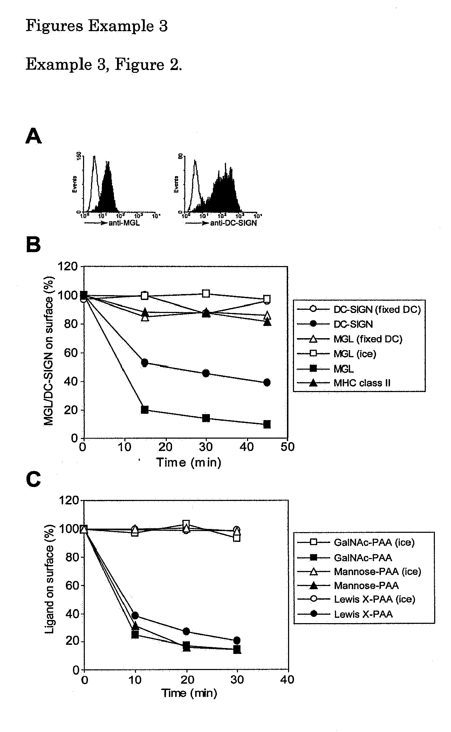

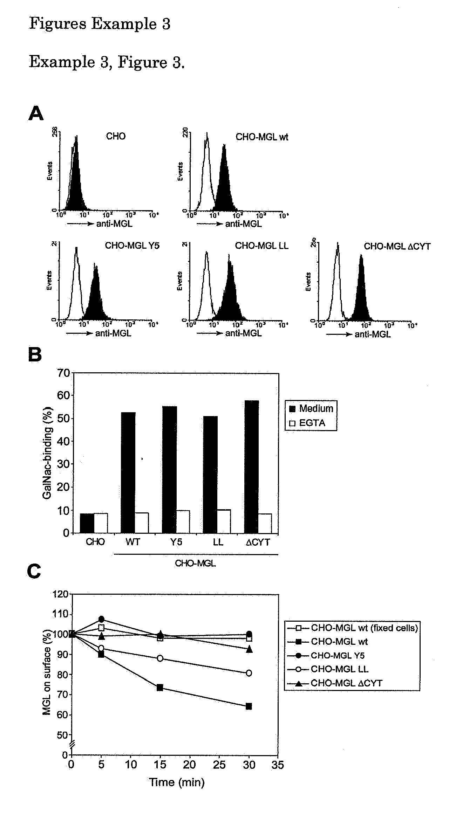

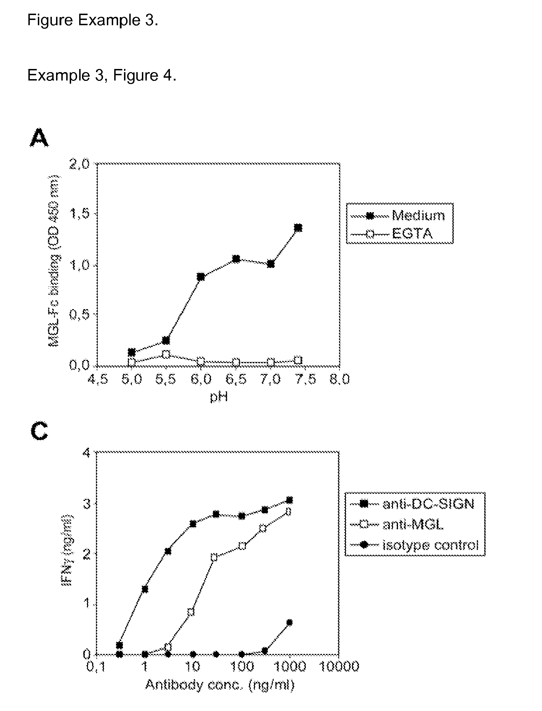

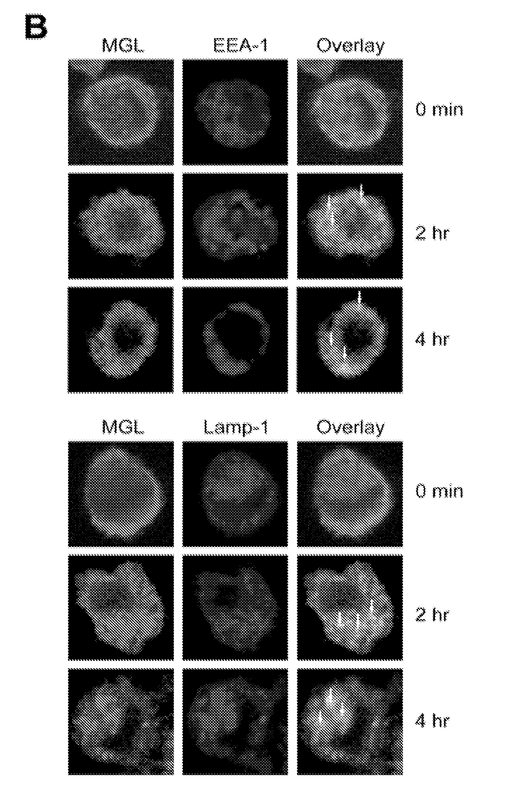

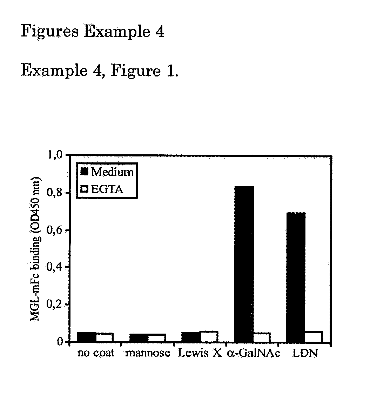

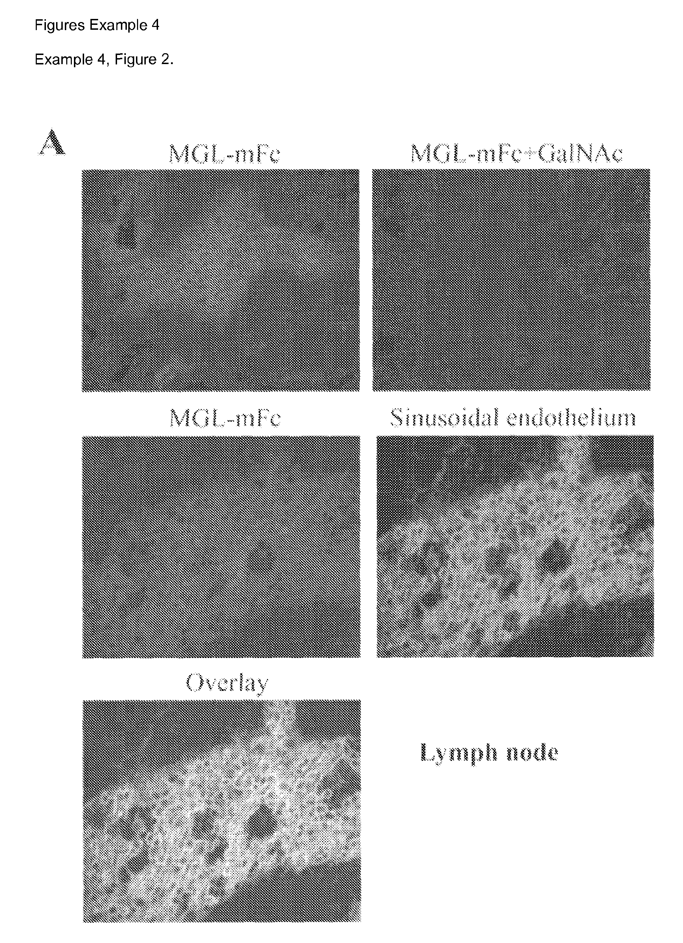

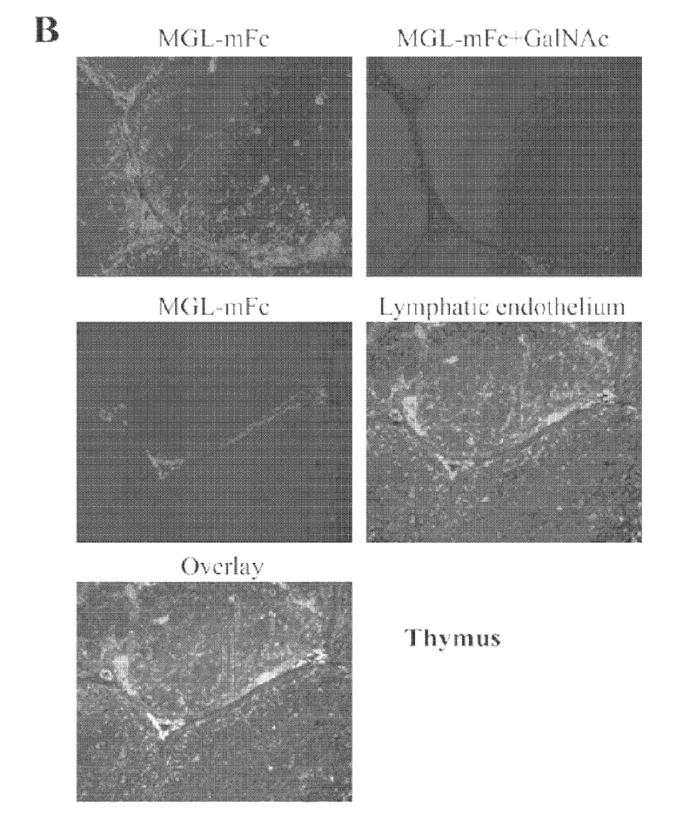

Example 1, Supplementary figure 10. Lectin profiling of surface glycans on mouse lymphocytes. Mouse lymphocytes were isolated from lymph node and spleen. Cell surface glycans were determined by staining with plant lectins and measured by flow cytometry. In Figure 10A the mean fluorescent intensity (MFI) is shown and in Figure 10B the percentage of positive cells.

Example 1, Supplementary figure 11. MGL recognition of mouse T cells (I). Beads binding was determined by flow cytometry. Binding is denoted as the percentage of cells, which have bound the coated beads.

Example 1, Supplementary figure 12. MGL recognition of mouse T cells (II). Binding of human MGL-Fc (A and B), mMGL1-Fc (C and D) and mMGL2-Fc (E and F) was determined by flow cytometry.

### Figure legends to the figures of example 2

Example 2, Figure 1: MUC1-Fc fusion proteins contain Tn-antigens MUC1-Fc was serially diluted and coated on ELISA plate. Glycan structures analysed with Tn-specific antibodies (5E5), HPA (specific for Tn-antigens) and TF-specific antibodies (HH8).

Example 2, Figure 2: MUC1-Tn interacts with dendritic cells MUC1-Fc proteins were coupled to fluorescent beads and incubated with day 5 immature or mature monocyte derived DC in the presence or absence of 10 mM EGTA (A). Beads coated with polyacrylamide coupled Lewis X structures (ligand for DC-SIGN on DC) served as a positive control for beads binding (B). The figure shows the percentage of cells binding beads as determined by flow-cytometry. Mature DC were obtained by incubating DC in the presence of 100 ng/ml lipopolysaccharide overnight. Samples were analyzed in triplicates (error bars represent standard deviation of the mean) and 1 representative experiment is shown out of 3.

Example 2, Figure 3: The Macrophage Galactose-type C-type lectin (MGL) binds to MUC1 (A) To determine the specificity of MUC1 interaction with DC, cells were pre-incubated with different carbohydrate structures before adding MUC1-Fc coated beads. Only GalNAc structures reduced binding of MUC1 to DC and mannan blocked binding of Lewis x. (B) MGL expression on day 5 immature and mature DC was determined by mAb 18E4 (C) DC were pre-incubated with different anti-CLR blocking antibodies before incubation with MUC1-Fc coated beads. The figure shows the percentage of cells binding beads as determined by flow-cytometry. Samples were analyzed in triplicates (error bars represent standard deviation of the mean) and 1 representative experiment is shown out of 3.

Example 2, Figure 4: MUC1 binds to MGL transfected CHO cells (A) MGL expression on CHO cells transfected with MGL (filled histogram).
Non-transfected CHO cells served as a negative control. MUC1-Fc-coupled beads were incubated with non-transfected CHO cells or CHO cells transfected with MGL (B). Cells were pre-incubated with EGTA or 25 mM GalNAc monosaccharides to block specific interaction with MGL. One representative experiment is shown out of 3.

Example 2, Figure 5: MGL interaction with colon derived MUC1 mucins MUC1 was captured from tissue lysates of a primary colon tumor (n=20), the corresponding normal colon (n=20) by mAb 214D4 in ELISA. The mucins were detected by the same antibody and binding by MGL-Fc, HPA, mAb HH8 and MAA investigated. A) The relationship between MGL-Fc binding and MUC1 concentration in normal colon lysates. B) The relationship between MGL-Fc binding and MUC1 concentration in colon carcinoma lysates. Relationship between MGL-Fc and C) HPA binding, D) HH8 binding and E) MAA binding. Each patient is represented by one symbol that can be found in panels C-E. Samples were analysed in duplicates and one representative experiment of 2 is shown.

Example 2, Figure 6: Characterization of MGL expressing cells Cryosections were stained with anti-MGL (18E4), anti-CD68 (EBM11), anti-DC-SIGN (AZN-D1) or anti-MUC1 antibodies (mAb 214D4) for 2 h at 37°C. After washing the sections were incubated with Alexa-594 labeled goat anti-mouse IgG2a (18E4) and Alexa-488 labeled goat anti-mouse IgG1. A) Colon carcinoma section stained for MGL (red) and CD68 (green). B) colon carcinoma section stained for MGL (red) and DC-SIGN (green). MUC1 staining was performed on both normal tissue (C) and colon carcinoma (D).

Example 2, Figure 7: MGL-Fc stains colon carcinoma Cryosections were stained with MGL-murine Fc for 2 h at 37°C, in the absence (A and B) or presence (C and D) of 100 mM GalNAc monosaccharides. After washing the sections were incubated with Alexa-488 labeled goat anti-mouse IgG. Both normal colon epithelium (A and C) and primary colon carcinoma (B and D) were stained from the same patient. Results from one representative patient out of 4 is shown.

Example 2, Figure 8. MGL-Fc detects secreted MUC1 from tumor cell line ZR75 A. MUC1 was harvested out of the supernatant of cultured ZR75 tumor cells and coated on ELISA plates. Both MGLFc and DC-SIGNFc were used to look for carbohydrate binding epitopes expressed on tumorassociated MUC1. As illustrated MGLFc binds strongly the secreted tumor associated MUC1 whereas DC-SIGN does not bind. Furthermore EGTA which inhibits lectin binding to carbohydrates illustrates that the recognition of MGL to MUC1 is carbohydrate mediated. B. In a second experiment the tumor associated MUC1 were coated on fluorescent beads coupled with MUC1 antibodies. Fluorescent MUC1 coupled beads where added to MGL expressing CHO cells and untransfected CHO cells. As demonstrated the Fluorescent beads loaded with tumor associated secreted MUC1 bound strongly to MGL expressing cells which could be blocked by EGTA and the glycan GalNAc which specifically binds MGL.

Example 2, Figure 9. MGL is expressed on subsets of AML. Bone marrow samples were of patients with AML were stained with MGL antibodies. Clearly a subset of these CD34 positive cells were MGL positive. The expression of MGL on these leukemic cells may inhibit good recognition and removal of the leukemic cells by effector T cells, by the induction of apoptosis or by reducing the effectiveness of the effector T cells.

Example 2, Figure 10. MUC1 from allergic mothers displays more GalNAc epitopes and MGL binding sites compared to MUC1 from healthy mothers. A. MUC1 concentration in milk. B. MUC1 concentration in allergic milk. C. MGL-Fc binding to MUC1. D. HPA binding to MUC1 in milk samples.

### Figure legends to the figures of example 3

Example 3, Figure 1. Cytoplasmic domain mutants of human MGL Example 3, Figure 2. Ligand binding induces endocytosis of MGL. (A) Expression of MGL and DC-SIGN on human monocyte-derived DCs as measured by flowcytometry. Open histogram, isotype control; filled histogram, MGL or DC-SIGN expression. (B) Internalization of cell surface bound antibodies to MGL, DC-SIGN and MHC II as measured by flowcytometry on immature DCs. Fixed cells and cells on ice were included to control for the antibody off-rate. Values represent mean of duplicates. (C) Internalization of cell surface bound ligands to MGL (GalNAc-PAA) and DC-SIGN (PAA-Mannose and PAA-Lewis X) as measured by flowcytometry on immature DCs. Maximal binding was set to 100% and corresponds to 30% of the cells binding of PAA-GalNAc and 98% binding of PAA-Lewis X and PAA-mannose. Cells on ice were included to control for the off-rate of the ligands.
Values represent mean of duplicates. Results are representative for three independent experiments.

Example 3, Figure 3. The YENF motif regulates MGL-mediated endocytosis. (A) Expression of wildtype and mutant MGL on CHO-transfectants as measured by flowcytometry. Open histogram, isotype control; filled histogram, MGL expression. (B) Binding of GalNAc-coated beads to CHO-transfectants of wildtype and mutant MGL. Standard deviation <5% (C) Internalization of cell surface bound antibodies to MGL as measured by flowcytometry on CHO-MGL transfectants. Values represent mean of duplicates. Results are representative for three independent experiments.

Example 3, Figure 4. MGL functions as an antigen receptor on human DCs. (A) MGL-Fc binding to GalNAc-PAA was measured by ELISA at indicated pH values. One representative experiment out of three is shown. (B) anti-MGL (Green) is targeted to early endosomes and lysosomes. Anti-MGL antibodies were allowed to internalize for 0, 2 or 4 hours from the DC surface. After fixation and permeabilization, cells were stained for LAMP-1 (lysosomes) or EEA-1 (early endosomes) (Red). Co-localization is indicated by the white arrows. (C) Proliferation of IgG1-specific T cells induced by immature DCs was analyzed in the presence of serial dilutions of anti-MGL (MLD-1), anti-DC-SIGN (AZN-D1), or anti-ICAM-2 (12A2, isotype control) antibodies. One representative experiment is shown.

### Figure legends to the figures of example 4

Example 4, Figure 1. MGL-mFc recognizes α-GalNAc and LacdiNAc structures. MGL-mFc binding was determined by an ELISA-based assay. Standard deviation <0.05 OD 450 nm. One representative experiment out of three is shown.

Example 4, Figure 2. MGL ligands can be detected in on the sinusoidal and lymphatic endothelium human lymph node and thymus. MGL-mFc (red) specifically interacted with the sinusoidal endothelium (LYVE-1, green) of lymph node (A) and with the lymphatic endothelium (LYVE-1, green) of the thymus (B) as determined by immunohistochemistry. No staining was observed in the presence of free GalNAc monosaccharides, showing the specificity of the MGL binding. Nuclei were visualized in blue. One out of two donors is shown. Original magnifications 200x and 400x.

Example 4, Figure 3. Distribution of MGLpos cells and sinusoidal or lymphatic endothelial cells in lymph node and thymus. Human tissue sections of lymph node and thymus were stained with anti-MGL (red) and LYVE-1 (green). Nuclei are visualized in blue. Sin. EC, sinusoidal endothelial cells, OZ, outer zone of the paracortex, Lymph. EC, lymphatic endothelial cells, C, cortex, IS, interlobular space. One out of two donors is shown.
Original magnification 400x.

Example 4, Figure 4. Endothelial structures express GalNAc epitopes as visualized by HPA staining. HPA (green) specifically interacted with the sinusoidal endothelium (LYVE-1, red) of LN (A) and lymphatic endothelium (LYVE-1, red) in thymus (B). Original magnification 400x.

Example 4, Figure 5. MGL specifically recognizes HMEC-1 endothelial cells. Binding of MGL-Fc to primary HUVEC (A) or HMEC-1 (B) was determined by flow cytometry. MGL-Fc binding to HMEC-1 can be blocked by EGTA, free GalNAc monosaccharides and anti-MGL antibodies. C, HMEC-1 cells express α-GalNAc, α1-2 linked fucose, α2-6 linked sialic acid and α2-3 linked sialic acid, as stained by the lectins HPA, UEA-1, SNA and MAA respectively. D, MGL-Fc binding can be partially blocked by the α-GalNAc-specific lectin HPA. * indicates a significant difference between the MGL-Fc binding and the MGL-Fc binding in the presence of HPA (p<0.05). All results are representative for three independent experiments.

Example 4, Figure 6. MGL mediates retention of immature DCs. A, MGL expression on immature DCs was determined by flow cytometry. Open histogram, isotype control; filled histogram MGL staining. B, Transendothelial migration of immature DCs across an HMEC-1 monolayer in response to a RANTES gradient in the presence of antibodies to MGL, β2-integrin or isotype control antibody. * indicates a significant difference between the isotype control and the anti-MGL (p<0.05). C and D, DC migration on glycoconjugate-coated plates was measured using time-lapse video microscopy. Anti-MGL antibodies increase DC migration on GalNAc, whereas they have no effect on the Lewis X coating. ** indicates a significant difference between isotype control and the anti-MGL antibodies (p<0.01). NS, not significant. All results are representative for three independent experiments.

### Figure legends to the figure of example 5

Example 5, Figure 1. Reaction scheme for synthesis of activated carbohydrate.

### Figure legends to the figures of example 6

Example 6, Figure 1. Carbohydrate specificity of mMGL1 and mMGL2. mMGL1-Fc and mMGL2-Fc binding to coated PAA-glycoconjugates was determined by ELISA.

Example 6, Figure 2. Binding of murine MGL1 expressed by CHO cells to carbohydrates. Binding of PAA-glycoconjugates (10 µg/ml) to CHO parental and CHO-mMGL1 transfectants was determined by flow cytometry.

Example 6, Figure 3. Lewis X is the high affinity ligand for mMGL1.. Binding of PAA-glycoconjugates (concentration range from 2-0.08 µg/ml) to CHO parental and CHO-mMGL1 transfectants was determined by flow cytometry.

Example 6, Figure 4. mMGL1 and mMGL2 bind Lewis X and GalNAc structures respectively. A) MGL-Fc binding was determined by glycan array analysis. B) Binding of bone marrow derived dendritic cells (BM-DC) to PAA-biotin conjugated glycans was analyzed by flow cytometry.

Example 6, Figure 5. Presentation of neo-glycoconjugates by BM-DC results in enhanced CD4 (A) and CD8 (B) T cell proliferation. Day 6 BM-DC were harvested and cultured with OVA-GalNAc and OVA-Lewis X for 5-6 hours. Cells were washed and cultured with OT-1 or OT-11 T cells for 48 hrs.
Culture was pulsed with ³H-thymidine for another 18 hrs. T cell proliferation was determined by measuring ³H-thymidine incorporation.

### Figure legends to the figures of example 7

Example 7, Figure 1. MGL specifically interacts with N. gonorrhoeae. A) Binding of MGL-Fc to coated N. gonorrhoeae was determined by ELISA. B and C) Binding of CHO and CHO-MGL transfectants to FITC-labeled N. gonorrhoeae (MOI 50) was determined by flow cytometry.

Example 7, Figure 2. Binding of N. gonorrhoeae to DCs. Binding of day 4 DCs to FITC-labeled N. gonorrhoeae (MOI 100) was determined by flow cytometry.

Example 7, Figure 3. Internalization of N. gonorrhoeae by DCs. DCs were incubated for various time points with FITC-labeled N. gonorrhoeae (MOI 100). Binding was determined in the absence (detecting extracellular and intracellular bacteria) or presence of 0.4% Tryphanblue (detecting only intracellular bacteria).

Example 7, Figure 4. N. gonorrhoeae induces DC maturation. DCs were incubated overnight at different MOI of N. gonorrhoeae. DC maturation was measured by flow cytometry using the maturation markers CD83, CD80 and CD86.

Example 7, Figure 5. N. gonorrhoeae induces macrophage activation. Macrophages were incubated overnight at different MOI of N. gonorrhoeae. Macrophage activation was measured by flow cytometry using the markers CD14, CD80 and CD86.

Example 7, Figure 6. Cytokine production of DCs and macrophages incubated with N. gonorrhoeae. DCs and macrophages were incubated overnight at different MOI of N. gonorrhoeae. Cytokine production was determined by ELISA.

Example 7, Figure 7. Cytokine mRNA levels in DCs incubated with N. gonorrhoeae. DCs were incubated overnight at different MOI of N. gonorrhoeae. Cytokine mRNA levels were determined by RT-PCR. Example 7, Figure 8. N. gonorrhoeae wildtype induces a Th2-inducing phenotype in DCs. DCs were incubated with TLR ligands or N. gonorrhoeae (MOI 100). After 48 hours mature DCs were cocultured with naive CD4+ T cells for an additional 14 days. T cells were restimulated with PMA and ionomycin and intracellular levels of IFNγ and IL-4 were determined by flow cytometry. Controls include: *E. coli* LPS (mixed Th1/Th2 response), LPS/PGE2 (Th2 response) and Poly I:C or Poly I:C/R837 (Th1 response).

Example 7, Figure 9. Core antigen of N. gonorrhoeae LOS (Erwin et al. J. Exp. Med 1996)

### Figure legends to the figures of example 8

Example 8, Figure 1. MGL-mediated immunomodulation via the MUC1-MGL interaction. A) MGL binding to MUC1 on transfected CHO cells, as measured by FACS. B) and C) Dexamethasone treated DC, incubated in the presence of CHO cells expressing MUC1. Cytokine production was measured by RT-PCR and is displayed as relative abundance compared to the housekeeping gene GAPDH, which served as an endogenous reference gene.

Example 8, Figure 2. Altered cytokine production in DCs incubated with anti-MGL and TLR-ligands. Cytokine production was measured by RT-PCR and displayed as relative abundance compared to the reference gene GAPDH.

Example 8, Figure 3. TGFβ secretion by dexamethasone treated DCs.

Example 8, Figure 4. CD86 expression by macrophages. Macrophages were stimulated for 20 hours with TLR-ligands in the presence or absence of anti-MGL. Subsequently the expression of maturation markers was determined by flow cytometry.

Example 8, Figure 5. Altered cytokine production in macrophages incubated with anti-MGL and TLR-ligands. Cytokine production was measured by RT-PCR and displayed as relative abundance compared to the reference gene GAPDH

### EXAMPLES

### EXAMPLE 1

### EXPERIMENTAL PROCEDURES

**Cells and cell lines.** All cell lines were maintained in RPMI containing 10% fetal calf's serum (Invitrogen, Carlsbad, CA) or Iscove's medium containing 5% fetal calf's serum. Immature DC were cultured for 4-7 days from monocytes obtained from buffy coats of healthy donors (Sanquin, Amsterdam) in the presence of IL-4 (500 U/ml) and GM-CSF (800 U/ml). DC were matured by addition of 1 µg/ml LPS (Salmonella typhosa, Sigma-Aldrich, St. Louis, MO). Mf were generated from monocytes by culturing in the presence of 1000 U/ml GM-CSF. Dexamethasone was added at 1 µM.

**Antibodies and reagents.** The following monoclonal antibodies were used: AZN-D1 (IgG1, anti-DC-SIGN20, SPV-L7 and NKI-L15 (anti-CD11a), MEM-59 (anti-CD43), MEM-28 (anti-CD45, kindly provided by dr. V. Horesji), D3/9 (anti-CD45), RP1/11 (anti-CD45RA), RP2/21 (anti-CD45RB), RP1/12 (anti-CD45RC, all kindly provided by dr. F. Sanchez-Madrid,17, GF4bio (anti-CD27, kindly provided by prof. dr. R. van Lier), T3b (stimulating anti-CD3,50, anti-CD45RO (UCHL-1, BD Biosciences, San Diego, CA), anti-CD4PE (clone 13B8.2, Beckman Coulter, Fullerton, CA), anti-CD8PE (clone B9.11, Beckman Coulter), 28-14-8 (mouse anti-mouse H2 Db), anti-CD1a (OKT6), anti-CD68 (EMB11, Dako, Glostrup Denmark), anti-CD163 (Edhu1), anti-Mannose receptor (3.29.B1), anti-CD11b (Bear-1) anti-CD11c (SHCL-3), anti-CD80 (BD Biosciences), anti-CD86 (BD Biosciences) and CD83 (Beckman Coulter). The anti-MGL antibodies 18E4 and 1G6.6 (both IgG2A) were generated by immunizing Balb/c mice with purified MGL-Fc. Hybridoma supernatants were screened for the presence of anti-MGL antibodies on CHO-MGL transfectants.

MGL-Fc and control Fc-proteins (ICAM-3-Fc or CAMEL-Fc) were generated as previously described¹³.

**Flowcytometry and MGL-Fc binding assays.** For determining surface expression, cells were incubated with primary antibody (5 µg/ml), followed by staining with a secondary FITC-labeled anti-mouse antibody (Zymed, San Francisco, CA) and analyzed on FACScalibur (BD Biosciences, San Diego, CA). To analyze MGL-ligand expression, cells were incubated with MGL-Fc (10 µg/ml) in TSA (20 mM Tris-HCl pH 7.4, 150 mM NaCl, 2 mM MgCl2, 1 mM CaCl2, 0.5% BSA) for 30 min at 37°C, followed by staining with a secondary FITC-labeled anti-human Fc antibody (Jackson, West grove, PA) and analyzed on FACScalibur. T cells were identified by co-staining for 10 min at RT with subset-specific markers.

**Immunohistochemistry.** Cryosections of healthy tissues (7µ) were fixed with 100% aceton and stained with primary antibodies (10 µg/ml) for 1 hour at 37°C. Sections were counterstained with isotype-specific Alexa488- or Alexa594-labeled anti-mouse antibodies (Molecular probes, Eugene, OR). Rheumatoid arthritis synovial biopsies were obtained after informed consent from patients with active RA undergoing arthroscopy.

**Cellular adhesion assays.** CHO and CHO-MGL were seeded 35.10³ cells/well in 96-wells plates. Calcein-AM (Molecular probes, Eugene, OR) labeled cells were added for the indicated times at 37°C. Non-adherent cells were removed by gentle washing. Adherent cells were lysed and fluorescence was quantified on a Fluorstar spectrofluorimeter (BMG Labtech, Offenburg, Germany).

**Immunoprecipitation and Western blot analysis.** Cells were surface-biotinylated with 0.5 mg/ml sulfo-NHS-biotin (Pierce, Rockford, IL) in PBS for 30 min at 4°C and subsequently lysed in lysisbuffer (10 mM TEA pH 8.2, 150 mM NaCl, 1 mM MgCl2, 1mM CaCl2, 1% Triton X-100, containing EDTA-free protease inhibitors (Roche Diagnotics, Penzberg Germany)) for 1 hour at 4°C. MGL ligands were immunoprecipitated using Fc-protein-coupled protein A-sepharose beads (CL-4B, Pharmacia, Uppsala, Sweden). The immunoprecipitations were seperated by 8% SDS-PAGE electrophoresis and transferred to nitrocellulose membranes. Blots were blocked with BSA and immunoblotted with streptavidin-coupled peroxidase (Vector Laboratories, Burlingame, CA) or specific antibodies followed by a secondary goat anti-mouse-Peroxidase (Jackson). Blots were developed by enhanced chemiluminescence.

**MGL-Fc ligand ELISA.** NUNC maxisorb plates (Roskilde, Denmark) were coated with goat anti-human-Fc antibody (4 µg/ml for 1 hour at 37°C, Jackson), followed by a 1% BSA blocking step (30 min at 37°C) and MGL-Fc (1 µg/ml for 1 hour at 37°C). MGL-Fc coated plates were incubated overnight at 4°C with cell lysates (10.10⁶ cells/ml for cell lines, 500.10⁶/ml for PBL). After extensive washing 1 µg/ml monoclonal antibodies were added for 2 hours at room temperature. Binding was detected using a peroxidase-labeled goat anti-mouse antibody (Jackson).

**CD45 phosphatase activity,** Lck phosphorylation and Ca2+-mobilization. Jurkat cells were stimulated on CHO or CHO-MGL for 20 min at 37°C, subsequently washed with ice-cold PBS and lysed in Ph lysis buffer (20 mM HEPES pH 7.2, 2 mM EDTA, 2 mM DTT, 1% Nonidet P-40, 10% glycerol containing protease inhibitors). Cellular debris and nuclear material were removed by centrifugating at 14000 rpm for 15 min at 4°C. CD45 was immunoprecipitated using MEM-28 and protein A-sepharose-beads. CD45 phosphastase activity was quantified by incubating the beads with 2 mM 4-Nitrophenyl phosphate (Roche, Basel, Switzerland) in CD45 Ph assay buffer (100 mM HEPES pH 7.2, 2 mM EDTA, 2 mM DTT) for 4 hours at 37°C. The resulting color change was measured at 410 nm. Specificity was determined by adding a specific CD45 phosphatase inhibitor (Calbiochem, Darmstadt, Germany). Serum starved Jurkat cells were incubated for indicated time points with MGL-Fc or Control-Fc and phosphorylation Lck Y505 was measured by flowcytometry using an Alexa Fluor 488-labeled antibody specific for the phosphorylated form of Lck Y505 (BD Biosciences) according to the manufacturers instructions. 5 mM H2O2 (15 min 37°C) was included as a positive control. Jurkat cells were loaded with the Ca2+ indicator Fluo-3-AM (Molecular probes), prewarmed at 37°C and analyzed by flowcytometry. Cells were stimulated with MGL-Fc coated beads or anti-CD3 (5 µg/ml).

**Cell death assay.** Jurkat cells were seeded 200.10³/24-well in a final volume of 500 µl and cultured overnight with MGL-Fc, control-Fc (10 µg/ml) with or without soluble anti-CD3 (2.5 µg/ml). Free GalNAc (50 mM) was added to block MGL function. Cellular apoptosis was determined on basis of FSC/SSC pattern and staining with Annexin V-FITC (BD Biosciences) and/or 7-AAD (Molecular probes).

**T cell proliferation and cytokine production.** 100.10³ PBL were seeded onto CHO or CHO-MGL and stimulated for 24 hours with soluble anti-CD3, or onto MGL-Fc (5 µg/ml)/anti-CD3 (1 µg/ml) or control-Fc/anti-CD3-coated plates and cultured for 24 hours. Cytokine levels in the supernatants were determined by ELISA according to the manufacturer's protocol (Biosource international,.Camarillo, CA). 100.10³ purified CD4+ T cells (>97% pure) were cultured with allogeneic APC at indicated ratio's for 4 days at 37°C. T cell proliferation was measured by [methyl-3H]Thymidine (Amersham biosciences, Uppsala, Sweden) incorporation.

### Statistical analysis

Student's t-test was used for statistical analysis. Significance was accepted at the p<0.05 level.

Results

### MGL is expressed on APC subsets in vivo and in vitro

Different APC subsets were cultured from human monocytes and analyzed for MGL expression. The CLR DC-SIGN was used as a well known general marker for DC and alternative active Macrophages (aaMf) ^{20,21}. MGL could be detected on immature DC and Mf, whereas its expression was enhanced both on the protein and mRNA level on immature DC and aaMf, generated in the presence of dexamethasone (Fig. 1a and van Vliet Immunobiology in press). LPS-induced DC maturation completely abolished MGL expression. MGL expression could not detected on other mature DC or activated Mf (Supplementary table 1). As in vitro cultured MGLpos APC, except immature Mf, co-expressed DC-SIGN, we examined whether in vivo MGL and DC-SIGN are found on identical APC subsets as well. Strikingly, in healthy lymph node and skin, MGL and DC-SIGN are expressed on separate APC subsets in the outer zones of the paracortex and dermis respectively, whereas in jejunum a complete cellular overlap between MGL and DC-SIGN expression was found (Fig. 1b). Further characterization of the MGLpos cells in skin and lymph node identifies them as myeloid-type APC (Supplementary table 2). To examine MGL expression in situ at sites of high number of infiltrating T cells, such as under chronic inflammatory conditions, synovial biopsies of four Rheumatoid Arthritis (RA) patients were stained for MGL and DC-SIGN expression. These biopsies were classified as low inflammatory RA with a diffuse infiltrate or high inflammatory RA with high numbers of leukocytes infiltrating the tissue. In the low inflammatory RA biopsies no MGL expression could be detected throughout the whole biopsy, whereas in the high inflammatory RA biopsy abundant MGL and DC-SIGN single- and double-positive cells with a bright CLR expression, were found (Fig. 1c and supplementary fig. 1). All RA biopsies contained high numbers of infiltrating Mf (data not shown).
Together these results show that MGL is preferentially expressed on dexamethasone-treated DC and Mf subets, as well as on APC in several human tissues. MGL levels appear to coincide with chronic inflammatory conditions.

### CD45 is the main counterreceptor for MGL on human T cells

To identify self-ligands a panel of human hematopoietic cell lines was screened for MGL binding. MGL-Fc, comprising of the extracellular domains of MGL fused to the human IgG1Fc-tail, strongly interacted with the erythroleukemic cell line K562 and with the T cell lines H9, HSB-2 and Jurkat (Fig. 2a and Supplementary table 3). MGL binding could be inhibited by addition of the Ca2+-chelator EGTA, free GalNAc monosaccharides and by anti-MGL antibodies, confirming the specificity of this interaction (Fig. 2a). To verify T cell binding, primary human PBMC were isolated and tested for MGL-Fc binding. MGL-Fc specifically recognized PBL, as shown by the complete block using EGTA (Fig. 2b). In addition to T cells, also some NK cells and B cells were stained by MGL-Fc, whereas monocytes were not recognized (data not shown). To confirm an interaction of MGL and T cells on a cellular level, cell-cell adhesion experiments of PBL and Jurkat to MGL-transfectants were performed. PBL specifically adhered to CHO-MGL, expressing high levels of MGL, as compared to the parental CHO cells. Also Jurkat cells firmly interacted with CHO-MGL, whereas only background adhesion was observed to CHO cells. (Fig. 2c).
To investigate the MGL counterreceptors we performed immunoprecipitations on lysates of cell-surface biotinylated Jurkat lysates with MGL-Fc. Immunoblotting for precipitated proteins identified MGL recognition of three membrane proteins of approximately 220, 190 and 110 kD (Fig. 2d). Based on the observed molecular weights and the heavy O-glycosylation we considered the mucin-type proteins CD43 (95-115 kD) and CD45 (180-220 kD), as primary candidates for the MGL counterreceptors. Immunoprecipitation of MGL ligands using MGL-Fc and immunoblot analysis with specific antibodies demonstrated that the upper two bands corresponded to two CD45 isoforms and the lower 110 kD band to CD43 (Fig. 2d). Following a similar procedure using cell surface biotinylation and immunoprecipitation with MGL-Fc, only one major MGL-ligand of approximately 200 kD was identified in PBL lysates. To investigate whether the immunoprecipitated band corresponded to CD45, the streptavidin-blot was stripped and re-probed with anti-CD45 or control antibodies. Immunoblotting with anti-CD45 antibodies clearly demonstrated CD45 to be the counterreceptor for MGL on human PBL (Fig. 2e). We never observed any MGL recognition of CD43 on PBL, probably due to the high content of sialic acid on lymphocyte CD43²², which inhibits recognition of GalNAc structures by MGL¹³.
To confirm these results we captured MGL ligands from lysates by MGL-Fc-coated plates and detected the counterreceptors with monoclonal antibodies. MGL-Fc specifically interacted with both CD43 and CD45 from Jurkat as shown by the complete inhibition with EGTA, whereas on PBL only CD45 was recognized and not CD43 (Supplementary fig. 2a). On all other cell lines studied that interacted strongly with MGL, CD45 constituted as the main counterreceptor for MGL (Supplementary table 3). MGL exclusively recognized CD45 from lymphocytes but not CD45 from other leukocytes, such as monocytes, immature DC and PMN (Supplementary fig. 2b).
The CD45 mRNA is subject to alternative splicing in the A-, B- and C-domains, giving rise to five different isoforms on human leukocytes (ABC, AB, BC, B and RO)^{15,23}. The different CD45 isoforms can be distinguished on the basis of their reactivity with CD45RA, RB or RC-specific moabs. None of these antibodies directed against the protein backbone, were capable of blocking the MGL interaction with specific glycan structures on CD45 (data not shown). To investigate MGL recognition of different CD45 isoforms we utilized single human CD45 isoform transfectants of the murine cell line 3001917. Flowcytometric analysis confirmed the expression of the correct CD45 isoform in the mouse transfectants (Table 1). To analyze binding to MGL, we employed the ELISA-based assay, in which the human CD45 isoforms were pulled down from lysates of the different transfectants with a panCD45 antibody, followed by a specific MGL-Fc detection step. MGL-Fc binding was observed to all A-, B- or C-containing isoforms, whereas CD45RO was not recognized by MGL, although it was efficiently captured from the lysate by the panCD45 antibody (Table 1 and data not shown). These results were confirmed for PBL, by capturing MGL-ligands from PBL lysates and probing these with anti-CD45 isoform-specific antibodies. Again no binding could be observed of MGL to CD45RO, whereas an interaction with A, B or C containing isoforms could readily be detected (Table 2). Thus, CD45 on human lymphocytes is the counterreceptor for MGL; whereby MGL is capable of interacting with all CD45 isoforms, except CD45RO. However expression of GalNAc epitopes on these CD45 isoforms constitutes a prerequisite for binding, a process probably regulated by T cell differentiation.

### MGL modulates CD45 phosphatase activity and TCR mediated signalling.

CD45 affects cellular responses by controlling the threshold of sensitivity to external stimuli, especially those received by the TCR. Therefore we investigated whether the MGL-CD45 interaction would influence CD45 phosphatase activity and function, using the Jurkat E6.1 cell line, which has proven to be an unsurpassed model for studying TCR-signaling pathways²⁴. CD45 phosphatase activity can be regulated via receptor dimerization, whereby increased dimerization results in a decreased activity²⁵. To achieve maximal receptor dimerization Jurkat cells were seeded on CHO or CHO-MGL, after which cells were lysed and the CD45 phosphatase activity was measured. MGL binding to CD45 resulted in a time-dependent decrease in CD45 phosphatase activity (Fig. 3a). No residual phosphatase activity in the presence of CD45-specifc phosphatase inhibitors was observed, confirming that the observed phosphatase activity was completely CD45-mediated. The first downstream target of CD45 in T cells is Lck. CD45 dephosphorylates the negative regulatory tyrosine Y505, resulting in an active pool of Lck. The reduced CD45 phosphatase activity after MGL-CD45 interaction, could lead to decreased Lck activity by enhanced phosphorylation on Y505. Indeed an increase in the phosphorylation status of Lck Y505 was observed 10-20 minutes after the addition of MGL-Fc, which acts as an agonistic agent mimicking binding to cellular MGL (Fig. 3b). Blocking MGL function with free GalNAc lowered Y505 phosphorylation to control and unstimulated levels. Engagement of the TCR leads to activation of downstream signaling pathways, including intracellular calcium mobilization, which plays a critical role in early T cell signaling transduction. Jurkat E6.1 cells were labeled with a calcium sensitive dye, Fluo-3/AM and stimulated with MGL-Fc coated beads and an anti-CD3 antibody. Within one sample the fluorescent beads were used to distinguish between cells that are subjected to MGL-mediated crosslinking or not. Binding of MGL-Fc beads alone did not trigger calcium mobilization, although the subsequent calcium flux triggered by the anti-CD3 antibody was strongly downregulated. The inhibition was not observed in Jurkat cells that had not bound MGL-Fc beads nor in Jurkat cells incubated with control-Fc beads (Fig. 3c). To verify that this effect was mediated by CD45, we employed the CD45neg Jurkat derivate, JS7 and the corresponding parental cell line D.J26. In contrast to D.J, JS7 completely lacked expression of CD45 (Fig. 3d). CD3 and CD43 were expressed at similar levels on both cell lines (data not shown). Both cell lines bound MGL-Fc with equal affinity; although on JS7 all binding is CD43-mediated (Fig. 3d and data not shown). Anti-CD3 triggered calcium mobilization was inhibited in D.J cells that had bound the MGL-Fc-beads. In contrast, in JS7 the calcium flux was only marginally lowered, probably due to the residual CD43-MGL interaction, as CD43 is able to modulate TCR-mediated responses19. These data confirm that the reduction in calcium mobilization is a direct effect of the MGL-CD45 interaction (Fig. 3e). In conclusion MGL can directly modulate CD45 activity and thereby negatively influence TCR-mediated signaling pathways.

### MGL-Fc induces cell death in a TCR- and CD45-dependent manner

Strikingly prolonged stimulation of Jurkat with anti-CD3 combined with MGL-Fc-induced crosslinking of CD45, led to enhanced cell death as shown by the shift in forward scatter and the reduction in the percentage of cells in the live-gate (Fig. 4a). MGL-Fc/anti-CD3 induced cell death resulted in exposure of phosphatidylserine as measured by staining with annexin V (Fig. 4b) and nuclear fragmentation (data not shown). Cell death was completely dependent on both CD3 triggering and MGL binding, as incubations with anti-CD3 or MGL-Fc alone had no effect. Occasionally some cell death was induced by MGL-Fc alone; however it was always markedly increased by TCR stimulation (data not shown). The observed apoptosis was MGL-specific as addition of free GalNAc could block all cell death and control Fc-proteins did not induce apoptosis (Fig. 4b). In contrast to D.J, cell death could not be triggered by anti-CD3/MGL-Fc in JS7, validating the complete CD45-dependence in the MGL-Fc-induced apoptosis (Fig. 4c).

### MGL modulates T cell function

To evaluate whether MGL can affect primary T cell activation as well, we investigated MGL recognition of different human T cell subsets within the CD4+ and CD8+ compartment. Naive, memory and effector T cells can readily be distinguished based on their differential expression of CD45RA and CD2727. Whereas naive T cells express both CD27 and CD45RA, memory T cells are characterized as CD27posCD45RAneg. Memory T cells are characterized by their expression of CD45RO, although expression of the CD45B-isoform is retained (¹⁷ and data not shown). Teff generally lose expression of CD27; however a number of Teff regain CD45RA. MGL displayed a strong preference for both CD27neg CD4+ and CD8+ Teff (Fig. 5a). MGL-Fc binding was specific as shown by the EGTA block.
To study the effect of MGL binding on T cell activation proliferative and cytokine responses of human lymphocytes were analyzed. PBL were seeded either on CHO or CHO-MGL and stimulated with anti-CD3. After 24 hours supernatants were harvested and analyzed for cytokine production. Both TNFα and IFNγ production were markedly reduced in the CD3-stimulated PBL-CHO-MGL co-culture compared to PBL-CHO co-culture (Fig. 5b). Equally, PBL stimulated on anti-CD3/MGL-Fc coated plates produced less IFNγ and TNFα compared to the anti-CD3/Control-Fc stimulated PBL (Fig. 5c). This effect was completely mediated by MGL as blocking MGL antibodies restored cytokine production to control levels. In both systems IL-6 production was not significantly affected, whereas IL-4, IL-5 or IL-10 could not be detected (data not shown). TNFα production was similarly reduced by MGL-Fc/anti-CD3 when purified CD4+ or CD8+ T cells were assayed instead of unfractionated PBL (supplementary fig. 3).
If MGL indeed has a downregulatory effect on T cell activation and thus on proliferation, blocking anti-MGL antibodies should revert this effect and enhance proliferation. Monocyte-derived APC expressing high or low levels of MGL (Fig. 1a) were cultured with highly purified allogeneic CD4+ T cells in the presence or absence of blocking MGL antibodies and after 4 days proliferation was measured by [methyl-3H]Thymidine incorporation. The different APC subsets could clearly be divided in immunogenic MGLlow/neg APC (immature DC, mature DC and Mf), capable of inducing high T cell proliferation and dexamethasone-treated MGLhigh APC, which lack the ability to stimulate strong T cell division, even at high APC:T cell ratios (Fig. 5d and 28). Anti-MGL antibodies had no effect on proliferation and cytokine production in the immunogenic APC-T cell co-cultures (Fig. 5d and data not shown). In contrast anti-MGL antibodies could enhance proliferation about 3-fold in the dexamethasone-APC-T cell co-cultures (Fig. 5d). This effect was not observed for two isotype-matched control antibodies, a blocking anti-LFA-1 and a non-binding isotype control (mouse anti-mouse H2 Db). Strikingly T cell proliferation by immature DC is not enhanced by antibodies against MGL in contrast to the dexDC. This is likely due to the higher levels of costimulatory molecules present on the immature DC (Supplementary table 4), which overrules any downregulatory effect of MGL. No cytokines could be detected in the supernatants of the dexamethasone-APC-T cell co-cultures. This indicates that blocking MGL function eliminates the inhibitory effect of MGL on CD45 and enhances T cell activation.
In conclusion, the CLR MGL on immature APC with a tolerogenic phenotype, specifically recognizes CD45 on Teff, thereby suppressing T cell activation.

### Experiments with a chimeric protein comprising the carbohydrate recognition domain of MGL and the multimerisation domain IgG1-Fc separated by a spacer

The above experiments are repeated using a construct containing merely the carbohydrate recognition domain of MGL coupled to IgG1-Fc by means of a short spacer.

### Effect of inhibition of the interaction between MGL and CD45 on autoimmune diseases.

MGL knock-out mice are made lacking the gene for MGL. Subsequently, it is determined whether the induction of EAE (Multiple Sclerosis (MS)-like disease in mice) is enhanced when the mice are vaccinated with MOG (myeline) peptides. The same experimental set-up can be followed for Rheumatoid Arthritis, and when vaccination takes place with collagen.

PBL of auto-immune patients, such as RA PBL or MS patients, were screened for upregulated expression of GalNAc on CD45. It is expected that addition of MGL-Fc will induce apoptosis.

### MGL binds to PBLs from chronic infected patients with RA ,OA and multiple sclerosis.

In supplementary figure 5 it is shown that MGL preferentially recognizes effector-type T cells as measured by 4-color analysis of MGL-Fc binding and co-staining with anti-CD4 or CD8 -specific antibodies. Standard deviation <5%. Frozen total PBLs were thawed and stained with MGL-Fc as earlier mentioned. Also plant lectins such as HPA, with specificity for GalNAc, Con A, high mannose and diantennary, and PNA, where used to determine glycan epitopes on subsets of cells.

Also HPA which has a similar glycan specificity as MGL showed enhanced staining of PBL from RA and OA patients. These results show that MGL-Fc can be used as a probe to detect chronic inflammatory status of patients that have RA and may be used for early detection of chronic inflammatory diseases.

PBL of autoimmune patients, here Multiple Sclerosis (MS) patients, were screened for upregulated GalNAc expression on CD45. It is expected that addition of MGL-Fc will induce apoptosis.
Supplementary figures 6-9 show the results of these experiments. Frozen PBL were thawed and stained with MGL-Fc (Figures 8 and 9). Also plant lectins such as HPA, with specificity for GalNAc, Con A, high mannose and diantennary glycans and PNA, TF antigen, were used to determine glycan epitopes present on subset of cells (Figures 6 and 7). Significant differences were observed between the MGL-Fc binding between SPMS and PPMS patients. In RRMS patients that were treated with IFNβ MGL binding increased. HD, healthy donors, SPMS, secondary progressive MS, PPMS, primary progressive MS, RRMS, relapsing-remitting MS.

### MGL recognizes a counter-receptor on mouse lymphocytes.

As the specificities of mouse MGL are overlapping with the human MGL, these mouse MGL molecules could share ligands as well. Therefore the recognition of mouse T cells and mouse CD45 was investigated to determine whether mMGL1 or 2 might interact with mouse T cells and mouse CD45, similar to human MGL.
Strikingly, the mMGL1 KO mouse has an elevated level of antigen experienced T cells, suggesting that the MGL-mediated control of effector T cells could be present in the mouse as well. Furthermore plant lectins such as HPA, with specificity for GalNAc, Con A, high mannose and diantennary glycans, PNA, TF antigen and LTA, α3-4 linked fucose, were used to determine glycan epitopes present on mouse lymphocytes. For these experiments control spleen and lymph node were used of uninfected control C57/B16 mice and MGL KO mice infected with several pathogens.
Supplementary figure 10 shows the lectin profiling. α-GalNAc epitopes, as measured by HPA staining are more abundant in spleen than in lymph node (LN). In infected mice this difference is less pronounced.
Next, beads coated with human or mouse MGL-Fc were incubated in the presence or absence of the Ca2+-chelator EGTA with mouse lymphocytes isolated from spleen and LN (for methods see methods section of example 2). Supplementary figure 11 shows the MGL recognition of mouse T cells. Specific binding of mMGL1-Fc coated beads was observed to all lymphocyte subpopulations tested.
In supplementary figure 12 the binding of soluble human and mouse MGL-Fc to mouse T cells was investigated. Human MGL-Fc binds T cells from uninfected control mice and from infected mice, however binding to the T cells from infected mice is much stronger, especially to T cells isolated from spleen. mMGL1-Fc shows low binding to T cells from LN and to splenic T cells from uninfected mice. mMGL1-Fc binding to splenic T cells from infected mice is increased. mMGL2-Fc does not bind mouse lymphocytes with the exception of splenic T cells from infected mice, which are recognized by mMGL2-Fc.
Thus, mMGL can recognize mouse effector T cells, as present during infection or inflammation, similar to the human situation.

### EXAMPLE 2

### MATERIALS AND METHODS

### Cells

Chinese hamster ovary cells (CHO) were cultured in RPMI containing 10% fetal bovine serum (FBS) and streptomycin/penicillin. Monocyte derived dendritic cells (MoDC) were cultured as described before 29, with modifications. In short, human blood monocytes were isolated from buffy coats by a Ficoll gradient step, followed by MACS beads isolation of CD14+ cells (Miltenyi Biotec, Bergisch Gladbach, Germany). The monocytes were cultured in the presence of IL-4 and GM-CSF (500 and 800 U/mL, respectively; ScheringPlough, Brussels, Belgium) for 4 to 6 days. The phenotype of immature DC was confirmed by flow cytometry (CD11bhigh, CD11chigh, ICAM-1high, CD80low, CD83low, CD86low). The DC were matured by incubation with lipopolysaccharide (LPS, Salmonella typhosa, 1 µg/mL, Sigma Aldrich, St. Louis, Mo). DC maturation was confirmed by CD80, CD83 and CD86 expression and DC purity was >90%.

### Recombinant MUC1-Fc fusion protein

MUC1-Fc containing 32 tandem repeats of MUC1 fused with the Fc part of murine IgG2a was produced in CHO-K1 cells as described before 30. Purified MUC1-Fc glycoprotein was treated sequentially with V. cholerae neuraminidase (Roche) and β-galactosidase from bovine testis (Sigma) to remove sialic acid and galactose residues. The resulting glycoform was determined by ELISA using glycan specific antibodies and lectins (see "Binding assay (ELISA)").

### MUC1 samples

Tissue samples from colon carcinoma were collected after surgical removal of the tumor with informed consent of the patients. Primary colon carcinoma tissue and the corresponding normal tissue were obtained from patients from resection specimens, following national and institutional ethical guidelines regarding the use of human tissues. Tissue samples were gently homogenized and digested in lysis buffer (TEA buffer containing 1% Triton-X-100, 2 mM CaCl2, 2 mM MgCl2) for 2 days at 4°C under rotation. The lysates were centrifuged at 14000 rpm for 15 min, supernatant collected and stored in aliquots at -80°C.

### Antibodies and CLR-Fc fusion proteins

The following antibodies were used: MUC1 specific antibodies (clone 214D4 provided by John Hilkens, Netherlands Cancer Institute, Amsterdam); this antibody was biotinylated using a biotinylation kit (Pierce, Rockford, IL, USA); DC-SIGN specific antibody AZN-D1 31; CD68 specific antibody EBM-11; FITC-labeled goat anti-mouse IgG (Jackson Immunoresearch, West Grove, PA); Peroxidase-labeled goat anti-human Fc (Jackson Immunoresearch); Alexa-488 labeled goat anti-mouse IgG1 and Alexa-594 labeled goat anti-mouse IgG2a (Molecular Probes). Anti-glycan antibodies 5E5 (MUC1-Tn) and HH8 (MUC1-TF) were provided by Henrik Clausen, University of Copenhagen, Denmark. The MGL-human Fc fusion protein was made in the following way: the extracellular part of MGL (amino acids 61-289) was amplified on pRc/CMV-MGL by PCR, confirmed by sequence analysis and fused at the C-terminus to human IgG1-Fc in the Sig-pIG1-vector 28. MGL-Fc was produced by transfection of CHO cells and concentration determined by ELISA. An MGL-murine Fc fusion protein was generated by cloning the extracellular part of MGL (including the CD38 signal sequence from the Sig-pIG1 vector) into an pcDNA3 expression vector containing exon 1-3 of murine IgG2a-Fc 30.
The 18E4 monoclonal antibody to MGL was generated by immunizing Balb/c mice with purified MGL-murine Fc. Hybridoma supernatants were screened for the presence of anti-MGL antibodies on CHO-MGL transfected cells (S. van Vliet, submitted).

### Beads adhesion assay

Carboxylate-modified TransFluorSpheres (488/645 nm, 1.0 µm; Molecular Probes, Eugene, OR) were coated with streptavidin as previously described 32. Streptavidin coated beads were incubated with biotinylated F(ab')2 fragments of goat anti-mouse IgG2a (6 µg/ml, Jackson Immunoresearch) in 0.5 mL PBS 0.5% BSA for 2 hours at 37°C, washed and further incubated with MUC1-Fc fusion proteins at 4°C overnight. Finally, beads were washed, stored in 100 µl PBS 0.5% BSA, and used within one week. The beads adhesion assay was performed as previously described 32. DC were incubated with coated beads in Tris-sodium buffer (20 mM Tris-HCl, pH 7, 150 mM NaCl, 2 mM CaCl2, 2 mM MgCl2) with 0.5% BSA and adhesion was determined in the absence or presence of blocking agents (10 mM EGTA, 25 mM monosaccharides (Sigma) or 5 mM mannan) at 37°C for 45 min. Cells were washed and analysed by flow cytometry.

### Binding assay (ELISA)

The solid phase adhesion assay was performed by coating ELISA plates with purified anti-MUC1 antibodies (clone 214D4) in 0.2 M Na2CO3, followed by blocking with 1% BSA in Tris-sodium buffer. Negative controls were included for each sample, where no anti-MUC1 antibodies were added. After washing with Tris-sodium buffer, tissue lysates were added and incubated at 4°C overnight. Plates were washed and further incubated with MGL-human Fc (0.5 µg/mL) or biotinylated HPA (Sigma), dioxygene labeled MAA (Boehringer Mannheim) or anti-TF-antibodies (clone HH8) for 1 h at room temperature (RT). After washing with Tris-sodium-0.02% tween the binding was detected by peroxidase labeled anti-human IgG, -streptavidin (Jackson Immunoresearch), -anti-dioxygene antibodies or -anti-murine IgG. Specificity was determined in the presence of 10 mM EGTA or 100 mM GalNAc monosaccharides. MUC1 coating was determined by biotinylated anti-MUC1 antibodies (clone 214D4, 2 µg/mL) followed by incubation with peroxidase-labeled streptavidin.

### Immunofluorescence analysis

Cryosections (7µ) of normal colon epithelium or primary colon carcinoma were fixed in 100% aceton (10 min), washed with PBS, and incubated with first antibody (10 µg/ml) for 1h at 37°C. After washing, the final staining was performed with Alexa-594 labeled goat-anti mouse IgG2a or Alexa-488 labeled goat-anti mouse IgG1 and nuclear staining was performed with Hoechst.
For staining with MGL-Fc, cryosections were fixed in 2% paraformaldehyde and all incubation steps were performed in Tris-sodium buffer. The final staining was performed with Alexa-488 labeled goat-anti mouse IgG.

### Statistical analysis

Statistical differences between groups were analysed by Wilcoxon Matched Pairs test using Graphpad Instat from Graphpad Software, Inc. Correlation was analysed by a non-parametric Spearman ranks test. Significance was accepted at the p < 0.05 level.

### Lectins, antibodies and Fc-chimeric proteins

Helix pomatia agglutinin (HPA, Helix pomatia) was obtained from Sigma-Aldrich (St. Louis, MO, USA). The extracellular domain of MGL (amino acid residues 61-289) was fused with the Fc domain of human IgG1. The following antibodies were used: MUC1 antibodies 214D4 (IgG1 isotype, provided by John Hilkens, Netherlands Cancer Institute, Amsterdam); Peroxidase-labeled goat anti-human Fc (Jackson Immunoresearch, West Grove, PA); Peroxidase-labeled streptavidin.

### Binding assay

The solid phase adhesion assay was performed by coating ELISA plates (Maxisorp, Nunc) directly with milk samples, diluted in 0.2 M Na₂CO₃ and incubated overnight at 4°C. The plates were washed and incubated with Tris-sodium buffer (20 mM Tris-HCl, pH 7, 150 mM NaCl, 2 mM CaCl₂, 2 mM MgCl₂) containing 1% BSA. For MUC1 specific ELISA, anti-MUC1 antibodies (214D4, 10 µg/ml) were coated in 0.2 M Na₂CO₃, followed by blocking with BSA and incubation with milk (1:100) overnight at 4°C. Plates were washed and further incubated with MGL-Fc supernatant (0.5 µg/ml) or biotinylated lectins (5 µg/ml) for 2 h at room temperature. Unbound lectins were washed away with Tris-sodium-0.02% tween and binding was detected by peroxidase labeled anti-human Fc or peroxidase-labeled streptavidin. MUC1 coating was detected by biotinylated anti-MUC1 antibodies (214D4, 2 µg/mL), followed by incubation with peroxidase-labeled streptavidin. The reaction was developed by TMB substrate and optical density measured by a spectrophotometer.

### RESULTS

### Characterisation of MUC1-Fc fusion proteins

The extracellular domain of MUC1, including 32 tandem repeats with 5 O-glycosylation sites each, was fused to a murine IgG Fc tail and purified in Chinese hamster ovary cells, as previously described 30. To obtain a glycoform of MUC1 with mainly Tn-antigen, the protein was desialylated and treated with β-galactosidase, leaving only the core GalNAcs on the protein. The resulting MUC1-Tn reacted with antibodies against Tn, but not against TF in ELISA (Figure 1). These results indicate that the glycosylation mainly consisted of GalNAcs.

### MUC1-Fc containing Tn-antigens interacts with monocyte derived dendritic cells

To study the potency of tumor associated MUC1 to target DC, MUC1-Fc containing Tn-antigens (GalNAcaSer/Thr) was coupled to fluorescent beads and validated for interaction with monocyte derived DC (moDC). Both immature DC and DC matured with lipopolysaccharide were analysed for binding. MUC1-Tn bound efficiently to immature DC and binding was calcium dependent, suggesting a role for CLR (Figure 2A). Mature DC showed strongly reduced interaction with MUC1-Tn, concomitant with the fact that mature DC down-regulate expression of CLR on their cell surface (Figure 2A). Fluorescent beads coated with polyacrylamide-coupled Lewis-X, a carbohydrate epitope for the CLR DC-SIGN 33, served as a positive control. As expected, Lewis-X bound strongly to immature DC that express high levels of the CLR DC-SIGN and binding to mature DC, that express low levels of DC-SIGN, was significantly reduced (Figure 2B). Beads coated only with goat anti-mouse antibodies did not bind to moDC (data now shown).
To determine the specificity of MUC1 binding, DC were pre-incubated with different carbohydrate monosaccharides to block interaction with CLR (Figure 3A). Only N-acetylgalactosamine (GalNAc) monosaccharides partially blocked binding of MUC1-Tn to DC, suggesting the involvement of a GalNAc-specific receptor (see below). No block was observed by pre-incubation with galactose, mannose or mannan, suggesting that CLR such as DC-SIGN or mannose receptor were not involved. Furthermore, MUC1 binding was abrogated after DC maturation, indicating down-modulation of the interacting receptor following DC maturation (Figure 2A).
A candidate C-type lectin, expressed on immature DC and macrophages, with specificity for GalNAc carbohydrate structures 28,34, is the Macrophage Galactose-type C-type lectin (MGL) 27. MGL expression is down-modulated upon DC maturation (Figure 3B), coinciding with loss of MUC1 binding. Pre-incubation of moDC with anti-MGL antibodies reduced binding of MUC1-Tn, indicating that MGL is a receptor for this MUC1 glycoform, although the block was not complete. EGTA, which inhibits the lectin domain of CLR to recognize glycans also inhibited the interaction to the same level as anti-MGL antibodies. We therefore concluded that all CLR binding activity was mediated by MGL (Figure 3C). Neither anti-DC-SIGN nor anti-mannose receptor antibodies blocked this interaction. The specificity of MUC1-Tn for MGL was confirmed using MGL transfected Chinese Hamster Ovary (CHO) cells (Figure 4A,B). MUC1-Tn bound strongly to MGL transfected cells and interaction was blocked by EGTA, GalNAc, or anti-MGL blocking antibodies,confirming the specificity of the interaction (Figure 4B). Together, these data strongly indicate that MGL on DC specifically interacts with tumor associated MUC1, in particular the Tn-antigen.

### MGL binds MUC1 derived from primary colon carcinoma

To investigate whether MGL specifically recognizes MUC1 in primary tumors, tissue samples (n=20) were obtained after surgical removal of colorectal tumors from carcinoma patients. The surgical procedure was performed at the VU University Medical Center (VUmc), Amsterdam, the Netherlands and both normal and malignant tissue was obtained from each patient. Total protein concentration was measured in the tissue lysates and found to be significantly higher in the tumor lysates (p<0.001) than in normal tissue lysates, probably due to differences in protein content. MUC1 concentration was also significantly higher (p<0.0001) in tumor tissue lysates (Figure 5A and 5B), however, there was no correlation between total protein concentration and concentration of MUC1.

When we analysed MGL-Fc binding to tumor MUC1, binding was significantly higher than to MUC1 out of normal epithelial tissue from the same patient (p<0.0001). Low binding to MUC1 of normal epithelium could be due to low MUC1 concentration in normal colon lysates. Interestingly, however, MGL-Fc binding and MUC1 concentration in tumor samples did not correlate (r=0.25; p=0.28, Figures 5A and B) suggesting that there were qualitative differences present that determined MGL-Fc interaction with tumor associated MUC1. To investigate this further, we analysed the carbohydrate content of isolated MUC1 from tumor samples and normal epithelial tissues using plant lectins and carbohydrate specific antibodies. As expected, MUC1 samples varied extensively in carbohydrate reactivity between the different colorectal cancer patients. Some contained Tn-antigens, others TF-antigens or sialylated structures. Interestingly, a strong correlation was observed between MGL-Fc binding and HPA binding (r=0.88; p<0.0001), indicating that MGL primarily reacts with Tn antigens on MUC1 in primary colon carcinoma (Figure 5C). In contrast, no correlation was observed between MGL-Fc binding and the presence of TF-antigens or α2-3 sialic acid (Figures 5D and E). These data show that both the in vitro-generated Tn glycoform of MUC1 and the in situ tumor associated MUC1 present in colorectal cancer patients bind strongly to MGL on immature DC.

### MGL-expressing cells are detected in the colon

To determine whether MGL expressing cells are present in colon tissue and whether these could interact with MUC1 in tumors, tissue sections were analysed for cells expressing MGL and MUC1. MGL expressing cells were detected within the normal colonic mucosa of 4 individuals analysed as well as in primary tumor sections of all these individuals. MGL-expressing cells did not appear to express the macrophage marker CD68 in the tumor sections (Figure 6A), although some co-localization was observed with the CLR DC-SIGN, suggesting that MGL may be expressed on certain DC populations (Figure 6B).
In normal tissue, MUC1 positive cells were detected at the epithelial layer and some MUC1 positive cells were scattered throughout the mucosa (Figures 6C and 6D). However, as expected MUC1 staining was strong in the tumor tissue. The finding that the MGL expressing cells are located in the vicinity of MUC1 expressing cells demonstrate that MGL positive antigen presenting cells are present in close contact with epithelial tumor cells. To determine whether tumors expressed MGL-binding glycan epitopes, we analysed the binding activity of MGL-Fc to these tissues. MGL-Fc showed strong staining of colon carcinoma cells and not with normal colon epithelium (Figure 7). Furthermore the fact that the binding of MGL-Fc was blocked by GalNAc monosaccharides, demonstrates the requirement of the carbohydrate recognition domain for staining.

### MGL-Fc detects secreted MUC1 from tumor cellline ZR75

This experiment is depicted in figure 8 of example 2 In figure 8 A MUC1 was harvested out of the supernatant of cultured ZR75 tumor cells and coated on ELISA plates. Both MGLFc and DC-SIGNFc were used to look for carbohydrate binding epitopes expressed on tumor associated MUC1. As illustrated MGLFc binds strongly the secreted tumor associated MUC1 whereas DC-SIGN does not bind. Furthermore EGTA which inhibits lectin binding to carbohydrates illustrates that the recognition of MGL to MUC1 is mediated by the expression of carbohydrates.

In a second experiment the tumor associated MUC1 were coated on fluorescentbeads coated with MUC1 antibodies (Figure 8 of example 2. Fluorescent MUC1 coupled beads where added to MGL expressing CHO cells and untransfected CHO cells. As demonstrated the Fluorescent beads loaded with tumor associated secreted MUC1 bound strongly to MGL expressing cells which could be blocked by EGTA and the glycan GalNAc which specifically binds MGL

### Interaction of MGL with the epithelial mucin MUC1 in milk of allergic mothers

Breast milk is a rich source of glycosylated MUC1. Here we investigated the carbohydrate content of MUC1 from healthy or allergic mothers to determine whether differences in glycosylation can be observed because of disease status. For example 2 figure 10, MUC1 was captured in ELISA from milk of 10 healthy mothers and 10 allergic mothers. Figure 10A and B show the MUC1 concentrations in milk, which is not significantly different between healthy and allergic mothers. However, MUC1 from allergic mothers displays a higher MGL binding (Figure 10C), which can be explained by the higher exposure of α-GalNAc epitopes as measured by the α-GalNAc-specific lectin HPA (Figure 10D).

### DISCUSSION

The epithelial mucin MUC1 is a tumor antigen where post translational modification is altered during malignant transformation ¹⁴. The protein backbone is retained, but O-linked glycan structures are modified. This creates epitopes that may be recognized by the immune system, including the protein backbone and carbohydrate structures generally not present in non-malignant tissues. Adenocarcinoma patients may develop humoral and cellular immune responses to the MUC1 protein. These responses are, however, weak and not sufficient to eradicate the tumor ^{35,36}. Nonetheless, anti-MUC1 antibody levels have been shown to correlate with survival ³⁷ indicating the importance of this tumor antigen for protection against cancer.

Here, we have studied how tumor related MUC1 containing Tn-antigens may be optimally targeted to DC. DC play a key role in the initiation of immune responses and DC-based tumor vaccines are a promising tool for immunotherapeutic strategies against cancer. Therefore, it is important to understand the molecular mechanisms involved in how tumor antigens are recognized and internalised by DC to trigger efficient anti-tumor immune responses.
Using recombinant MUC1-Fc proteins containing 32-tandem repeats of the extracellular domain, and a sensitive flow cytometry-based binding assay, we demonstrated that MUC1-Tn bound efficiently to DC. The interaction was calcium dependent and was significantly contributed by the CLR MGL, that is expressed on immature DC, a receptor for GalNAc containing carbohydrate structures ^{28,38}. DC maturation resulted in abrogation of MUC1-Tn binding coinciding with loss of MGL expression. We did not observe any involvement of the mannose receptor, that has previously been described to be a receptor for glycosylated MUC1 ³⁹. The discrepancy between the two studies is likely due to differences in MUC1 glycoforms. The MUC1 glycoforms studied by Hiltbold et al., may have contained additional glycan epitopes that target the mannose receptor. The glycoform of MUC1 in our study, primarily contained GalNAc moieties that do not interact with the mannose receptor.
We demonstrated MGL-Fc interaction with MUC1 present in tissue samples of primary colon carcinoma patients (n=20) that strikingly correlated strongly with Tn-antigen expression (binding by the lectin Helix pomatia agglutinin) whereas no correlation was observed with expression of TF-antigens or sialylated MUC1. Tn-antigens are known to be highly expressed in colon carcinoma, but not in normal colon tissue ¹⁶. Detection of these carbohydrate epitopes by HPA (a GalNAc-specific lectin from the snail *Helix pomatia*) has been shown to be useful for identifying adenocarcinomas with metastatic potential ^{40,41}. Primary tumors of epithelial origin, which are stained positively with HPA, are more likely to metastasize and this is highly correlated with poor prognosis. It further suggests that these epitopes may play is in favor for its function to be involved in antigen uptake and presentation. an important role during metastasis ^{17,18} although it is not clear how this may be achieved or whether there is a role for such structures in tumor cell survival. Interaction of these carbohydrate structures with antigen presenting cells may promote efficient antigen uptake and presentation to T-cells. However, signalling that occurs during tumor antigen uptake may significantly contribute to the quality of the T-cell response. For example, antigen uptake in the absence of Toll-like receptor signalling may result in immunological tolerance ^{42.} where tumor cells may evade immune responses. The finding that MGL contains internalization motifs in its cytoplasmic tail similar to the MR and DC-SIGN, and can internalize GalNAc containing antigens ²⁷ We demonstrated the presence of MGL positive cells both within normal colon mucosa and within primary carcinomas, demonstrating that these cells may be in close contact with the MUC-Tn expressing tumor cells. Interestingly, these cells seemed to exhibit DC-like phenotype, and they did not express the macrophage marker CD68. MGL-Fc bound strongly to colon carcinoma cells, supporting the hypothesis that MGL positive cells may interact with tumor cells.

Tumor cells may shed MUC1 into the micromilieu and it may be detected in serum of cancer patients ⁴. These forms of MUC1 lack a cytoplasmic tail and can (in advanced colon cancer patients) reach such high levels that they can interfere with cell interactions, including selectin interactions ⁴³. These shed forms of MUC1 thus have the possibility to interfere with the immune system and help the tumor cells escape immune surveillance ^{43,44}. Interaction with MGL on antigen presenting cells may similarily influence immune responses. It is interesting to mention that MGL is expressed on human immature DC and in particular highly expressed on tolerogenic DC and/or macrophages that are cultured in the presence of dexamethazone (S. van Vliet, submitted).
In conclusion, our data demonstrate that immature DC may interact with tumor associated glycoforms of MUC1 via the CLR MGL. Our finding that MGL binding to MUC1 highly correlates with HPA binding, and the fact that MGL is highly expressed on immature or tolerigenic APC, suggests that this interaction leads to immunosuppressive effects.

### References to example 2

1. Baeckstrom D, Hansson GC, Nilsson O et al. Purification and characterization of a membrane-bound and a secreted mucin-type glycoprotein carrying the carcinoma-associated sialyl-Lea epitope on distinct core proteins. J Biol Chem. 1991;266:21537-47.
2. Gendler SJ, Burchell JM, Duhig T et al. Cloning of Partial Cdna-Encoding Differentiation and Tumor-Associated Mucin Glycoproteins Expressed by Human Mammary Epithelium. PNAS. 1987;84:6060-4.
3. Lan MS, Batra SK, Qi WN, Metzgar RS, Hollingsworth MA. Cloning and Sequencing of A Human Pancreatic Tumor Mucin Cdna. J Biol Chem. 1990;265:15294-9.
4. Hollingsworth MA, Swanson BJ. Mucins in cancer: protection and control of the cell surface. Nat Rev Cancer. 2004;4:45-60.
5. Agrawal B, Krantz MJ, Parker J, Longenecker BM. Expression of MUC1 mucin on activated human T cells: implications for a role of MUC1 in normal immune regulation. Cancer Res. 1998;58:4079-81.
6. Cloosen S, Thio M, Vanclee A et al. Mucin-1 is expressed on dendritic cells, both in vitro and in vivo. Int Immunol. 2004;16:1561-71.
7. Wykes M, MacDonald KP, Tran M et al. MUC1 epithelial mucin (CD227) is expressed by activated dendritic cells. J Leukoc Biol. 2002;72:692-701.
8. Correa I, Plunkett T, Vlad A et al. Form and pattern of MUC1 expression on T cells activated in vivo or in vitro suggests a function in T-cell migration. Immunology. 2003;108:32-41.
9. Nakamori S, Ota DM, Cleary KR, Shirotani K, Irimura T. MUC1 mucin expression as a marker of progression and metastasis of human colorectal carcinoma. Gastroenterol. 1994;106:353-61.
10. Boland CR, Deshmukh GD. The carbohydrate composition of mucin in colonic cancer. Gastroenterol. 1990;98:1170-7.
11. Dalziel M, Whitehouse C, McFarlane I et al. The Relative Activities of the C2GnT1 and ST3Gal-I Glycosyltransferases Determine O-Glycan Structure and Expression of a Tumor-associated Epitope on MUC1. J Biol Chem. 2001;276:11007-15.
12. Hanisch FG, Uhlenbruck G, Peter-Katalinic J et al. Structures of neutral O-linked polylactosaminoglycans on human skim milk mucins. A novel type of linearly extended poly-N-acetyllactosamine backbones with Gal beta(1-4)GlcNAc beta(1-6) repeating units. J Biol Chem. 1989;264:872-83.
13. Hanisch FG, Stadie TR, Deutzmann F, Peter-Katalinic J. MUC1 glycoforms in breast cancer-cell line T47D as a model for carcinoma-associated alterations of 0-glycosylation. Eur J Biochem. 1996;236:318-27.
14. Lloyd KO, Burchell J, Kudryashov V, Yin BW, Taylor-Papadimitriou J. Comparison of O-linked carbohydrate chains in MUC-1 mucin from normal breast epithelial cell lines and breast carcinoma cell lines. Demonstration of simpler and fewer glycan chains in tumor cells. J Biol Chem. 1996;271:33325-34.
15. Cao Y, Stosiek P, Springer GF, Karsten U. Thomsen-Friedenreich-related carbohydrate antigens in normal adult human tissues: a systematic and comparative study. Histochem Cell Biol. 1996;106:197-207.
16. Itzkowitz SH, Yuan M, Montgomery CK et al. Expression of Tn, sialosyl-Tn, and T antigens in human colon cancer. Cancer Res. 1989;49:197-204.
17. Schumacher U, Higgs D, Loizidou M et al. Helix pomatia agglutinin binding is a useful prognostic indicator in colorectal carcinoma. Cancer. 1994;74:3104-7.
18. Brooks SA, Leathem AJ. Prediction of lymph node involvement in breast cancer by detection of altered glycosylation in the primary tumour. Lancet. 1991;338:71-4.
19. Kakeji Y, Tsujitani S, Mori M, Maehara Y, Sugimachi K. Helix pomatia agglutinin binding activity is a predictor of survival time for patients with gastric carcinoma. Cancer. 1991;68:2438-42.
20. Figdor CG, van Kooyk Y, Adema GJ. C-type lectin receptors on dendritic cells and Langerhans cells. Nature Rev Immunol. 2002;2:77-84.
21. Geijtenbeek TB, van Vliet SJ, Koppel EA et al. Mycobacteria Target DC-SIGN to Suppress Dendritic Cell Function. J Exp Med. 2003;197:7-17.
22. Chieppa M, Bianchi G, Doni A et al. Cross-linking of the mannose receptor on monocyte-derived dendritic cells activates an anti-inflammatory immunosuppressive program. J Immunol. 2003;171:4552-60.
23. Bonifaz L, Bonnyay D, Mahnke K et al. Efficient targeting of protein antigen to the dendritic cell receptor DEC-205 in the steady state leads to antigen presentation on major histocompatibility complex class I products and peripheral CD8+ T cell tolerance. J Exp Med. 2002;196:1627-38.
24. Steinman RM, Hawiger D, Liu K et al. Dendritic cell function in vivo during the steady state: A role in peripheral tolerance. Immune Mechanisms and Disease. 2003;987:15-25.
25. van Gisbergen KPJM, Aarnoudse CA, Meijer GA, Geijtenbeek TBH, van Kooyk Y. Dendritic cells recognize tumor-specific glycosylation of carcinoembryonic antigen on colorectal cancer cells through dendritic cell-specific intercellular adhesion molecule-3-grabbing nonintegrin. Cancer Res. 2005;65:5935-44.
26. Mungul A, Cooper L, Brockhausen I et al. Sialylated core 1 based O-linked glycans enhance the growth rate of mammary carcinoma cells in MUC1 transgenic mice. Int J Oncol. 2004;25:937-43.
27. Higashi N, Fujioka K, Denda-Nagai K et al. The macrophage C-type lectin specific for galactose/N- acetylgalactosamine is an endocytic receptor expressed on monocyte- derived immature dendritic cells. J Biol Chem. 2002;277:20686-93.
28. van Vliet SJ, van Liempt E, Saeland E et al. Carbohydrate profiling reveals a distinctive role for the C-type lectin MGL in the recognition of helminth parasites and tumor antigens by dendritic cells. Int Immunol. 2005;17:661-9.
29. Sallusto F, Lanzavecchia A. Efficient presentation of soluble antigen by cultured human dendritic cells is maintained by granulocyte/macrophage colony-stimulating factor plus interleukin 4 and downregulated by tumor necrosis factor alpha. J Exp Med. 1994;179:1109-18.
30. Backstrom M, Link T, Olson FJ et al. Recombinant MUC1 mucin with a breast cancer-like O-glycosylation produced in large amounts in Chinese-hamster ovary cells. Biochem J. 2003;376:677-86.
31. Geijtenbeek TBH, Torensma R, van Vliet SJ et al. Identification of DC-SIGN, a novel dendritic cell-specific ICAM-3 receptor that supports primary immune responses. Cell. 2000;100:575-85.
32. Geijtenbeek TBH, van Kooyk Y, van Vliet SJ et al. High frequency of adhesion defects in B-lineage acute lymphoblastic leukemia. Blood. 1999;94:754-64.
33. Van Die I, van Vliet SJ, Kwame Nyame A et al. The dendritic cell specific C-type lectin DC-SIGN is a receptor for Schistosoma mansoni egg antigens and recognizes the glycan antigen Lewis-x. Glycobiology. 2003. 13;471-8.
34. Iida S, Yamamoto K, Irimura T. Interaction of human macrophage C-type lectin with O-linked N-acetylgalactosamine residues on mucin glycopeptides. J Biol Chem. 1999;274:10697-705.
35. Jerome KR, Barnd DL, Bendt KM et al. Cytotoxic T-lymphocytes derived from patients with breast adenocarcinoma recognize an epitope present on the protein core of a mucin molecule preferentially expressed by malignant cells. Cancer Res. 1991;51:2908-16.
36. Kotera Y, Fontenot JD, Pecher G, Metzgar RS, Finn OJ. Humoral immunity against a tandem repeat epitope of human mucin MUC-1 in sera from breast, pancreatic, and colon cancer patients. Cancer Res. 1994;54:56-2860.
37. Mensdorff-Pouilly S, Verstraeten AA, Kenemans P et al. Survival in early breast cancer patients is favorably influenced by a natural humoral immune response to polymorphic epithelial mucin. J Clin Oncol. 2000;18:574-83.
38. Suzuki N, Yamamoto K, Toyoshima S, Osawa T, Irimura T. Molecular cloning and expression of cDNA encoding human macrophage C- type lectin. Its unique carbohydrate binding specificity for Tn antigen. J Immunol. 1996;156:128-35.
39. Hiltbold EM, Vlad AM, Ciborowski P, Watkins SC, Finn OJ. The mechanism of unresponsiveness to circulating tumor antigen MUC1 is a block in intracellular sorting and processing by dendritic cells. J Immunol. 2000;165:3730-41.
40. Dwek MV, Ross HA, Streets AJ et al. Helix pomatia agglutinin lectin-binding oligosaccharides of aggressive breast cancer. Int J Cancer. 2001;95:79-85.
41. Laack E, Nikbakht H, Peters A et al. Lectin histochemistry of resected adenocarcinoma of the lung: helix pomatia agglutinin binding is an independent prognostic factor. Am J Pathol. 2002;160:1001-8.
42. Steinman RM, Nussenzweig MC. Avoiding horror autotoxicus: The importance of dendritic cells in peripheral T cell tolerance. PNAS. 2002;99:351-8.
43. Zhang K, Baeckstrom D, Brevinge H, Hansson GC. Secreted MUC1 mucins lacking their cytoplasmic part and carrying sialyl-Lewis a and x epitopes from a tumor cell line and sera of colon carcinoma patients can inhibit HL-60 leukocyte adhesion to E-selectin-expressing endothelial cells. J Cell Biochem. 1996;60:538-49.
44. Zhang K, Sikut R, Hansson GC. A MUC1 mucin secreted from a colon carcinoma cell line inhibits target cell lysis by natural killer cells. Cell Immunol. 1997;176:158-65.

**Table 1 MGL recognition of CD45 isoforms**

| | | | Antibody | staining | | | MGL-Fc binding |
|---|---|---|---|---|---|---|---|
| 30019 cells | Isotype | panCD45 | CD45RA | CD45RB | CD45RC | CD45RO | |
| Mock | - | - | - | - | - | - | - |
| CD45ABC | - | + | + | + | + | - | ++ |
| CD45AB | - | + | + | + | - | - | ++ |
| CD45BC | - | + | - | + | + | - | ++ |
| CD45B | - | + | - | + | - | - | ++ |
| CD45RO | - | + | - | - | - | + | - |

| | | | | | | | |
|---|---|---|---|---|---|---|---|
| CD45-isoform transfectants were checked for expression by flowcytometry using A-, B- or C-domain-specific antibodies. -, no staining, +, positive staining. The different CD45 isoforms were pulled down with a panCD45 antibody and probed using MGL-Fc. Specificity was determined in the presence of EGTA (data not shown). -, no binding, ++, high binding (OD 450nm>0.25). | | | | | | | |

**Table 2 MGL-Fc recognition of CD45 isoforms on PBL**

| Capture | | | Detection | | | |
|---|---|---|---|---|---|---|
| | Isotype | panCD45 | CD45RA | CD45RB | CD45RC | CD45RO |
| MGL-Fc | - | ++ | + | ++ | ++ | - |

| | | | | | | |
|---|---|---|---|---|---|---|
| MGL counterstructures were captured from PBL lysates and probed with specific anti-CD45 isoform-specific antibodies. Specificity was determined in the presence of EGTA (data not shown). -, no binding, +, low binding (OD 450 nm<0.25), ++, high binding (OD 450nm>0.25). | | | | | | |

**Supplementary table 1 MGL expression on cultured APC**

| APC subset | Isotype control (MFI) | MGL expression (MFI) |
|---|---|---|
| Mf | 10 | **81** |
| Alternatively activated Mf | 14 | **122** |
| (Dexamethasone) | | |
| Innate activated Mf | 21 | 20 |
| (LPS) Classically activated Mf | 71 | 85 |
| (LPS/IFN_{Y}) | | |
| Immature DC | 6 | **28** |
| Immature DC | 5 | **25** |
| (IL-10) Immature DC | 10 | **81** |
| (Dexamethasone) | | |
| Mature DC (LPS) | 7 | 9 |
| Mature DC (Poly I: C) | 3 | 5 |
| Mature DC (Flagellin) | 3 | 4 |

Different APC subsets were cultured from freshly isolated human monocytes in the presence of indicated cytokines. The following concentrations were used: for Mf culture: GM-CSF 1000 U/ml, dexamethasone 10⁻⁶ M, LPS 1 µg/ml, IFN_{Y} 1000 U/ml and for DC culture: GM-CSF 500 U/ml, IL-4 800 U/ml, dexamethasone 10⁻⁶ M, LPS 1 µg/ml, Poly I:C 10 µg/ml and Flagellin 5 µg/ml. MGL expression was determined by flowcytometry using the 18E4 antibody. One representative experiment out of two is shown.

**Supplementary table 2 Characterization of MGL^{pos} APC in skin and lymph node**

| Marker | Skin | Lymph node |
|---|---|---|
| CD1a | + | - |
| CD68 | +/- | +/- |
| CD163 | - | - |
| DC-SIGN | - | - |
| Mannose | + | + |
| receptor | | |
| CD11b | + | + |
| CD11c | + | + |
| CD3 | NT | - |

Cryosections of human skin and lymph node (7µ) were fixed with 100% aceton and stained with anti-MGL (10 ug/ml) and primary antibodies to the markers listed (5 µg/ml) for 1 hour at 37°C. Sections were counterstained with isotype-specific Alexa488- or Alexa594-labeled anti-mouse antibodies (Molecular probes). The following antibodies were used: anti-CD1a (OKT6), anti-CD68 (EMB11, Dako), anti-CD163 (Edhu1), anti-DC-SIGN (AZN-D1), anti-Mannose receptor (3.29.B1), anti-CD11b (Bear-1), anti-CD11c (SHCL-3) and anti-CD3 (T3b). Listed is the co-expression of markers on the MGL^{pos} cells: -, negative, +, positive, +/-, subpopulation of the MGL^{pos} cells co-express the marker, NT, not tested. One donor tissue out of two is shown.
MGL thus represents a marker for the skin APC described in Angel et al JI 2006.

**Supplementary table 3 MGL-Fc recognition of hematopoietic cell lines**

| Name | Cell type | MGL-Fc recognition | MGL counter receptors |
|---|---|---|---|
| K562 | Erythro-leukemic cell line | ++ | CD45 |
| HSB-2 | Acute lymphoblastic leukemia | ++ | CD43, CD45 |
| H9 | Cutaneous T cell lymphoma | + | CD45 |
| Jurkat E6.1 | T cell leukemia line | | CD43, CD45 |
| | | +++ | |
| Peer | Acute lymphoblastic leukemia | - | NA* |
| Raji | Burkitt's lymphoma | - | NA |
| MonoMac- | Monocyte-macrophage | | NA |
| 6 | cell line | - | |
| U937 | Histiocytic lymphoma | - | NA |

Cell lines were examined for MGL recognition by staining with MGL-Fc. Counterstructures were determined by MGL-Fc ligand ELISA. -, no binding, +, low MGL-Fc binding (MFI<100), ++, intermediate MGL-Fc binding (MFI 100-1000), +++, high MGL-Fc binding (MFI>1000), *NA, not applicable

**Supplementary table 4 Expression of co-stimulatory molecules on cultured APC**

| APC subset | Isotype (MFI) | CD80 (MFI) | CD83 (MFI) | CD86 (MFI) |
|---|---|---|---|---|
| Immature DC | 6 | 31 | 52 | 238 |
| Immature DC + | 25 | 29 | 26 | 81 |
| dex Mature DC | 6 | 395 | 188 | 786 |
| Macrophages | 6 | 29 | 9 | 160 |
| Macrophages + | 20 | 21 | 20 | 49 |
| dex | | | | |

APC subsets were cultured from freshly isolated human monocytes in the presence of GM-CSF 500 U/ml/IL-4 800 U/ml (DC) or GM-CSF 1000 U/ml (Macrophages). Dexamethasone was added at 10⁻⁶ M and LPS at 1 µg/ml. Expression of co-stimulatory molecules was determined by flowcytometry using the specific antibodies to CD80, CD86 (both from BD Biosciences) and CD83 (Beckman Coulter).

### EXAMPLE 3

### MATERIALS AND METHODS

### Antibodies and reagents

The following antibodies were used: anti-MGL (18E4 (IgG2a), 1G6.6 (IgG2a) [1] and MLD-1 (IgG1), kindly provided by dr. T. Irimura [2], anti-DC-SIGN (AZN-D1, IgG1) [3], anti-MHC II (Q5/13, IgG2a), anti-LAMP-1 (H4A3, IgG1, BD Pharmingen), EEA-1 (IgG1, Abcam, Cambridge, UK) and anti-ICAM-2 (12A2, IgG1 isotype control [4]). Biotinylated polyacrylamide (PAA)-coupled glycoconjugates were purchased from Lectinity (Lappeenranta, Finland).

### Cells

Immature monocyte derived DCs were cultured for 4-7 days from monocytes obtained from buffy coats of healthy donors (Sanquin, Amsterdam) in the presence of IL-4 (500 U/ml) and GM-CSF (800 U/ml). The phenotype of the cultured DC was confirmed by flowcytometric analysis. DC consistently expressed high levels of MHC class I and II and low levels of CD80 and CD86. CHO and CHO-MGL cells were maintained in RPMI 1640 medium (Invitrogen, Carlsbad, CA) containing 10% Fetal Calf Serum. Mutations in the cDNA encoding MGL were generated using the QuikChange site-directed mutagenesis kit (Stratagene, La Jolla, CA) and pRc/CMV-MGL plasmid according to the manufacturer's protocol. The cytoplasmic tail deletion mutant was generated by replacing the entire cytoplasmic region by a new protein initiation codon. Plasmid sequencing confirmed the introduction of the mutations in the MGL cytoplasmic region (Fig. 1). Stable CHO-MGL transfectants were generated using lipofectamin (Invitrogen) and selected by the addition of 2 mg/ml Geneticin (Invitrogen). MGL positive cells were sorted using the MoFlo (DAKOcytomation, Glostrup, Denmark).

### Flowcytometry, beads adhesion and internalization assays

Cells were incubated with primary antibody (5 µg/ml), followed by staining with a secondary FITC-labeled anti-mouse antibody (Zymed, San Francisco, CA) and analyzed on FACScalibur (BD Biosciences, San Diego, CA). Streptavidin coated fluorescent beads (488/645 nm, Molecular Probes, Eugene, OR) were incubated with 1 µg of the PAA-coupled GalNAc. Fluorescent bead adhesion assay was performed as previously described [5] and analyzed on FACScalibur and presented as the percentage of cells which have bound the fluorescent beads.
Immature DCs were incubated with antibodies (10 µg/ml) or biotinylated PAA-glycoconjugates (5 µg/ml) in TSA (20 mM Tris-HCl (pH 8.0), 150 mM NaCl, 1 mM CaCl2, 2 mM MgCl2, and 0.5% BSA) for 1 h on ice and subsequently washed. Cells were incubated at 37°C for various time points, placed on ice, and incubated with FITC-conjugated secondary antibodies or streptavidin-Alexa488 (Molecular Probes). To control for off rate of the antibodies or ligands, cells were fixed before antibody binding or kept on ice during the whole experiment to prevent membrane transport. Cells were analyzed using flowcytometry, and the relative differences in mean fluorescence intensities were compared.

### MGL-Fc adhesion assay.

PAA-coupled GalNAc was coated (5 µg/ml) on NUNC maxisorb plates (Roskilde, Denmark) overnight at room temperature. Plates were blocked with 1% BSA and MGL-Fc was added (0.5 µg/ml in TSA) for 2 hours at room temperature in the presence or absence of 10 mM EGTA. Experiments were performed at the indicated pH. Binding was detected using a peroxidase labeled anti-human IgG-Fc antibody (Jackson, West grove, PA).

### Confocal laser scanning microscopy

Anti-MGL 1G6.6 was labeled with Alexa Fluor 488 according to the manufacturer's protocol (Molecular Probes, Eugene, OR). Dendritic cells were incubated for 4 hours on ice, 2 hrs on ice followed by 2 hours at 37°C or 4 hours at 37°C with Alexa Fluor 488-conjugated anti-MGL (10 µg/ml). Labeled cells were fixed and permeabilized for 20 min at 4°C (BD cytofix/cytoperm™, BD). Cells were stained in phosphate-buffered saline containing 0.5 % bovine serum albumin and 0.1% saponin, with antibodies against LAMP-1 (lysosomes) or EEA-1 (early endosomes) and subsequently with Alexa Fluor 594-conjugated anti-mouse IgG1. Cells were allowed to adhere to poly-L-lysine-coated glass slides, mounted with anti-bleach reagent and analyzed by confocal microscopy. Leica AOBS SP2 confocal laser scanning microscope (CLSM) system was used, containing a DM-IRE2 microscope with glycerol objective lens (PL APO 63x/NA1.30) and images were acquired using Leica confocal software (version 2.61).

### Antigen presentation

HD7, a CD4+ T cell that recognizes a peptide derived from mouse IgG1 antibodies in HLA-DR0101/DQw1, was used [6]. Immature DCs (20.10³/well) from a typed donor were preincubated with serial dilutions of antibodies and cocultured with 80.10³ T cells. After 48 hours, IFN_{Y} production by the T cells was measured by ELISA according to the manufacturer's protocol (Biosource international,.Camarillo, CA).

### RESULTS

### MGL mediates rapid internalization of ligands

The presence of two putative internalization motifs (YENF and LL, Fig. 1) in the cytoplasmic domain of human MGL prompted us to investigate whether MGL expressed on DCs functions in antigen internalization and presentation.

The C-type lectin DC-SIGN, known to rapidly internalize after ligand binding [4], was used as a positive control for endocytic uptake by DCs. Human monocyte-derived DCs expressed moderate levels of MGL and high levels of DC-SIGN (Fig. 2A). Mature DCs do not express the MGL molecule (data not shown). To investigate MGL-mediated internalization, we employed antibodies directed against the MGL carbohydrate recognition domain that mimic ligand binding and induce receptor crosslinking essential for endocytosis. Antibodies to MGL were internalized from the DC surface similar to anti-DC-SIGN antibodies (Fig. 2B). The observed entry was not the result of an enhanced antibody off-rate at 37°C, since no decrease in antibody staining was seen on fixed DCs. Furthermore, no internalization was observed if cells were kept on ice during the whole experiment. Internalization was not caused by the normal turnover of molecules, as antibodies to MHC II were not internalized within the 60-minute time frame of the experiment. Next, we determined whether soluble carbohydrate ligands are endocytosed by MGL as well. The MGL ligand PAA-GalNAc [7] and the DC-SIGN ligands PAA-Lewis X and PAA-Mannose [8] were internalized with faster kinetics from the cell surface compared to antibodies (Fig. 2C). These data demonstrate that, in addition to DC-SIGN, antigens bound by MGL are rapidly endocytosed from the DC surface.

### The YENF motif is essential for MGL-mediated endocytosis.

To identify the motifs involved in the MGL-mediated internalization process, we generated MGL cytoplasmic domain mutants by site-directed mutagenesis and a complete cytoplasmic deletion mutant (Fig. 1). Stable cell lines were generated by transfecting the constructs encoding wildtype and mutant MGL into CHO cells. MGL mutants were expressed at the cell surface at comparable levels to the wildtype MGL (Fig. 3A).
Furthermore, mutating or deleting the cytoplasmic tail did not affect ligand recognition, as all MGL-transfectants displayed equal binding to GalNAc-coated beads (Fig. 3B), demonstrating that the cytoplasmic region is not involved in ligand binding. MGL binding could be inhibited by addition of the Ca²⁺-chelator EGTA, confirming the specificity of this interaction.
Antibodies to MGL are internalized with similar kinetics on immature DCs and CHO-MGL (compare fig. 1B and fig. 3C). As expected, deletion of the entire MGL cytoplasmic tail (MGL ΔCYT) abrogated MGL-mediated endocytosis (Fig. 3C). Disruption of the YENF motif (MGL Y5) completely blocked internalization, whereas mutating the dileucine motif (MGL LL) reduces internalization by 45%. Thus, the YENF motif directly facilitates and is therefore essential to MGL-mediated internalization.

### MGL captures antigens for processing and presentation

During transit through the endosomal/lysosomal pathway lectins and their cargo are exposed to a lowering pH gradient. An important property of recycling C-type lectin receptors, such as the MR, is their ability to dissociate from their cargo at low pH, enabling recycling of the receptor to the cell surface. We analyzed MGL binding to its ligand GalNAc at different pH to investigate at which pH lectins dissociate form their antigens. The pH used mimics the pH of the different intracellular compartments. The MGL-Fc-GalNAc interaction was disrupted at pH values <6.0, indicating that MGL can dissociate from its ligand in the late endosomal or lysosomal compartments and potentially recycle back to the cell surface (Fig. 4A). Lectins that continuously recycle, such as the MR, often possess a large intracellular pool [9]. Also the MGL protein is mainly located in intracellular vesicles (data not shown) [10]. However, so far we have not been able to demonstrate any MGL recycling using previously described methods to assess receptor-mediated recycling (data not shown) [11, 12]. Whereas MGL lost all ligand binding at pH 5.5 (Fig. 4A), related C-type lectins DC-SIGN and MR still display some residual binding at pH 5.5 and only completely dissociate from their ligands at pH 5 [4, 13].

To investigate whether MGL endocytosis delivers antigens to MHC molecules for presentation to T cells, we employed a CD4+ T cell clone that recognizes a peptide derived for mouse IgG1. First, we determined the fate of internalized MGL antibodies by confocal scanning laser microscopy. After 2 to 4 hours of internalization a clear co-localization could be observed between anti-MGL and early endosomes and lysosomes (Fig. 4B), indicating that antigens endocytosed by MGL are targeted to lysosomes for degradation. No colocalization could be detected if cells were kept on ice to prevent MGL-mediated endocytosis (Fig. 4B). Next, immature DC from a donor with a compatible haplotype were preincubated with serial dilutions of MGL antibodies and their capacity to activate T cells was investigated. Incubation of DC with an IgG1-antibody to MGL resulted in specific IFN_{Y} secretion by responder T cells at concentrations above 3 ng/ml, demonstrating that ligands that are endocytosed by MGL, can be presented in the context of MHC class II molecules (Fig. 4C). More efficient antigen presentation was seen after antibody targeting to DC-SIGN compared to MGL, which might reflect antibody affinity or more likely, cell surface expression levels, which are a proportional 10-fold higher for DC-SIGN (Fig. 2A). Isotype control antibodies to DC-expressed ICAM-2 did not induce specific IFN_{Y} secretion. Thus, we conclude that MGL functions as an efficient antigen receptor on DC.

### DISCUSSION

Here we demonstrate that the human C-type lectin MGL functions as a genuine antigen uptake receptor on DC. Similar to DC-SIGN, MR and Dectin-1, MGL is capable of targeting soluble ligands for degradation and presentation on MHC class II molecules, resulting in activation of responder T cells [4, 14, 15]. It will be interesting to pursue whether endocytosis via MGL leads to crosspresentation of antigens in MHC class I as well.

By mutational analysis, we have shown that the YENF motif in the MGL cytoplasmic tail is crucial for this process. Disruption of the tyrosine-5 residue in this YXXØ consensus motif completely abrogated MGL-mediated endocytosis in CHO cells, whereas mutating the dileucine motif that only partially matches the consensus motif, only slightly reduced internalization. It is likely that this YENF motif is also functionally relevant in DC. The YXXØ motif facilitates chlathrin-mediated endocytosis, through the direct interaction of the adaptor protein AP-2 [16, 17]. Subsequently, clathrin is recruited and endocytosis is triggered. Clathrin-coated vesicles are uncoated after entry and then fuse with the early endosomes [18]. The YXXØ motif has also been implicated in lysosomal targeting, whereby acidic amino acids at the X positions favour lysosomal sorting [16]. The presence of an acidic glutamic acid (E) at position two of the MGL YENF motif indicates that MGL might direct proteins for lysosomal destruction. However, compared to DC-SIGN that dissociates in the late endosomal/lysosomal compartments [4], MGL releases its cargo at higher pH, thus at an earlier stage in the endocytic pathway, likely in early/late endosomes. Therefore, it seems unlikely that MGL travels together with its antigen to the lysosomes. Since the internalization route is also partly dependent on the antigen [19], ligand-induced transfer to the lysosomes cannot be excluded, as we observed clear targeting of MGL-endocytosed cargo to the lysosomes. A similar YXXF motif is present in the cytoplasmic tail of the human transferring receptor [20], which continuously recycles between the plasma membrane and the endosomal compartments. Since MGL mostly resides intracellular [10] and already dissociates from its ligands at pH<6, it might similarly recycle to the cell surface. However, we have not been able to demonstrate any steady state or ligand-induced recycling of MGL (data not shown). Although we observed no major effect of disruption of the dileucine motif on MGL-mediated internalization, we cannot rule out that this motif is involved in the regulation of subsequent intracellular cargo transport.

The tumor-associated Tn-antigen (α-GalNAc-Ser/Thr), a high affinity ligand for MGL, is highly expressed by a variety of adenocarcinomas. Generally, Tn-expression positively correlates with tumor aggressiveness and early death of patients [21]. In vitro and in vivo studies have shown that Tn-antigens can be presented in the context of MHC class I, resulting in the generation of functionally competent effector T cells [22, 23]. Moreover, anti-Tn antibodies are raised in carcinoma patients, proving that Tn-epitopes elicit humoral responses and it is therefore an interesting tumor vaccine candidate. The efficacy of such a vaccine is currently being tested in several clinical trials [24, 25]. Our data demonstrates that MGL functions as an efficient antigen receptor on DC, capable of delivering soluble antigens for processing and presentation to T cells. The high affinity binding of MGL to Tn-epitopes on tumor antigens suggests that in vivo MGL may actively participate in stimulating anti-tumor immune responses. This hypothesis is supported by the data obtained in a murine model of ovarian cancer. In this model mMGL+ cells specifically home to the metastatic tumor site and injection of blocking mMGL-antibodies increases metastatic tumor loads, suggesting that MGL positively contributes to anti-tumor immunity [26, 27]. However, MGL levels are increased on human DC with a tolerogenic phenotype [1], indicating that presentation of tumor antigens by these subsets might result in the induction of regulatory T cells or T cell unresponsiveness, thereby promoting tumor growth instead of preventing growth.
In conclusion, the human DC-expressed C-type lectin MGL internalizes antigens for presentation to T cells. Future studies are needed to address whether MGL would be useful as a target in tumor-associated glycan-based immunotherapy.

### References to example 3

1. van Vliet,S.J., van Liempt,E., Geijtenbeek,T.B., & van Kooyk,Y. Differential regulation of C-type lectin expression on tolerogenic dendritic cell subsets. Immunobiology 211, 577-585 (2006).
2. Higashi,N. et al. The macrophage C-type lectin specific for galactose/N-acetylgalactosamine is an endocytic receptor expressed on monocyte-derived immature dendritic cells. J. Biol. Chem. 277, 20686-20693 (2002).
3. Geijtenbeek,T.B.H. et al. Identification of DC-SIGN, a novel dendritic cell-specific ICAM-3 receptor that supports primary immune responses. Cell 100, 575-85 (2000).
4. Engering,A. et al. The dendritic cell-specific adhesion receptor DC-SIGN internalizes antigen for presentation to T cells. J Immunol 168, 2118-26 (2002).
5. Geijtenbeek,T.B.H. et al. High frequency of adhesion defects in B-lineage acute lymphoblastic leukemia. Blood 94, 754-64 (1999).
6. Lanzavecchia,A. et al. Antibodies as antigens. The use of mouse monoclonal antibodies to focus human T cells against selected targets. J. Exp. Med. 167, 345-352 (1988).
7. van Vliet,S.J. et al. Carbohydrate profiling reveals a distinctive role for the C-type lectin MGL in the recognition of helminth parasites and tumor antigens by dendritic cells. Int. Immunol. 17, 661-669 (2005).
8. Appelmelk,B.J. et al. Cutting edge: carbohydrate profiling identifies new pathogens that interact with dendritic cell-specific ICAM-3-grabbing nonintegrin on dendritic cells. J. Immunol. 170, 1635-1639 (2003).
9. Mahnke,K. et al. The dendritic cell receptor for endocytosis, DEC-205, can recycle and enhance antigen presentation via major histocompatibility complex class II-positive lysosomal compartments. J Cell Biol 151, 673-84 (2000).
10. Valladeau,J. et al. Immature human dendritic cells express asialoglycoprotein receptor isoforms for efficient receptor-mediated endocytosis. J Immunol 167, 5767-74 (2001).
11. Engering,A.J. et al. The mannose receptor functions as a high capacity and broad specificity antigen receptor in human dendritic cells. Eur J Immunol 27, 2417-25 (1997).
12. Mitchell,H., Choudhury,A., Pagano,R.E., & Leof,E.B. Ligand-dependent and -independent transforming growth factor-beta receptor recycling regulated by clathrin-mediated endocytosis and Rab11. Mol. Biol. Cell 15, 4166-4178 (2004).
13. Lennartz,M.R., Wileman,T.E., & Stahl,P.D. Isolation and characterization of a mannose-specific endocytosis receptor from rabbit alveolar macrophages. Biochem. J. 245, 705-711 (1987).
14. Carter,R.W., Thompson,C., Reid,D.M., Wong,S.Y., & Tough,D.F. Preferential Induction of CD4+ T Cell Responses through In Vivo Targeting of Antigen to Dendritic Cell-Associated C-Type Lectin-1. J. Immunol. 177, 2276-2284 (2006).
15. Engering,A.J. et al. Mannose receptor mediated antigen uptake and presentation in human dendritic cells. Adv. Exp. Med. Biol. 417, 183-187 (1997).
16. Bonifacino,J.S. & Traub,L.M. Signals for sorting of transmembrane proteins to endosomes and lysosomes. Annu. Rev. Biochem. 72, 395-447 (2003).
17. Sorkin,A. Cargo recognition during clathrin-mediated endocytosis: a team effort. Curr. Opin. Cell Biol. 16, 392-399 (2004).
18. Le Roy,C. & Wrana,J.L. Clathrin- and non-clathrin-mediated endocytic regulation of cell signalling. Nat. Rev. Mol. Cell Biol. 6, 112-126 (2005).
19. Herre,J. et al. Dectin-1 uses novel mechanisms for yeast phagocytosis in macrophages. Blood 104, 4038-4045 (2004).
20. Daniels,T.R., Delgado,T., Rodriguez,J.A., Helguera,G., & Penichet,M.L. The transferrin receptor part I: Biology and targeting with cytotoxic antibodies for the treatment of cancer. Clin. Immunol.(2006).
21. Springer,G.F., Desai,P.R., Ghazizadeh,M., & Tegtmeyer,H. T/Tn pancarcinoma autoantigens: fundamental, diagnostic, and prognostic aspects. Cancer Detect. Prev. 19, 173-182 (1995).
22. Xu,Y., Gendler,S.J., & Franco,A. Designer Glycopeptides for Cytotoxic T Cell-based Elimination of Carcinomas. J. Exp. Med. 199, 707-716 (2004).
23. Xu,Y., Sette,A., Sidney,J., Gendler,S.J., & Franco,A. Tumor-associated carbohydrate antigens: a possible avenue for cancer prevention. Immunol. Cell Biol. 83, 440-448 (2005).
24. Springer,G.F. et al. T/Tn antigen vaccine is effective and safe in preventing recurrence of advanced breast carcinoma. Cancer Detect. Prev. 19, 374-380 (1995).
25. Slovin,S.F. et al. Fully synthetic carbohydrate-based vaccines in biochemically relapsed prostate cancer: clinical trial results with alpha-N-acetylgalactosamine-O-serine/threonine conjugate vaccine. J. Clin. Oncol. 21, 4292-4298 (2003).
26. Ichii,S., Imai,Y., & Irimura,T. Tumor site-selective localization of an adoptively transferred T cell line expressing a macrophage lectin. J. Leukoc. Biol. 62, 761-770 (1997).
27. Ichii,S., Imai,Y., & Irimura,T. Initial steps in lymph node metastasis formation in an experimental system: possible involvement of recognition by macrophage C-type lectins. Cancer Immunol. Immunother. 49, 1-9 (2000).

### EXAMPLE 4

### MATERIALS AND METHODS

### Cells and reagents

Immature monocyte-derived DCs were cultured for 4-7 days from monocytes obtained from buffy coats of healthy donors (Sanquin, Amsterdam) in the presence of IL-4 and GM-CSF (500 U/ml and 800 U/ml respectively, Biosource, Camarillo, CA). HMEC-1 cells were cultured in MCDB 131 medium (Invitrogen, Carlsbad, CA) supplemented with 10% FCS, 10 ng/ml epidermal growth factor and 1 µg/ml hydrocortisone. Human umbilical vein endothelial cells (HUVEC) were isolated as previously described and cultured in M199 medium (Cambrex, East Ruhterford, NJ) supplemented with 10% human serum, 10% newborn calf serum, 5 U/ml heparin and 5 ng/ml basic fibroblast growth factor. Polyacrylamide (PAA)-coupled glycoconjugates were purchased from Lectinity (Lappeenranta, Finland). Biotinylated Helix pomatia agglutinin (HPA) was purchased form Sigma Aldrich (St. Louis, Mo). Biotinylated Maackia amurensis agglutinin (MAA), Sambucus nigra agglutinin (SNA) and Ulex europaeus agglutinin-1 (UEA-1) were purchased from Vector Laboratories (Burlingame, CA). An MGL-murine Fc fusion protein was generated by cloning the extracellular part of MGL into an pcDNA3 expression vector containing exon 1-3 of murine IgG2a-Fc. MGL-mFc was produced by transient transfection in CHO cells and MGL-mFc concentrations were determined by ELISA.

### ELISA-based MGL-Fc binding assays

PAA-glycoconjugates were coated at 5 µg/ml on NUNC maxisorb plates (Roskilde, Denmark) overnight at room temperature. Plates were blocked with 1% BSA and MGL-mFc was added (0.5 µg/ml) for 2 hours at room temperature in the presence or absence of 10 mM EGTA. Binding was detected using a peroxidase-labeled anti-mouse IgG Fc antibody (Jackson, West grove, PA).

### Immunohistochemistry

Cryosections of healthy tissues (7 µm) were fixed with 2% paraformaldehyde. MGL-mFc (25 µg/ml) or anti-MGL (18E4, 10 µg/ml) were added in TSM buffer (20 mM Tris-HCl, pH 7.4, 150 mM NaCl, 2 mM CaCl₂, 2 mM MgCl₂) and incubated for 2 hours at 37°C. Binding was detected with an Alexa 594-conjugated goat anti-mouse IgG2A-specific antibody (Molecular Probes, Eugene, OR). To visualize the presence of α-GalNAc epitopes sections were incubated with HPA (5 µg/ml) for 2 hours at 37°C. HPA binding was detected using an Alexa 488-conjugated streptavidin (Molecular Probes). Where indicated, sections were co-stained using primary mouse antibodies to LYVE-1 (lymphatic/sinusoidal endothelium) for 1 hour at room temperature, followed by a secondary Alexa 488- or Alexa 594-conjugated goat anti-mouse IgG1-specific antibody (Molecular Probes). Sections were counterstained using Hoechst.

### Flow cytometry and MGL-Fc binding

MGL protein expression was determined by incubating sells with primary antibody (5 µg/ml), followed by staining with a secondary FITC-labeled goat anti-mouse antibody (Zymed, San Francisco, CA) and analyzed on FACScalibur (BD Biosciences, San Diego, CA). To assess the expression of carbohydrate epitopes on the cell surface, cells were incubated with 10 µg/ml of the biotinylated lectins in TSM supplemented with 0.5% BSA for 30 min at 37°C, followed by staining with a Alexa 488-conjugated streptavidin (Molecular Probes) and analyzed on FACScalibur. To analyze MGL ligand expression, cells were incubated with MGL-Fc (10 µg/ml) in TSM supplemented with 0.5% BSA for 30 min at 37°C, followed by staining with a secondary FITC-labeled anti-human IgG Fc antibody (Jackson, West grove, PA) and analyzed on FACScalibur. In blocking experiments, MGL-Fc was preincubated for 15 minutes at room temperature with 10 mM EGTA, 100 mM free GalNAc (Sigma Aldrich, St. Louis, MO), 20 µg/ml of the lectins or 20 ug/ml anti-MGL antibodies.

### Migration assays

Transwell 24-well plates (8 µm pore, Greiner Bio-one, Frickenhausen, Germany) were coated with 1% gelatine for 1 hour at 37°C. HMEC-1 cells (70.000) were seeded on the inserts and after 24 hours 200.000 DCs were added to the monolayer of endothelial HMEC-1 cells. The lower chamber contained human RANTES (100 ng/ml, Biosource). After 2 hours at 37°C, the number of transmigrated DCs (lower chamber) was determined by flow cytometry. Transendothelial migration was measured in the presence or absence of blocking or isotype-matched antibodies (20 µg/ml). DC migration on coated PAA-glycoconjugates (5 µg/ml) was studied using time-lapse video microscopy. 45.000 DCs were added to the plates, allowed to settle for 30 minutes and the number of migrating DC was assessed for a 30-minute period. The migration assay was conducted in the absence and presence blocking antibodies (20 µg/ml). Migration was scored as the percentage of cells displaying spatial movement on the coating, accompanied with changes in cell shape.

### RESULTS

### MGL ligands can be detected in healthy human lymph node and thymus

A recombinant protein consisting of the extracellular domain of MGL fused to the mouse IgG2a Fc tail was generated. Example 4 figure 1 shows MGL-mFc displays an identical carbohydrate recognition profile of terminal GalNAc residues as MGL expressed by human immature DCs.

Next, cryosections of healthy human tissues were labeled with MGL-mFc to investigate the expression of potential MGL ligands in situ. In LN and thymus MGL ligands could be identified on the lymphatic endothelium (Example 4, fig. 2).

The distribution of MGL was analyzed in comparison to the localization of the lymph endothelial cells. In LN MGL+ APCs are located just below the lymph node sinusoidal endothelium (Example 4, fig. 3). In thymus MGL+ APCs are found in the interlobular space, where also the lymphatic vessels are situated.

### MGL specifically interacts with endothelial cells in an α-GalNAc-dependent manner

Human umbilical vein endothelial cells (HUVECs), a model system for endothelial cells lining blood vessels, were not bound by MGL-Fc (Example 4, fig. 4A). In contrast, HMEC-1 cells, a human endothelial cell line that expresses several lymphatic endothelial markers, strongly interacted with MGL-Fc (Example 4, fig. 4B). MGL binding could be blocked by the addition of the Ca²⁺-chelator EGTA, free GalNAc monosaccharides or anti-MGL antibodies, demonstrating the specificity of this interaction.
To explore the nature of the MGL ligand on these cells, we investigated by flow cytometry, using well-characterized plant/invertebrate lectins, which glycan epitopes are present on HMEC-1 cells. Previous studies have demonstrated a strong correlation between MGL binding and the expression of glycan epitopes recognized by the α-GalNAc-specific roman snail lectin Helix pomatia agglutinin (HPA). Indeed, HMEC-1 expressed HPA-reactive glycan structures (Example 4, fig. 4C). HPA binding could be blocked by the addition of free GalNAc, indicating that HPA recognized α- GalNAc containing glycans on the HMEC-1 cells. In addition α1-2 linked fucose, α2-3 and α2-6 linked sialic acid structures are present on HMEC-1 cells, as visualized by the reactivity of Ulex europaeus agglutinin-1 (UEA-1), Sambucus nigra agglutinin (SNA) and Maackia amurensis agglutinin (MAA) respectively (Example 4, fig. 4C).

However, when these lectins were used to block MGL-Fc binding, only HPA could significantly interfere with the MGL-HMEC-1 interaction, demonstrating that an α-GalNAc-containing glycoprotein or -lipid constitutes as ligands for MGL on HMEC-1 cells (Fig. 4D).
Strikingly, although HPA stained additional vessels in LN and thymus, it displayed a complete overlap with the sinusoidal and lymphatic endothelium (Example 4, fig. 5A and B). These subpopulations of endothelial cells were also recognized by MGL (Example 4, Fig. 2A and B). The GalNAc block of MGL binding to tissues (Fig. 2) combined with the specific HPA staining (Fig. 5) demonstrate that MGL interacts with endothelial cells in LN or thymus and the connective tissue in skin in an α-GalNAc-dependent manner.

### MGL mediates retention of immature dendritic cells

Immature DCs expressed moderate levels of MGL (Example4, fig. 6A). Next, we measured the capacity of these DCs to transmigrate across an HMEC-1 monolayer. In response to the chemokine RANTES, transmigration of immature DCs was enhanced about 3-fold and largely dependent on the β2-integrin, as shown by the block using anti- β2 antibodies. Strikingly, anti-MGL significantly increased transmigration compared to isotype control antibodies (Example 4, fig. 6B).
We followed DC mobility on glycoconjugate-coated plates with time-lapse videomicroscopy. Migration was scored as the percentage of cells displaying spatial movement on the coating, accompanied with changes in cell shape. DCs did not interact with the glucitol or galactose coating (Example 4, fig. 6C). In contrast, DCs firmly adhered to Lewis X and Man3, which inhibited DC mobility. Surprisingly, on GalNAc, DCs displayed a strong migratory movement, while continuously interacting with the coated surface (Example 4, fig. 6C). Similar to the transmigration experiments, anti-MGL antibodies significantly increased the percentage of migrating DCs on the GalNAc-coating, as well as the speed by which the DCs moved (Example 4, fig. 6D and data not shown). The increased migration was not due to a nonspecific effect on the immature DCs, as isotype control antibodies did not induce enhanced mobility. Furthermore, the anti-MGL antibodies did not have any enhancing effect on DC migration on Lewis X (Example 4, fig. 6D). DCs incubated with anti-MGL antibodies still interacted with the GalNAc-coated surface, suggesting that immature DCs express another GalNAc receptor in addition to MGL.
Thus, expression of MGL on immature DCs and the presence of GalNAc sugars hamper DC migration and favors DC retention in the tissue. Blocking of MGL function or downregulation of expression, due to DC maturation, can relieve DCs from the GalNAc constraints and enhance the migratory capacities of DCs.

### EXAMPLE 5

Different methods have been described in the literature on the generation of neoglycoconjugates (proteins which are chemically modified with carbohydrates structures). Many of these procedures are difficult to carry out, are time consuming and result in less stable reactive carbohydrate derivatives [Wong et al. Biochem. J. (1993) 296, 817-825]. Some of them are even toxic or result in degradation of carbohydrate structure by oxidation steps in order to chemically activate the carbohydrate [D. Mislovicova et. al. Bioconjugate Chem. 2002, 13, 136-1422].

### MATERIALS AND METHODS

Coupling of carbohydrates to ovalbumin was achieved via a 2 step coupling reaction with the use of a bifunctional crosslinker (4-N-Maleimidophenyl)butyric acid hydrazide; Pierce). This crosslinker contains a hydrazide moiety and a maleimide moiety. The first step is coupling of carbohydrate structure to the bifunctional crosslinker. Via a one pot reductive amination reaction the hydrazide moeity of the crosslinker is covalently coupled to the reducing terminus of the carbohydrate since the carbohydrate has a free reducing terminus which is in equilibrium between the ring closed (cyclic) and ring open (acylic) forms. The hydrazide group of the crosslinker performs a nucleophilic attack on the carbonyl carbon of the acyclic reducing terminal residue to form a partially stable Schiff's base. The Schiff's base imine group is subsequently chemically reduced with a mild reductant to give a stable carbohydrate-MPBH derivate (see Example 5, Figure 1).

### Preparation of neo-glycoconjugates

After purification of activatide carbohydrate the second coupling is performed. The Maleimide moiety of the carbohydrate-MPBH derivative reacts at neutral pH with the side chain of cysteines (thiols) and forms a covelant bond. Gel filtration is performed to remove the access carbohydrate-MPBH derivative. This two step coupling procedure results in pure ovalbumin which contains carbohydrate structures of interests.

After the first coupling and purification step the activated carbohydrate structure can be stored indefenitely at minus -20 (purified carbohydrate derivative results in a dry powder). This activated carbohydrate can be coupled to any protein of interest even if the protein doesn't contain any cysteines on its surface. By changing the pH of the second coupling step, the covalent linkage between primary amines (lysines) can also be achieved.

Not directly coupling of the carbohydrate to protein, but rather producing a stable reactive carbohydrate intermediate has two big advantages over the prior one. Stable activated carbohydrate is ready-to-use and has an universal applicability, namely it can be used to glycomize any protein of interest.

### EXAMPLE 6

### MATERIALS AND METHODS

### Cells

Chinese hamster ovary cells (CHO) and CHO-MGL transfectants were cultured in RPMI containing 10% fetal bovine serum (FBS) and streptomycin/penicillin.

### Mice

C57BL/6 mice were purchased from Charles River Laboratories and used 8-12 weeks of age. OT-I mice and OT-II mice bred in our animal facility under pathogen free conditions, express a transgenic Vα2 Vβ5.1/5.2 T cell receptor (TCR) specific for the OVA peptides presented on H2-Kb (amino acids 257-264; SIINFEKL) or on I-Ab (amino acids 323-339; ISQAVHAAHAEINEAGR), respectively.

### Cell culture of Bone marrow derived DCs (BM-DC)

The femeur and tibia of mice were removed, both ends were cut and marrow was flushed with IMDM medium using a syringe with 0.45mm diameter needle. The bone marrow suspension was vigorously resuspended and passed over a 100µm gauge to obtain single cell suspension. After washing cells were seeded per 2x10⁶cells per 100mm petridish (Greiner Bio-One, Alphen aan de Rijn, The Netherlands) in 10ml Iscove's modified dulbecco's medium (IMDM, Gibco) supplemented with 2 mM L-glutamine, 50 U/ml penicillin, 50 ug/ml streptomycin and 50 µM β-mercaptoethenol (Merck, Damstadt, Germany) and containing 20 ng/ml recombinant murine GM-CSF (rm GM-CSF). At day 2, another 10 ml medium containing 20 ng/ml rm GM-CSF was added. At day 5 another 10 ng/ml rmGM-CSF was added to each plate. From day 6 onwards, the non-adherent BM-DC were harvested and used for subsequent experiments.

### Antigen specific T cell isolation

OVA specific CD4⁺ and CD8⁺ T cells were prepared from spleen and lymph nodes cell suspensions from OT-II and OT-I mice respectively. Lymph nodes and spleen were crushed and cell suspensions were pelleted. RBCs were lysed in spleen cell suspensions and the cells were passed through cell strainer. CD4 and CD8 T cells were isolated from the suspensions using Dynal mouse CD4/CD8 negative isolation kit according to the manufacturer's protocol.

### ELISA-based MGL binding assays

PAA-glycoconjugates were coated at 5 µg/ml on NUNC maxisorb plates (Roskilde, Denmark) overnight at room temperature. Plates were blocked with 1% BSA and mMGL1-FC, mMGL2-Fc or human MGL-Fc was added (0.5 µg/ml) for 2 hours at room temperature in the presence or absence of 10 mM EGTA. Binding was detected using a peroxidase-labeled anti-mouse IgG Fc antibody (Jackson, West grove, PA).

### Glycan array (Consortium for Functional Glycomics.

Biotinylated synthetic or natural glycan structures were coated at saturating densities to streptavidin coated high binding capacity black plates (Pierce, Rockford, IL) and probed with mMGL1-Fc and mMGL2-Fc (2.5 µg/ml). Bound MGL-Fc was detected using a FITC-labeled anti-human IgG-Fc antibody. Plates were read at 485-535 nm on a Wallac Victor² 1420 multi-label counter (Perkin Elmer, Wellesley, MA). Standard procedures for glycan array testing are available at (www.functionalglycomics.org).

### Cellular adhesion assays

Cells were incubated with biotinylated PAA coupled glycoconjugates (10 µg/ml; Lectinity) for 30 minutes at 37° C and afterwards stained with streptavidine-conjugated Alexa488 (Molecular Probes) for detection. Binding of glycoconjugates was analysed on FACSCalibur and presented as the percentage of cells that have bound the glycoconjugate.

### T cell proliferation assay

Irradiated BM-DC were pulsed with neo-glycoconjugates (see example 5) in round bottom 96-wells plate and washed three times. Purified T cells either CD4+ Tcells from OT-II mice or CD8+ve T cells from OT-I mice were added to each well and co-cultured with antigen-pulsed BM-DC for 48 hours. After 48 h, [³H]thymidine (1 µCi; Amersham Biosciences) was added for 16 h to detect incorporation into DNA of proliferating T cells. Cells were harvested onto filters and [³H]thymidine incorporation was assessed using beta counter.

### RESULTS

### Carbohydrate and ligand specificity of mouse MGL1 and MGL2

To validate the results obtained for the human MGL homologue and to set up in vivo models for MGL, the mouse MGL homologues mMGL1 and mMGL2 were cloned. Also Fc-chimeric proteins of these homologues were generated. Next the carbohydrate recognition profiles of mMGL1 and mMGL2 were determined.First, mMGL1-Fc and mMGL2-Fc were tested on coated PAA-glycoconjugates in an ELISA based assay. In this assay, mMGL1 and mMGL2 have distinct carbohydrate recognition profiles. Whereas mMGL2-Fc recognized both α-GalNAc and TF antigen, mMGL1-Fc displayed an exclusive specificity for Lewis X structures. Human MGL-Fc was taken as a control and recognized only α-GalNAc (Example 6, figure 1).However, when cellularly expressed mMGL1 was tested in a cellular adhesion assay using CHO-mMGL1 transfectants, mMGL1 also recognized sialyl Lewis X and some structures that contain terminal Galactose or GalNAc sugars (Example 6, figure 2).
When the PAA-glycoconjugates were titrated down, mMGL1 binding to sialylated or galactose/GalNAc-containing structures was lost, only Lewis X is still bound by mMGL-1 at the lowest concentration tested, demonstrating that Lewis X is indeed the high affinity ligand for mMGL1 (Example 6, figure 3).
To further dissect the mMGL carbohydrate recogniton profile both mMGL1 and mMGL2 were tested on the glycan array developed by the consortium for functional glycomics (Example 6, figure 4A). Again mMGL1 and mMGL2 have distinct carbohydrate recognition profiles. Whereas mMGL2-Fc recognized strucutres containing both terminal GalNAc and galactose, mMGL1-Fc displayed an exclusive specificity for Lewis X structures. Mouse bonemarrow-derived dendritic cells (BM-DC) express both mMGL1 and mMGL2. Therefore the binding of glycoconjugates to BM-DC was investigated. BM-DC bind Lewis X and terminal GalNAc sugars, via mMGL1 and mMGL2 respectively (Example 6, figure 4B).
We investigated whether directing antigens to MGL facilitates enhanced antigen presentation to T cells. OVA-neoglycoconjugates (see example 5) containing GalNAc or Lewis X were incubated with BM-DC, washed and subsequently co-cultured with OVA-specific CD4+ and CD8+ T cells, OT-I and OT-II respectively. OVA-GalNAc as wells as OVA-Lewis X induced more substantial proliferation of the responder CD4+ and CD8+ T cells than native OVA. OVA-GalNAc was superieur in these assays compared to OVA-Lewis X. Our results show that modification of model antigens with GalNAc or Lewis X carbohydrates leads to enhanced binding to mouse MGL molecules and superior antigen presentation to specific T cells. These data demonstrate that MGL is be a suitable target in immunization and vaccination protocols.

### EXAMPLE 7

### MATERIALS AND METHODS

### Cells and cell lines.

All cell lines were maintained in RPMI containing 10% fetal calf's serum (Invitrogen, Carlsbad, CA). Immature DC were cultured for 4-7 days from monocytes obtained from buffy coats of healthy donors (Sanquin, Amsterdam) in the presence of IL-4 (500 U/ml) and GM-CSF (800 U/ml). DC were matured by addition of 1 µg/ml LPS (Salmonella typhosa, Sigma-Aldrich, St. Louis, MO). Macrophages were generated from monocytes by culturing in the presence of 1000 U/ml GM-CSF. Dexamethasone was added at 1 µM.

### ELISA-based MGL binding assays

Paraformaldehyde fixed Neisseria gonorrhoeae were coated at 2.5 10⁶/well on NUNC maxisorb plates (Roskilde, Denmark) overnight at room temperature. Plates were blocked with 1% BSA and human MGL-Fc was added (0.5 µg/ml) for 2 hours at room temperature in the presence or absence of 10 mM EGTA, blocking anti-MGL antibodies (1G6.6, 20 µg/ml) or free GalNAc monosaccharides (100 mM). Binding was detected using a peroxidase-labeled anti-mouse IgG Fc antibody (Jackson, West grove, PA).

### Cellular binding assays

Cells were incubated with FITC-labeled paraformaldehyde-fixed Neisseria gonorrhoeae at an MOI of 50 or 100 for 45 minutes at 37° C. Binding of the bacteria was analyzed by flow cytometry and presented as the percentage of cells that have bound bacteria.

### DC internalization assay

DC were incubated with FITC-labeled bacteria at DC:bacteria ratio of 1:100 in RPMI containing 10% fetal calf's serum for 15 min to 4 h. DC were washed and analyzed twice by flowcytometry in the presence or absence of 0,4% Tryphan blue, detecting intracellalur and both intracellular and extracellularly bound bacteria, respectively.

### DC and macrophage maturation

Immature monocyte derived DC or macrophages (day 4) were stimulated with paraformaldehyde fixed N. gonorrhoeae at indicated MOI for 20 hrs at 37°C. Cell-surface expression of CD14 (Macrophages only), CD80, CD83 (DC only), and CD86 using PE-conjugated antibodies was used to determine maturation.

### Cytokine measurements

For the detection of cytokines, culture supernatants were harvested 20 hrs after DC or macrophage activation and frozen at -80°C until analysis. Cytokines were measured by enzyme-linked immunosorbant assay (ELISA) with CytoSets^{™} ELISA kits (Biosource) according to the manufacturer's instructions.

### mRNA isolation and cDNA synthesis

mRNA was isolated by poly (A+) RNA capture in streptavidin-coated tubes with an mRNA Capture kit (Roche, Switzerland) and cDNA was synthesized with the Reverse Transcription System kit (Promega, USA) following manufacturer's guidelines. In brief, cells were washed twice with ice-cold PBS and harvested with 100 µl lysis-buffer. Lysates were incubated with biotin-labeled oligo(dT)₂₀ for 5 min at 37°C. The mix was transferred to streptavidin-coated tubes and incubated for 5 min at 37°C. After washing 3 times with 200 µl washing buffer, 30 µl of the reverse transcription mix (5mM MgCl₂, 1x reverse transcription buffer, 1 mM dNTP, 0.4 U recombinant RNasin ribonuclease inhibitor, 0.4 U AMV reverse transcriptase, 0.5 µg random hexamers in nuclease-free water) were added to the tubes and incubated for 10 min at room temperature followed by 45 min at 42°C. To inactivate AMV reverse transcriptase and separate mRNA from the streptavidin-biotin complex, samples were heated at 99°C for 5 min, transferred to microcentrifuge tubes and incubated on ice for 5 min, diluted 1:2 in nuclease-free water, and stored at -20°C.

### Quantitative Real-Time PCR

Oligonucleotides were designed by using computer software Primer Express 2.0 (Applied Biosystems, USA) and synthesized by Isogen lifescience (IJsselstein, the Netherlands). Primer specificity was computer tested (BLAST, National center for Biotechnology Information) by homology search with the human genome and later confirmed by dissociation curve analysis. PCR reactions were performed with SYBR green method in an ABI 7900HT sequence detection system (Applied Biosystems, USA). The reactions were set on a 96 well-plate by mixing 4 µl of the 2 times concentrated Power SYBR Green Master Mix (Applied Biosystems) with 2 µl of the primer solution containing 5 nmol/µl of both primers and 2 µl of a cDNA solution. The thermal profile for all the reactions was 2 min at 50°C, followed by 10 min at 95°C and then 40 cycles of 15 sec at 95°C and 1 min 60°C. The fluorescence monitoring occurred at the end of each cycle. The Ct value is defined as the number of PCR cycles where the fluorescence signal exceeds the threshold value, which is fixed above 10 times the standard deviation of the fluorescence during the first 15 cycles and typically corresponds to 0.2 relative fluorescence units. This threshold is set constant throughout the study and corresponds to the log linear range of the amplification curve. The normalized amount of target, or relative abundance, reflects the relative amount of target transcripts with respect to the expression of the endogenous reference gene. GAPDH served as an endogenous reference gene.

### Dendritic cell-driven Th1/ Th2 differentiation

Immature DCs were cultured from monocytes of healthy donors in RPMI Medium (Gibco), supplemented with 10% FCS (BioWithaker, Verviers, Belgium), 500 U/ml IL-4 and 800 U/ml GM-CSF (Biosource). At day 4, DC-maturation was induced with N. gonorrhoeae at an MOI of 100. The following positive controls were included in the assay: (1) 10 ng/ml *E*. *coli* LPS, (mixed Th1/ Th2 response); (2) 10 µg/ml PGE2 and 10 ng/ml LPS (Th2); (3) 10 µg/ml poly I:C (Th1) and (4) 20 µg/ml poly I:C and 5 µg /ml R837 (Th1). After 2 days, DCs were washed a At day 5, rIL-2 (10 U/ml) was added, and the cultures were expanded for the next 7 days. To determine cytokine production by Th cells, at day 12-15 quiescent T cells were re-stimulated with 10 ng/ml PMA and 1 µg/ml ionomycin (both Sigma-Aldrich) for 6 h. After 1 h 10 µg/ml Brefeldin A (Sigma-Aldrich) was added to the T cells. Single cell production of IL-4 and IFN_{Y} was determined by intracellular flow cytometric analysis. Cells were fixed in 2% PFA, permeabilized with 0.5% saponin (Sigma-Aldrich) and stained with anti-human IFN_{Y}-FITC and anti-human IL-4-PE (BD Pharmingen).

### RESULTS

### Specific interaction of MGL with Neisseria gonorrhoeae

The Neisseria gonorrhoeae lipooligosaccharide (LOS) undergoes substantial phase variation in vivo. This can lead to the exposure of different terminal sugars on the LOS core structure (See Example 7, Figure 9). The F62 wildtype strain has a terminal GalNAc sugar and is thus a potential ligand for the MGL molecule. Here we tested the ability of MGL to interact with N. gonorrhoeae F62 wildtype (WT, terminal GalNAc), N. gonorrhoeae lgtD mutant (terminal galactose) or N. gonorrhoeae lgtB mutant (terminal GlcNAc). Furthermore, the functional consequences of an interaction between N. gonorrhoeae and dendritic cells or macrophages were investigated.
In an ELISA_based assay, MGL-Fc specifically interacted with N.gonorrhoeae WT and not with the lgtD or lgtB mutants. The MGL interaction could be blocked by the addition of EGTA, anti-MGL antibodies or an excess of amount of free ligand (Example 7, Figure 1A). Next, binding of cellularly expressed MGL was tested. CHO and CHO-MGL were incubated with FITC-labeled N. gonorrhoeae and binding was analzyed by flow cytometry. Only background binding of the bacteria to parental CHO cells was observed, strong binding was observed of CHO-MGL to N. gonorrhoeae WT (Example 7, Figure 1B).) Binding of the wildtype strain could be blocked by Ca2+-depletion (EGTA), blocking anti-MGL antibodies (1G6.6) and the addition of excess amount of free ligand (GalNAc), demonstrating MGL-dependent binding. The low binding of MGL to the lgtB strain is probably caused by differential glycosylation of the N. gonorrhoeae pili, of which the glycosylation is independently regulated compared to the LOS glycosylation.

**Binding of N. gonorrhoeae to DC.**Human immature DC naturally express the MGL receptor. So binding of N. gonorrhoeae to DC was investigated. Binding of the wildtype strain could be blocked by Ca2+-depletion (EGTA) or blocking anti-MGL antibodies (1G6.6) and not with an isotype control antibody (anti-LFA-1), demonstrating MGL-dependent binding (Example 7, figure 2). N. gonorrhoeae lgtD displayed only low binding to DCs. Binding of N. gonorrhoeae lgtB to DCs was higher compared to the wildtype strain, however binding could only be blocked with EGTA and not with anti-MGL antibodies, suggesting that the observed binding is C-type lectin-mediated but not MGL-dependent. C-type lectins function in the internalization and degradation of pathogens. All N. gonorrhoeae strains are internalized by DCs, although uptake of the wildtype stain seems to be delayed compared to N. gonorrhoeae lgtD and lgtB (Example 7, figure 3).

### Functional consequences of the Neisseria gonorrhoeae interaction.

Day 4 immature DC and macrophages were incubated with N. gonorrhoeae at indicated MOI. After 20 hr incubation maturation was determined using specific maturation markers, such as CD14 (macrophages only), CD80, CD83 (DC only) and CD86. N. gonorrhoeae induced both DC and macrophage maturation (Example 7, figures 4 and 5). No major differences were observed between strains, irrespective of the MOI used.

Next, the cytokine production of DC and macrophages incubated with N. gonorrhoeae was investigated by measuring cytokine secretion in the supernatants of the stimulated cells. DC and macrophages stimulated with N. gonorrhoeae wildtype secrete reduced amounts of IL-10 compared to DC and macrophages incubated with N. gonorrhoeae lgtD or lgtB. The lower IL-10 levels correlate with an increased production of IL-12p70 in the DC (Example 7, figure 6).

Next to actual cytokine secretion, also the cytokine mRNA levels in DCs incubated with N. gonorrhoeae were determined. DCs incubated with N. gonorrhoeae wildtype displayed strongly reduced levels of IL-23p19 and TGFβ and slightly lower mRNA levels of TNFα, IL-6 and IL-10 (Example 7, figure 7).

Differences in DC maturation or cytokine prodfuction can alter the capacity of DC to induce T helper responses. Therefore we investigated the Th1 or Th2 inducing capacity of DC incubated with N. gonorrhoeae. DC stimulated N. gonorrhoeae wildtype displayed a Th2-inducing phenotype in DCs (Example 7, figure 8). Compared to the lgtB or lgtD strain, N. gonorrhoeae wildtype induces a more pronounced Th2 profile in naive T cells.

In conclusion, MGL specifically interacts with N. gonorrhoeae wildtype that contains a terminal GalNAc sugar. This interaction leads to functional modification of the DC or macrophage phenotype, indicated by the increased IL-12p70 production, the lower IL-10 secretion and the Th2-inducing capacity.

### EXAMPLE 8

### MATERIALS AND METHODS

### Cells and cell lines.

All cell lines were maintained in RPMI containing 10% fetal calf's serum (Invitrogen, Carlsbad, CA). Immature DC were cultured for 4-7 days from monocytes obtained from buffy coats of healthy donors (Sanquin, Amsterdam) in the presence of IL-4 (500 U/ml) and GM-CSF (800 U/ml). DC were matured by addition of 1 µg/ml LPS (Salmonella typhosa, Sigma-Aldrich, St. Louis, MO). Macrophages were generated from monocytes by culturing in the presence of 1000 U/ml GM-CSF. Dexamethasone was added at 1 µM.

### Binding of MGL to MUC1 transfected CHO cells

Chinese hamster ovary cells (CHO) cells and CHO cells transfected with MUC1 were incubated with MGL-Fc (0.5 µg/ml) in Tris-sodium buffer for 30 min at room temperature. The cells were washed and further incubated with FITC-labeled goat anti-human IgG (Jackson laboratories) for 30 min. Binding was analyzed by flow cytometry.

### DC and macrophage maturation

Immature monocyte derived DC or macrophages (day 4) were stimulated with anti-MGL (20 µg/ml) alone or anti-MGL in combination with LPS (10 ng/ml), Poly I:C (10 µg/ml), Pam3CysK4 (1 µg/ml) or R837 (5 µg/ml) for 20 hrs at 37°C. Cell-surface expression of the maturation marker CD86 using PE-conjugated antibodies was used to determine maturation.
Monocyte derived DC (day 4) were generated in the presence of dexamethasone. 200.10³ DC were incubated in the presence of MUC1 transfected CHO cells and CHO cells overnight, before the addition of LPS (10 ng/ml) or poly I:C (20 µg/ml). The cells were further incubated for 6 hours, collected and lysed. Cytokines were quantified by real time PCR.

### Cytokine measurements

For the detection of cytokines, culture supernatants were harvested 20 hrs after DC or macrophage activation and frozen at -80°C until analysis. Cytokines were measured by enzyme-linked immunosorbant assay (ELISA) with CytoSets™ ELISA kits (Biosource) according to the manufacturer's instructions.

### mRNA isolation and cDNA synthesis

mRNA was isolated by poly (A+) RNA capture in streptavidin-coated tubes with an mRNA Capture kit (Roche, Switzerland) and cDNA was synthesized with the Reverse Transcription System kit (Promega, USA) following manufacturer's guidelines. In brief, cells were washed twice with ice-cold PBS and harvested with 100 µl lysis-buffer. Lysates were incubated with biotin-labeled oligo(dT)₂₀ for 5 min at 37°C. The mix was transferred to streptavidin-coated tubes and incubated for 5 min at 37°C. After washing 3 times with 200 µl washing buffer, 30 µl of the reverse transcription mix (5mM MgCl₂, 1x reverse transcription buffer, 1 mM dNTP, 0.4 U recombinant RNasin ribonuclease inhibitor, 0.4 U AMV reverse transcriptase, 0.5 µg random hexamers in nuclease-free water) were added to the tubes and incubated for 10 min at room temperature followed by 45 min at 42°C. To inactivate AMV reverse transcriptase and separate mRNA from the streptavidin-biotin complex, samples were heated at 99°C for 5 min, transferred to microcentrifuge tubes and incubated on ice for 5 min, diluted 1:2 in nuclease-free water, and stored at -20°C.

### Quantitative Real-Time PCR

Oligonucleotides were designed by using computer software Primer Express 2.0 (Applied Biosystems, USA) and synthesized by Isogen lifescience (IJsselstein, the Netherlands). Primer specificity was computer tested (BLAST, National center for Biotechnology Information) by homology search with the human genome and later confirmed by dissociation curve analysis. PCR reactions were performed with SYBR green method in an ABI 7900HT sequence detection system (Applied Biosystems, USA). The reactions were set on a 96 well-plate by mixing 4 µl of the 2 times concentrated Power SYBR Green Master Mix (Applied Biosystems) with 2 µl of the primer solution containing 5 nmol/µl of both primers and 2 µl of a cDNA solution. The thermal profile for all the reactions was 2 min at 50°C, followed by 10 min at 95°C and then 40 cycles of 15 sec at 95°C and 1 min 60°C. The fluorescence monitoring occurred at the end of each cycle. The Ct value is defined as the number of PCR cycles where the fluorescence signal exceeds the threshold value, which is fixed above 10 times the standard deviation of the fluorescence during the first 15 cycles and typically corresponds to 0.2 relative fluorescence units. This threshold is set constant throughout the study and corresponds to the log linear range of the amplification curve. The normalized amount of target, or relative abundance, reflects the relative amount of target transcripts with respect to the expression of the endogenous reference gene. GAPDH served as an endogenous reference gene.

### RESULTS

To investigate whether ligand binding to MGL leads to changes in DC or macrophage phenotype, antigen presenting cells were incubated with MGL ligands, here MUC1, or specific anti-MGL antibodies (which mimick ligand binding) and evaluated for maturation markers and cytokine responses.

### MGL-MUC1 interaction

CHO cells were transfected with the MUC1 protein and binding of MGL-Fc to these cells to MGL was measured by flow cytometry. MGL-Fc specifically interacted with MUC1 transfected into CHO cells, whereas parental cells are not bound (Example 8, figure 1A). Next, dexamethasone treated DC were incubated in the presence of CHO cells or CHO cells expressing MUC1. Cytokine secretion of the dexamethasone DC was determined by RT-PCR and is displayed as relative abundance compared to the housekeeping gene GAPDH, which served as an endogenous reference gene. Dexamethasone DC incubated on CHO-MUC1 displayed increased mRNA levels of IL-10 (Example 8, figure 1B)and IL-27 (Example 8, figure 1C).

### Immunomodulation of antigen presenting cells

Antigen presenting cells (APCs), immature DCs, dexamethasone DCs and macrophages, were stimulated with TLR-ligands and anti-MGL, as a surrogate ligand. Anti-LFA-1 was included as an isotype control. After 20 hours incubation cells were evaluated for maturation markers and cytokine production. Incubation of APCs with TLR-ligands in the presence or absence of anti-MGL does not lead to altered expression of maturation markers (data not shown).
1. Dendritic cells: Immature DC were treated with TLR ligands in the presence or absence of anti-MGL and evaluated for maturation markers or cytokine mRNA levels. No alteration in the expression of maturation markers was observed (data not shown). However, incubation of anti-MGL without any TLR-ligands increased mRNA levels or IL-1β. Poly I:C treated DC displayed reduced levels of IL-27p28, IL-23 and TNFα, and higher levels of TGFβ. R837 treated DC displayed lower levels of TGFβ and higher levels of IL-23 and IL-6 (Example 8, figure 2).
2. Dexamethasone DC: Also in dexamethasone cultured DC incubation of TLR-ligands in the presence of anti-MGL did not lead to altered expression of maturation markers (data not shown). However, TGFβ secretion was increased in dexamethasone DCs incubated with anti-MGL alone or anti-MGL in combination with Poly I:C (Example 8, figure 3).
3. Macrophages: Strikingly, in some macrophage donors a change in maturation markers in the presence of anti-MGL (Example 8, figure 4) occurred. Macrophages were stimulated for 20 hours with TLR-ligands in the presence or absence of anti-MGL. Subsequently the expression of maturation markers was determined by flow cytometry. CD86 expression is shown as an example, similar results were obtained for CD80 and HLA-DR. Anti-MGL did not alter the expression of maturation markers in 1 out of 4 donors tested (donor 2), whereas in two donors expression was lowered (donors 1 and 3) and in one donor anti-MGL alone induced maturation (donor 4).
   Also the cytokine profiles of macrophages incubated with anti-MGL showed remarkable differences. Cytokine production was measured by RT-PCR and displayed as relative abundance compared to the reference gene GAPDH (Example 8, figure 5). The following differences were observed: anti-MGL alone: Upregulation of IL-10, (IL-6), IL-23, IL-1α, IL-1β and IL-27EBI3. Downregulation of TGFβ; anti-MGL+LPS: Upregulation of TGFβ, IL-23, IL-27p28 and TNF α. Downregulation of IL-10, IL-1β and IL-27EBI3; anti-MGL+Poly I:C: Upregulation of IL-10. Downregulation of TGFβ and IL-23; anti-MGL+R837 or Pam3CysK4: Upregulation of IL-6 and TGFβ. Downregulation of IL-10 and IL-1β.

Together these results demonstrate that MGL triggering leads to modulation of immune responses depending on the other danger signals the APC receives.

### REFERENCES except for examples 2 and 3.

1. Janeway, C.A. & Medzhitov, R. Innate immune recognition. Annu Rev Immunol 20, 197-216 (2002).
2. Steinman, R.M., Hawiger, D., & Nussenzweig, M.C. Tolerogenic dendritic cells. Annu Rev Immunol 21, 685-711 (2003).
3. Mosser, D.M. The many faces of macrophage activation. J. Leukoc. Biol. 73, 209-212 (2003).
4. Piemonti, L. et al. Glucocorticoids affect human dendritic cell differentiation and maturation. J. Immunol. 162, 6473-6481 (1999).
5. Schebesch, C. et al. Alternatively activated macrophages actively inhibit proliferation of peripheral blood lymphocytes and CD4+ T cells in vitro. Immunology 92, 478-486 (1997).
6. Figdor, C.G., van Kooyk, Y., & Adema, G.J. C-type lectin receptors on dendritic cells and Langerhans cells. Nature Rev Immunol 2, 77-84 (2002).
7. Geijtenbeek, T.B., van Vliet, S.J., Engering, A., 't Hart, B.A., & van Kooyk, Y. Self- and Nonself-Recognition by C-Type Lectins on Dendritic Cells. Annu Rev Immunol 22, 33-54 (2004).
8. Engering, A. et al. The dendritic cell-specific adhesion receptor DC-SIGN internalizes antigen for presentation to T cells. J Immunol 168, 2118-26 (2002).
9. Bonifaz, L. et al. Efficient targeting of protein antigen to the dendritic cell receptor DEC-205 in the steady state leads to antigen presentation on major histocompatibility complex class I products and peripheral CD8+ T cell tolerance. J Exp Med 196, 1627-38 (2002).
10. Gantner, B.N., Simmons, R.M., Canavera, S.J., Akira, S., & Underhill, D.M. Collaborative induction of inflammatory responses by dectin-1 and Toll-like receptor 2. J. Exp. Med. 197, 1107-1117 (2003).
11. Raes, G. et al. Macrophage galactose-type C-type lectins as novel markers for alternatively activated macrophages elicited by parasitic infections and allergic airway inflammation. J. Leukoc. Biol. 77, 321-327 (2005).
12. Higashi, N. et al. The macrophage C-type lectin specific for galactose/N-acetylgalactosamine is an endocytic receptor expressed on monocyte-derived immature dendritic cells. J. Biol. Chem. 277, 20686-20693 (2002).
13. van Vliet, S.J. et al. Carbohydrate profiling reveals a distinctive role for the C-type lectin MGL in the recognition of helminth parasites and tumor antigens by dendritic cells. Int. Immunol. 17, 661-669 (2005).
14. Tsuiji, M. et al. Molecular cloning and characterization of a novel mouse macrophage C-type lectin, mMGL2, which has a distinct carbohydrate specificity from mMGL1. J. Biol. Chem. 277, 28892-28901 (2002).
15. Hermiston, M.L., Xu, Z., & Weiss, A. CD45: a critical regulator of signaling thresholds in immune cells. Annu. Rev. Immunol. 21, 107-137 (2003).
16. Furukawa, K. et al. Structural study of the O-linked sugar chains of human leukocyte tyrosine phosphatase CD45. Eur. J. Biochem. 251, 288-294 (1998).
17. Zapata, J.M., Pulido, R., Acevedo, A., Sanchez-Madrid, F., & de Landazuri, M.O. Human CD45RC specificity. A novel marker for T cells at different maturation and activation stages. J. Immunol. 152, 3852-3861 (1994).
18. Garcia, G.G., Berger, S.B., Sadighi Akha, A.A., & Miller, R.A. Age-associated changes in glycosylation of CD43 and CD45 on mouse CD4 T cells. Eur. J. Immunol. 35, 622-631 (2005).
19. Daniels, M.A., Hogquist, K.A., & Jameson, S.C. Sweet 'n' sour: the impact of differential glycosylation on T cell responses. Nat Immunol 3, 903-10 (2002).
20. Geijtenbeek, T.B. et al. Identification of DC-SIGN, a novel dendritic cell-specific ICAM-3 receptor that supports primary immune responses. Cell 100, 575-85 (2000).
21. Puig-Kroger, A. et al. Regulated expression of the pathogen receptor dendritic cell-specific intercellular adhesion molecule 3 (ICAM-3)-grabbing nonintegrin in THP-1 human leukemic cells, monocytes, and macrophages. J. Biol. Chem. 279, 25680-25688 (2004).
22. Carlsson, S.R. & Fukuda, M. Isolation and characterization of leukosialin, a major sialoglycoprotein on human leukocytes. J. Biol. Chem. 261, 12779-12786 (1986).
23. Rogers, P.R., Pilapil, S., Hayakawa, K., Romain, P.L., & Parker, D.C. CD45 alternative exon expression in murine and human CD4+ T cell subsets. J. Immunol. 148, 4054-4065 (1992).
24. Abraham, R.T. & Weiss, A. Jurkat T cells and development of the T-cell receptor signalling paradigm. Nat. Rev. Immunol. 4, 301-308 (2004).
25. Xu, Z. & Weiss, A. Negative regulation of CD45 by differential homodimerization of the alternatively spliced isoforms. Nat. Immunol. 3, 764-771 (2002).
26. Chu, D.H. et al. The Syk protein tyrosine kinase can function independently of CD45 or Lck in T cell antigen receptor signaling. EMBO J. 15, 6251-6261 (1996).
27. Hintzen, R.Q. et al. Regulation of CD27 expression on subsets of mature T-lymphocytes. J. Immunol. 151, 2426-2435 (1993).
28. Xia, C.Q., Peng, R., Beato, F., & Clare-Salzler, M.J. Dexamethasone induces IL-10-producing monocyte-derived dendritic cells with durable immaturity. Scand. J. Immunol. 62, 45-54 (2005).
29. Ju, T. & Cummings, R.D. A unique molecular chaperone Cosmc required for activity of the mammalian core 1 beta 3-galactosyltransferase. Proc. Natl. Acad. Sci. U. S. A 99, 16613-16618 (2002).
30. Ohta, T., Kitamura, K., Maizel, A.L., & Takeda, A. Alterations in CD45 glycosylation pattern accompanying different cell proliferation states. Biochem. Biophys. Res. Commun. 200, 1283-1289 (1994).
31. Stamenkovic, I., Sgroi, D., Aruffo, A., Sy, M.S., & Anderson, T. The B lymphocyte adhesion molecule CD22 interacts with leukocyte common antigen CD45RO on T cells and alpha 2-6 sialyltransferase, CD75, on B cells. Cell 66, 1133-1144 (1991).
32. Walzel, H., Schulz, U., Neels, P., & Brock, J. Galectin-1, a natural ligand for the receptor-type protein tyrosine phosphatase CD45. Immunol. Lett. 67, 193-202 (1999).
33. London, C.A., Lodge, M.P., & Abbas, A.K. Functional responses and costimulator dependence of memory CD4+ T cells. J. Immunol. 164, 265-272 (2000).
34. Penninger, J.M., Irie-Sasaki, J., Sasaki, T., & Oliveira-dos-Santos, A.J. CD45: new jobs for an old acquaintance. Nat. Immunol. 2, 389-396 (2001).
35. Majeti, R., Bilwes, A.M., Noel, J.P., Hunter, T., & Weiss, A. Dimerization-induced inhibition of receptor protein tyrosine phosphatase function through an inhibitory wedge. Science 279, 88-91 (1998).
36. Nam, H.J., Poy, F., Saito, H., & Frederick, C.A. Structural basis for the function and regulation of the receptor protein tyrosine phosphatase CD45. J. Exp. Med. 201, 441-452 (2005).
37. Tchilian, E.Z. & Beverley, P.C. Altered CD45 expression and disease. Trends Immunol. 27, 146-153 (2006).
38. Irie-Sasaki, J. et al. CD45 is a JAK phosphatase and negatively regulates cytokine receptor signalling. Nature 409, 349-354 (2001).
39. Klaus, S.J., Sidorenko, S.P., & Clark, E.A. CD45 ligation induces programmed cell death in T and B lymphocytes. J. Immunol. 156, 2743-2753 (1996).
40. Fortin, M. et al. Apoptosis mediated through CD45 is independent of its phosphatase activity and association with leukocyte phosphatase-associated phosphoprotein. J. Immunol. 168, 6084-6089 (2002).
41. Lesage, S. et al. CD4+ CD8+ thymocytes are preferentially induced to die following CD45 cross-linking, through a novel apoptotic pathway. J. Immunol. 159, 4762-4771 (1997).
42. Liu, Z., Dawes, R., Petrova, S., Beverley, P.C., & Tchilian, E.Z. CD45 regulates apoptosis in peripheral T lymphocytes. Int. Immunol.(2006). 43. Latinis, K.M. et al. Regulation of CD95 (Fas) ligand expression by TCR-mediated signaling events. J. Immunol. 158, 4602-4611 (1997).
44. Powrie, F. et al. Inhibition of Th1 responses prevents inflammatory bowel disease in scid mice reconstituted with CD45RBhi CD4+ T cells. Immunity. 1, 553-562 (1994).
45. Fowell, D. & Mason, D. Evidence that the T cell repertoire of normal rats contains cells with the potential to cause diabetes. Characterization of the CD4+ T cell subset that inhibits this autoimmune potential. J. Exp. Med. 177, 627-636 (1993).
46. Luke, P.P., O'Brien, C.A., Jevnikar, A.M., & Zhong, R. Anti-CD45RB monoclonal antibody-mediated transplantation tolerance. Curr. Mol. Med. 1, 533-543 (2001).
47. Gregori, S. et al. An anti-CD45RO/RB monoclonal antibody modulates T cell responses via induction of apoptosis and generation of regulatory T cells. J. Exp. Med. 201, 1293-1305 (2005).
48. Barrat, F.J. et al. In vitro generation of interleukin 10-producing regulatory CD4(+) T cells is induced by immunosuppressive drugs and inhibited by T helper type 1 (Th1)- and Th2-inducing cytokines. J. Exp. Med. 195, 603-616 (2002).
49. Kumamoto, Y. et al. Identification of sialoadhesin as a dominant lymph node counter-receptor for mouse macrophage galactose-type C-type lectin 1. J. Biol. Chem. 279, 49274-49280 (2004).
50. van Kooyk, Y., van de Wiel-van Kemenade, P., Weder, P., Kuijpers, T.W., & Figdor, C.G. Enhancement of LFA-1-mediated cell adhesion by triggering through CD2 or CD3 on T lymphocytes. Nature 342, 811-813 (1989).

## Claims

1. A binding molecule comprising a terminal GalNAc residue or a complex comprising said binding molecule for use in modulating an immune response in a subject, wherein said binding molecule is a glycoconjugate provided with said terminal GalNAc residue in vitro (a neo-glycoconjugate).

2. A binding molecule according to claim 1, wherein said molecule or said complex comprises a (poly)peptide or lipid comprising said terminal GalNAc residue.

3. A binding molecule according to claim 1 or claim 2, wherein modulating said immune response comprises initiating or enhancing an immune response.

4. A binding molecule according to claim 3, wherein an antigen specific immune response is initiated or enhanced.

5. A binding molecule according to claim 4, wherein said antigen is linked to said molecule or said complex thereby directing said antigen to MGL positive antigen presenting cells.

6. A binding molecule according to any one of claims 3-5, further comprising administering to said subject a composition for stimulating DC-maturation and/or activation.

7. A binding molecule according to claim 6, wherein said composition for stimulating DC maturation comprises an anti-CD40 molecule, a Toll-like receptor ligand, a pro-inflammatory cytokine such as Interleukin-1, IL-6, tumor necrosis factor alpha, and/or prostaglandin E-2, a general adjuvant such as Montanide adjuvant or Freund adjuvant, or in situ maturation through the injection of immature DCs into adjuvant-treated skin.

8. A binding molecule according to any one of claims 1-7, wherein said neo-glycoconjugate has been provided with said GalNAc residue in a cell free environment.

9. A binding molecule according to any one of claims 1-8, wherein said neoglyconjugate comprises at least 2 terminal GalNAc residues.

10. A binding molecule according to any one of claims 1-9, wherein said terminal GalNAc residue is part of a LacdiNAc structure.

11. A binding molecule according to any one of claims 1-10, wherein said terminal GalNAc residue is part of a Tn-structure.

12. A method for preferentially marking immature and/or tolerogenic professional antigen presenting cells (APC) in a subset of cells comprising marking said cells with a binding molecule specific for MGL, wherein said binding molecule is a glycoconjugate provided with said terminal GalNAc residue in vitro (a neo-glycoconjugate).

13. A method for altering cell-cell mediated signalling in a culture of T-cells and APC, comprising altering MGL-CD45 mediated signalling in said collection of cells by providing said culture with a molecule capable of blocking said interaction, wherein said molecule is GalNAc or comprises a terminal GalNac residue.

14. A vaccine comprising a binding molecule comprising a terminal GalNAc residue and an antigen against which the immune response is to be directed.

15. Use of a binding molecule comprising a terminal GalNac residue for the preparation of a composition for mobilizing antigen presenting cells, preferably dendritic cells.
